(19)

Europäisches Patentamt
European Patent Office
Office européen des brevets

(11) **EP 4 305 183 B1**

(12) **EUROPEAN PATENT SPECIFICATION**

(45) Date of publication and mention
of the grant of the patent:
**07.05.2025 Bulletin 2025/19**

(21) Application number: **22710122.7**

(22) Date of filing: **11.03.2022**

(51) International Patent Classification (IPC):
*C12N 15/87* (2006.01)    *A61K 41/00* (2020.01)

(52) Cooperative Patent Classification (CPC):
**C12N 15/87**

(86) International application number:
**PCT/EP2022/056333**

(87) International publication number:
**WO 2022/189627 (15.09.2022 Gazette 2022/37)**

(54) **METHODS FOR DELIVERING A CARGO INTO A CELL**

VERFAHREN ZUM EINBRINGEN VON CARGO IN EINE ZELLE

PROCÉDÉS D'ADMINISTRATION DE CARGO DANS UNE CELLULE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2021 EP 21162290**

(43) Date of publication of application:
**17.01.2024 Bulletin 2024/03**

(73) Proprietor: **Trince bv
9000 Gent (BE)**

(72) Inventors:
• **BRAECKMANS, Kevin**
  **9160 Lokeren (BE)**
• **HARIZAJ, Aranit**
  **9041 Gent (BE)**
• **DE SMEDT, Stefaan**
  **9030 Mariakerke (BE)**
• **SAUVAGE, Félix**
  **59100 Roubaix (FR)**

(74) Representative: **Brantsandpatents bv
Pauline Van Pottelsberghelaan 24
9051 Ghent (BE)**

(56) References cited:
• **WU JINGXIAN ET AL: "A Universal Intracellular
Delivery and Cell Releasing System via the
Photoporation of Polydopamine and the
Thermal-Responsiveness of Poly(N-
isopropylacrylamide)", CHEM. REV. LYU Z. ADV.
FUNC. MATER, 2019, pages 1 - 1, XP055836655,
Retrieved from the Internet <URL:http://
abstracts.biomaterials.org/data/papers/2019/
abstracts/743.pdf> [retrieved on 20210901]**
• **KUK-YOUN JU ET AL: "Bioinspired
Polymerization of Dopamine to Generate
Melanin-Like Nanoparticles Having an Excellent
Free-Radical-Scavenging Property",
BIOMACROMOLECULES, vol. 12, no. 3, 14 March
2011 (2011-03-14), pages 625 - 632, XP055030742,
ISSN: 1525-7797, DOI: 10.1021/bm101281b**
• **WANG XING ET AL: "A Polydopamine
Nanoparticle-Knotted Poly(ethylene glycol)
Hydrogel for On-Demand Drug Delivery and
Chemo-photothermal Therapy", vol. 29, no. 3, 14
February 2017 (2017-02-14), US, pages 1370 -
1376, XP055836645, ISSN: 0897-4756, Retrieved
from the Internet <URL:https://pubs.acs.org/doi/
pdf/10.1021/acs.chemmater.6b05192> DOI:
10.1021/acs.chemmater.6b05192**

EP 4 305 183 B1

- RAES LAURENS ET AL: "Intracellular Delivery of mRNA in Adherent and Suspension Cells by Vapor Nanobubble Photoporation", vol. 12, no. 1, 27 September 2020 (2020-09-27), pages 185 - 185, XP055836210, ISSN: 2311-6706, Retrieved from the Internet <URL:https://link.springer.com/content/pdf/10.1007/s40820-020-00523-0.pdf> DOI: 10.1007/s40820-020-00523-0
- LIU JING ET AL: "Surface Functionalization with Polyethylene Glycol and Polyethyleneimine Improves the Performance of Graphene-Based Materials for Safe and Efficient Intracellular Delivery by Laser-Induced Photoporation", INTERNATIONAL JOURNAL OF MOLECULAR SCIENCES, vol. 21, no. 4, 24 February 2020 (2020-02-24), pages 1540, XP055836206, DOI: 10.3390/ijms21041540
- JING LIU ET AL: "Repeated photoporation with graphene quantum dots enables homogeneous labeling of live cells with extrinsic markers for fluorescence microscopy", LIGHT: SCIENCE & APPLICATIONS, vol. 7, no. 1, 8 August 2018 (2018-08-08), XP055677299, DOI: 10.1038/s41377-018-0048-3
- HARIZAJ ARANIT ET AL: "Photoporation with Biodegradable Polydopamine Nanosensitizers Enables Safe and Efficient Delivery of mRNA in Human T Cells", vol. 31, no. 28, 25 April 2021 (2021-04-25), DE, pages 2102472, XP055836191, ISSN: 1616-301X, Retrieved from the Internet <URL:https://onlinelibrary.wiley.com/doi/full-xml/10.1002/adfm.202102472> DOI: 10.1002/adfm.202102472

**Description**

**FIELD OF THE INVENTION**

The invention is broadly in the field of cell engineering, more precisely in the field of delivering a cargo such as a nucleic acid or a protein into cells.

**BACKGROUND OF THE INVENTION**

The delivery of various macromolecules into the cytosol of different cell types is required for a variety of applications such as cancer immunotherapy, stem cell therapy and other biomedical research areas. The majority of the macromolecules cannot spontaneously cross the cell membrane as it is non-permeable for many compounds. Therefore, different techniques are needed to allow the cytosolic delivery of such cell-impermeable compounds. For several years, electroporation has been used as a standard delivery tool. Electroporation has the ability to form pores into the cell membrane following the application of electrical pulses. The cell-impermeable compounds can then migrate through these pores from the surrounding medium into the cell's interior. Despite that electroporation can reach high delivery efficiencies for a variety of cell types, several limitations have been reported, including high cytotoxicity, the induction of genomic alterations, the induction of phenotypic changes and a low delivery efficiency in several primary (immune) cells. Hence, novel techniques, which do not affect the genotype or phenotype of the cells and which can achieve a high delivery efficiency with a high viability, are required.

For example, safe and efficient production of chimeric antigen receptor (CAR)-T cells is of crucial importance for cell-based cancer immunotherapy. While, historically, viral vectors are preferentially used to transduce T cells, they are associated with safety concerns and offer limited flexibility in terms of cargo type and size. Physical transfection methods, therefore, are gaining in importance, being readily compatible with different cell types and a broad variety of cargo molecules. In particular, nanoparticle-sensitized photoporation using nanoparticles composed of metals, metal oxides or carbon allotropes has been introduced in recent years as a gentle method to transiently permeabilize cells, allowing subsequent entry of external cargo molecules into the cells. Unfortunately, these nanoparticles do not - or only very slowly - degrade over time, thus hampering clinical translation. Indeed, more stringent regulatory issues apply to the medical use of non-degradative nanomaterials. In addition, there is a generic safety concern about the use of inorganic nanoparticles, such as gold nanoparticles. Of particular concern is that fact that after laser treatment, gold nanoparticles for photoporation, for instance with an initial size of 60 nm, tend to fragment into very small nanoparticles (<10 nm) which could potentially go through the nuclear membrane and intercalate with the DNA, causing genotoxic effects.

A method applying photoporation for the transfection of cells using photoresponsive gold nanoparticles is disclosed in the prior art (L. Raes et al., "Intracellular delivery of mRNA in adherent and suspension cells by vapor nanobubble photoporation," NANO-MICRO LETTERS, vol. 12, no. 1, 2020). In particular, the delivery of mRNA to Jurkat cells using the described transfection method is disclosed.

Some other documents disclose methods applying photoporation for the transfection of cells using photoresponsive graphene nanoparticles (J. Liu et al., "Surface functionalization with polyethylene glycol and polyethyleneimine improves the performance of graphene-based materials for safe and efficient intracellular delivery by laser-induced photoporation," INTERNATIONAL JOURNAL OF MOLECULAR SCIENCES, vol. 21, no. 4, 2020; J. Liu et al., "Repeated photoporation with graphene quantum dots enables homogeneous labeling of live cells with extrinsic markers for fluorescence microscopy," LIGHT-SCIENCE & APPLICATIONS, vol. 7, 2018).

The above cited methods all comprise the use of photoresponsive inorganic nanoparticles. As earlier stated, there is a generic safety concern about the use of photoresponsive inorganic nanoparticles for medical purposes, relating for example to genotoxic effects. Subsequently, the cited methods are less likely to be suitable for therapeutic use.

In order to avoid exposure of cells to small (gold) fragments coming from inorganic nanoparticles, such as gold nanoparticles, prior art methods use photoresponsive micromachined substrates that consist of an array of micro-sized pyramids that are covered with a gold layer. Cells are grown on top of these gold-covered pyramids and irradiated. The pyramids heat up, especially at the tips which are in contact with the cells, and temporarily permeabilize the cell membrane at these locations, allowing the influx of exogenous molecules (Saklayen et al., ACS Nano, 2017, 11(4):3671-3680). However, such methods are technically complicated.

In view of the above, there remains a need in the art for further and/or improved methods for delivering cargo molecules such as nucleic acids into cells.

**SUMMARY OF THE INVENTION**

By extensive experiments, the present inventors have found a method for the delivery of a cargo such as mRNA into cells using photoresponsive organic particles. The experiments demonstrated for the first time successful vapour bubble formation from photoresponsive organic particles upon irradiation with electromagnetic radiation, and delivery of a cargo.

Accordingly, a first aspect of the invention relates to an *in vitro* or *ex vivo* method for delivering a cargo into a cell, the method comprising:

- contacting a cell with one or more photoresponsive organic particles and a cargo, wherein the cargo is not bound to the one or more photoresponsive organic particles, and wherein the organic particle is selected from the group consisting of a polymer-based particle, a protein-based particle, a lipid-based particle, and a combination thereof, thereby obtaining a mixture of the cell, the cargo, and the one or more photoresponsive organic particles; and

- irradiating the mixture of the cell, the cargo, and the one or more photoresponsive organic particles with electromagnetic radiation, thereby causing permeabilization of a barrier of the cell and delivering the cargo into the cell; or

- contacting a cell with one or more photoresponsive organic particles, wherein the organic particle is selected from the group consisting of a polymer-based particle, a protein-based particle, a lipid-based particle, and a combination thereof, thereby obtaining a mixture of the cell and the one or more photoresponsive organic particles;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with a cargo, thereby delivering the cargo into the cell.

After extensive experiments, the present inventors have found that the methods employing the principles of the invention allow successful vapour bubble formation and delivery of a cargo, such as a nucleic acid, e.g. mRNA, into cells for instance into HeLa cells, as well as into hard-to-transfect human T cells. The present methods allow high efficiency, e.g. high transfection efficiency, while resulting in low cytotoxicity and high cell viability, and hence high yield of delivery. Importantly, about two times more living mRNA transfected primary human T cells were obtained after the present methods as compared to nucleofection. Considering that photoresponsive organic particles may be prepared from a clinically approved precursor such as polydopamine, the present methods open up the possibility to allow for the production of engineered therapeutic cell products, such as CAR-T cells.

Furthermore, the present methods using photoresponsive organic particles have the following advantages over prior art methods:

1. The present methods using photoresponsive organic nanoparticles have the advantage of being a technologically simple approach, only requiring exposing cells to the photoresponsive organic particles and providing electromagnetic irradiation.

2. The photoresponsive organic particles are better biodegradable and biocompatible compared to inorganic particles, which is an advantage from a safety and regulatory point of view.

3. The photoresponsive organic particles can be prepared from precursor molecules that are already clinically approved, e.g. dopamine hydrochloride, which can be used to create photoresponsive organic particles, e.g. polydopamine particles, witch excellent photothermal properties.

4. Such photoresponsive organic particles can be easily synthesized in a variety of sizes, which is much more difficult for inorganic nanoparticles such as gold nanoparticles.

In view thereof, these methods involving photoresponsive organic particles can easily be translated to *in vivo* methods for the delivery of a cargo into cells of a subject.

A further aspect thus provides one or more photoresponsive organic particles as defined herein and a cargo as defined herein, for use in a method of therapy in a subject, wherein a cargo is delivered to a cell of a subject, the method comprising:

- administering the one or more photoresponsive organic particles to the surrounding of the cell of the subject;

- irradiating at least part of the surrounding of the cell of the subject, thereby causing permeabilization of a barrier of the cell; and

- administering the cargo to the surrounding of the cell of the subject, thereby delivering the cargo into the cell;

or the method comprising:

- administering the one or more photoresponsive organic particle and the cargo to the surrounding of the cell of the subject; and

- irradiating at least part of the surrounding of the cell of the subject, thereby causing permeabilization of a barrier of the cell and delivering the cargo into the cell.

As illustrated in the examples section, an interesting additional finding was that larger photoresponsive organic particles, e.g. of 300 nm, 500 nm, 750 nm and 1000 nm diameter, had the ability to enhance the delivery of very large nucleic acids, like mRNA and pDNA, compared to the use of smaller 60 nm gold nanoparticles and of 100 nm photoresponsive organic particles.

Hence, the present invention preferably provides an *in vitro* or *ex vivo* method for delivering a cargo into a cell, the method comprising:

- contacting a cell with one or more photoresponsive organic particles and a cargo, wherein the cargo is not bound to the one or more photoresponsive organic particles, wherein the largest distance between two points of the one or more photoresponsive organic particles is about 100 nm to about 2000 nm, e.g. about 250 nm to about 1250 nm, and wherein the organic particle is selected from the group consisting of a polymer-based particle, a protein-based particle, a lipid-based particle, and a combination thereof, thereby obtaining a mixture of the cell, the cargo, and the one or more photoresponsive organic particles; and

- irradiating the mixture of the cell, the cargo, and the one or more photoresponsive organic particles with electromagnetic radiation, thereby causing permeabilization of a barrier of the cell and delivering the cargo into the cell; or

- contacting a cell with one or more photoresponsive organic particles, wherein the largest distance between two points of the one or more photoresponsive organic particles is about 100 nm to about 2000 nm, e.g. about 250 nm to about 1250 nm, and wherein the organic particle is selected from the group consisting of a polymer-based particle, a protein-based particle, a lipid-based particle, and a combination thereof, thereby obtaining a mixture of the cell and the one or more photoresponsive organic particles;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with a cargo, thereby delivering the cargo into the cell.

The examples show that such methods provide a high delivery efficiency, while maintaining sufficient cell viability, thereby resulting in high delivery yield of a cargo into the cell. The delivery yield when using photoresponsive organic particles having a size of about 250 nm to about 1250 nm is markedly improved as compared to the delivery yield with photoresponsive organic particles having a size of about 100 nm, or compared to the delivery yield with gold nanoparticles of about 60 nm.

The above and further aspects and preferred embodiments of the invention are described in the following sections and in the appended claims. The subject-matter of appended claims is hereby specifically incorporated in this specification.

**DESCRIPTION OF THE DRAWINGS**

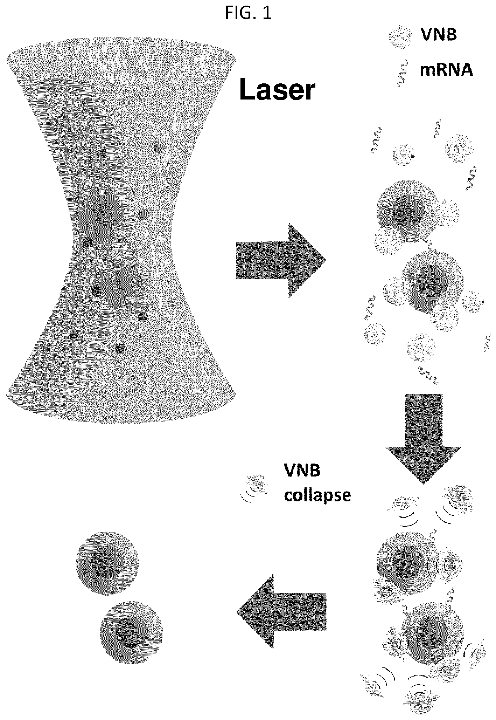

FIG. 1: Schematic representation of a method according to an embodiment of the invention for polydopamine-sensitized delivery of mRNA into cells.

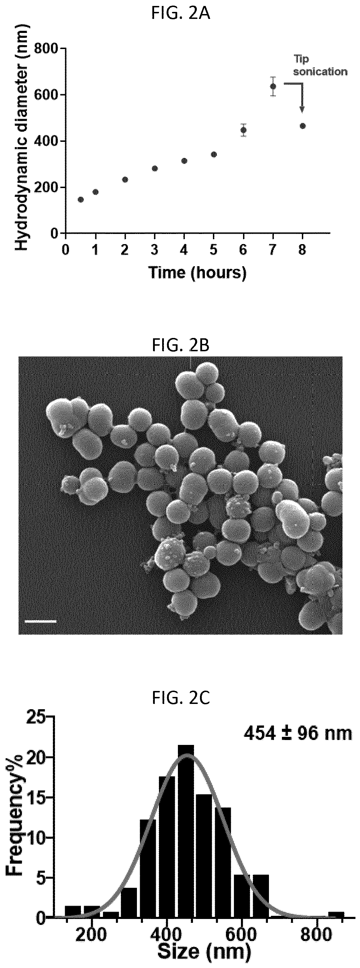

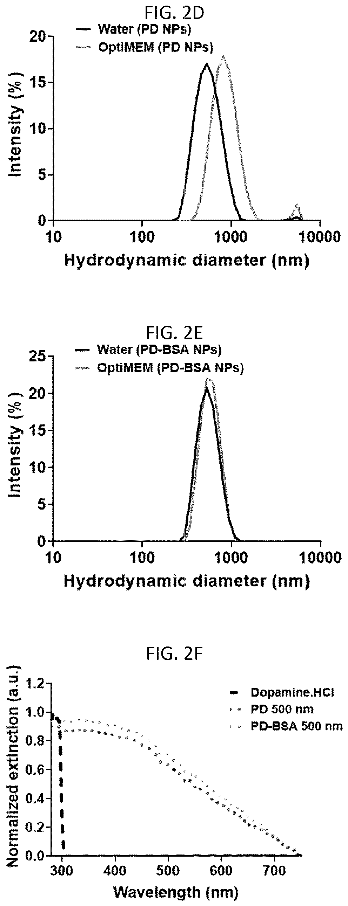

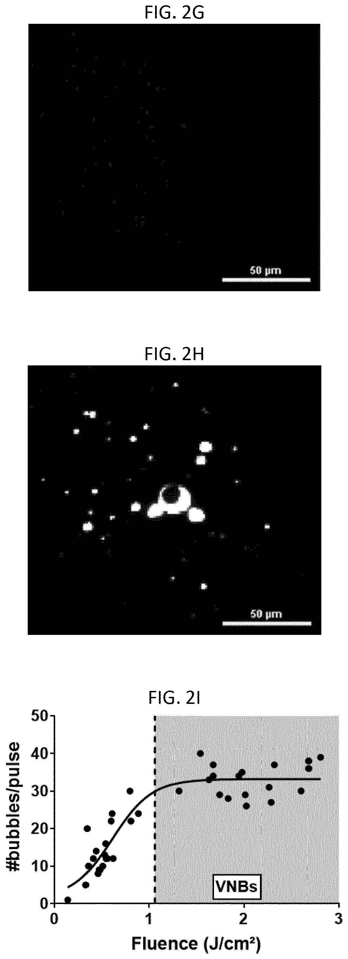

FIG. 2: Physicochemical characterization of bare (non-coated) NPs according to an embodiment of the invention and BSA-coated polydopamine (PD) nanoparticles (NPs) according to an embodiment of the invention. (A) Graph representing the hydrodynamic diameter (in nm) of PD NPs in function of time during the synthesis as measured with dynamic light scattering (DLS) up to 7 hours. Sonication was finally applied to remove any remaining agglomerates which may have formed during the synthesis. (B) Representative SEM image of bare 500 nm PD NPs (scale bar = 500 nm). (C) Graph representing the corresponding size distribution of bare 500 nm PD NPs showing a mean size of $454 \pm 96$ nm. (D) Graph representing the hydrodynamic diameter of bare PD NPs as measured in respectively water (black curve) and Opti-MEM (light grey curve) by DLS (incubation time $\leq$ 10 minutes), showing a clear size increase in Opti-MEM. (E) Graph representing the hydrodynamic diameter of BSA coated PD NPs as measured in respectively water (black curve) and Opti-MEM (light grey curve) by DLS (incubation time $\leq$ 10 minutes). (F) Graph representing the UV/VIS spectrum of respectively dopamine.HCl (black striped line), bare PD NPs (dark grey dotted line), and BSA coated PD NPs (light grey dotted line). (G-H) Dark field microscopy images of PD NPs (G) before and (H) after the application of a laser pulse (7 ns pulse duration, 561 nm wavelength, 1.6 J/cm$^2$) (scale bar = 50 $\mu$m). (I) Graph showing the quantification of the number of VNBs from dark field microscopy images as a function of laser fluence. The dotted line to the left of the grey filled area represents the laser fluence threshold above which 90% of the irradiated PD NPs will form vapour bubbles.

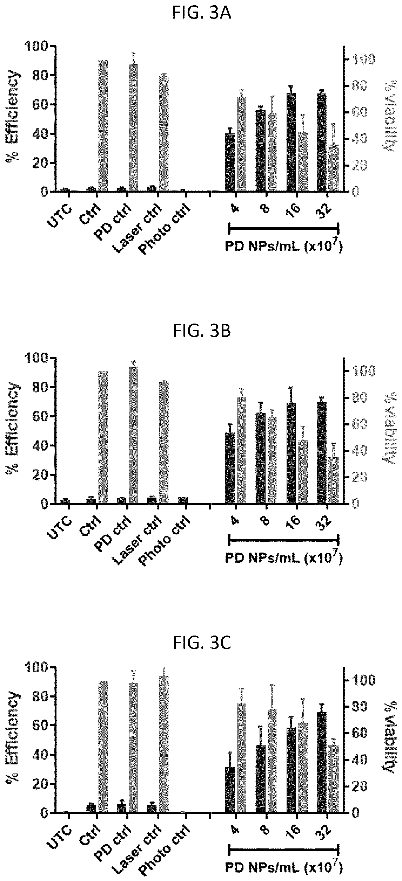

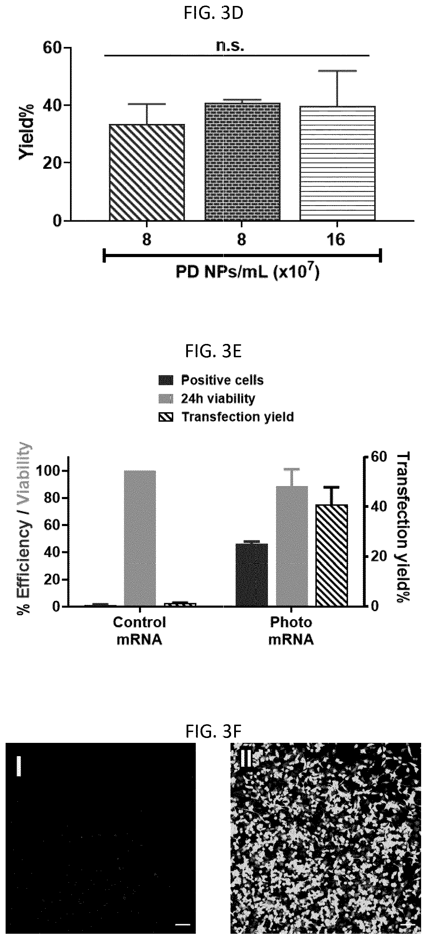

**FIG. 3:** Comparison of two incubation methods according to embodiments of the invention for 500 nm polydopamine-bovine serum albumin (PD-BSA) NPs on HeLa cells. (A) Graph representing the delivery efficiency of 500 kDa FITC-dextran (FD500) (left y-axis, dark grey bars) and cell viability (2 hours after photoporation, right y-axis, light grey bars) via the method with incubation in adherent HeLa cells. UTC: untreated control, Ctrl: mock FD500 control, PD ctrl: PD-BSA NPs and FD500 control, Laser ctrl: laser and FD500 control, Photo ctrl: mock photoporation control ($32\times10^7$ PD-BSA NPs/mL). (B) Graph representing the delivery efficiency of FD500 (left y-axis, dark grey bars) and viability (2 hours, right y-axis, light grey bars) via the mixing method in adherent HeLa cells. (C) Graph representing the delivery efficiency of FD500 (left y-axis, dark grey bars) and viability (2 hours, right y-axis, light grey bars) via the mixing method in suspension HeLa cells (i.e. after trypsinization). (D) Graph representing the yield percentage of viable FD500 positive cells for the different methods: incubation method on adherent HeLa cells (left bar with diagonal stripes), mixing method on adherent HeLa cells (middle bar with horizontal dots) and mixing method on suspension HeLa cells (right bar with horizontal stripes). (E) Graph representing the transfection efficiency of mRNA, encoding for eGFP, (left y-axis, dark grey bars) and corresponding viability (24 hours, left y-axis, light grey bars) with the mixing method ($8\times10^7$ NPs/mL) in suspension HeLa cells. The transfection yield (right y-axis, bars with stripes) is the percentage of viable eGFP positive cells relative to the entire initial cell population. (F) Confocal microscopy images of HeLa cells transfected with eGFP-mRNA for respectively (I) mRNA control and (II) photoporation in the presence of eGFP-mRNA. Scale bar = 100 $\mu$m. Data are shown as mean $\pm$ SD. Statistical significance: One-way ANOVA; n.s. refers to non-significant.

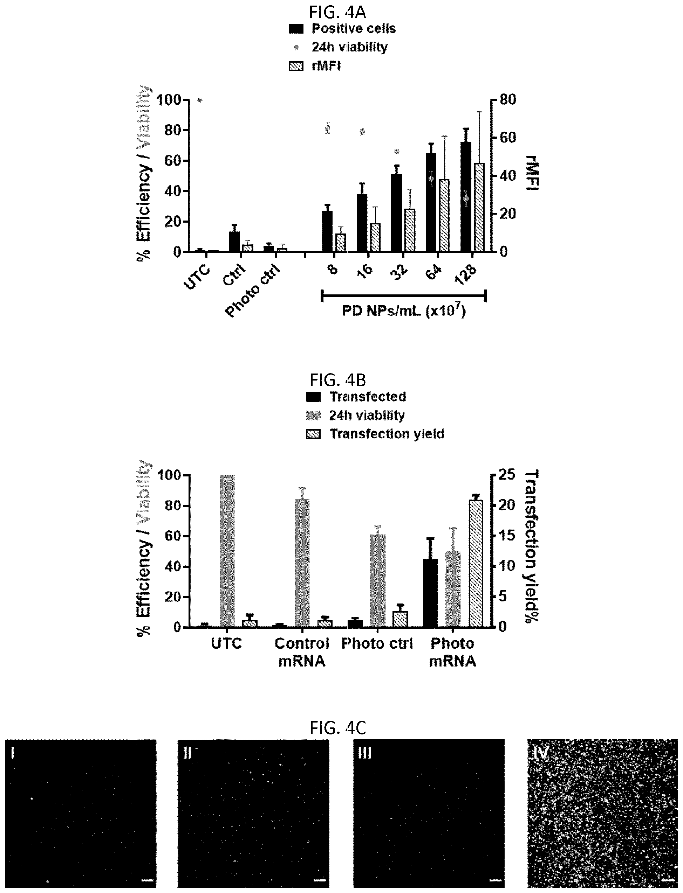

**FIG. 4:** Methods according to embodiments of the invention for delivery of FD500 and eGFP-mRNA into Jurkat cells by the mixing photoporation method with PD-BSA NPs as sensitizers. (A) Graph representing the delivery efficiency (left y-axis, black bars) and relative mean fluorescence intensity (right y-axis, bars with stripes) of FD500 into Jurkat cells after photoporation for an increasing concentration of PD500-BSA NPs. The corresponding viability was measured after 24 hours with the metabolic CellTiter Glo assay (left y-axis, light grey dots). UTC: untreated control, Ctrl: mock FD500 control, Photo ctrl: mock photoporation control (i.e. only laser irradiation with PD-BSA NPs). (B) Graph representing the delivery efficiency for eGFP-mRNA (left y-axis, black bars) by photoporation with a fixed concentration of PD-BSA NPs ($64\times10^7$ PD-BSA NPs/mL). The corresponding viability was measured after 24 hours with the CellTiter Glo assay (left y-axis, light grey bars). The transfection yield (right y-axis, bars with stripes) expresses the percentage of living and transfected cells compared to the initial population of cells. (C) Confocal microscopy images of Jurkat cells transfected with eGFP-mRNA by (I) untreated control (UTC), (II) mRNA control, (III) mock photoporation control ($64\times10^7$ NPs/mL) and (IV) photoporation in the presence of mRNA ($64\times10^7$ NPs/mL). Scale bar = 100 $\mu$m. Data are shown as mean $\pm$ SD.

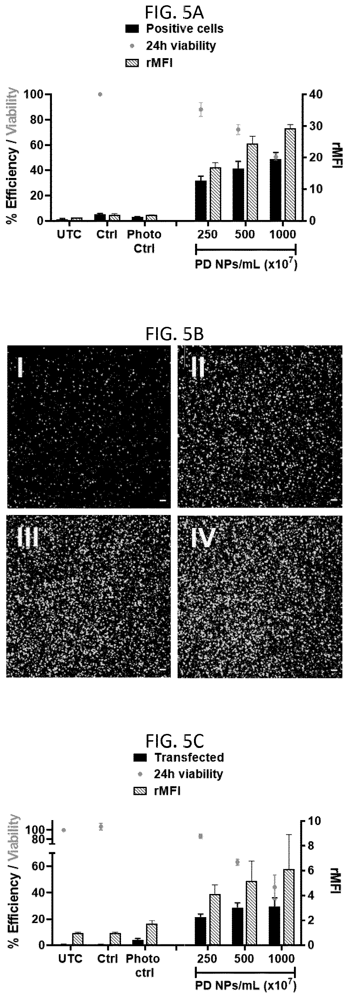

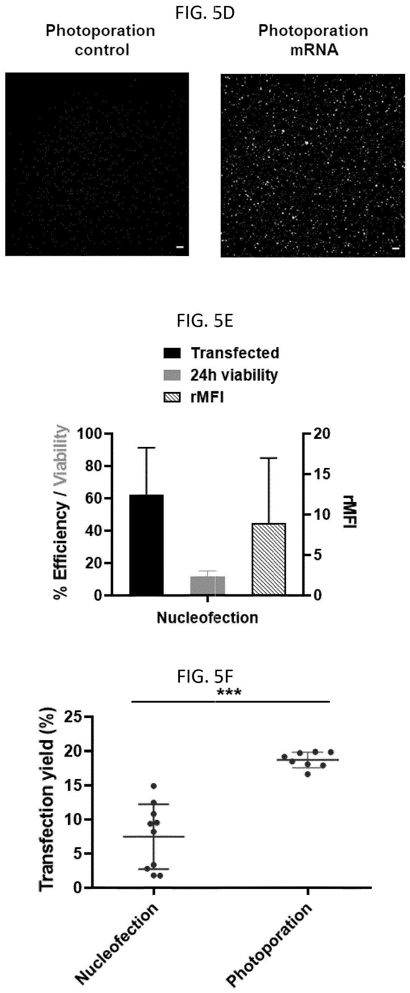

**FIG. 5:** Methods according to embodiments of the invention for delivery of FD500 and eGFP-mRNA into human T cells by the mixing photoporation protocol with PD-BSA NPs. (A) Graph representing the delivery efficiency (left y-axis, black bars) and relative mean fluorescence intensity (rMFI) (right y-axis, bars with stripes) of FD500 into human T cells after photoporation for an increasing concentration of PD-BSA NPs. The corresponding viability was measured after 24 hours with the CellTiter Glo assay (left y-axis, light grey dots). UTC: untreated control, Ctrl: mock FD500 control, Photo ctrl: Mock photoporation control. (B) Representative confocal microscopy images of FD500 'loaded' human T cells without (I) and with (II-IV) photoporation for a PD-BSA NP concentration of respectively (II) $250\times10^7$ NPs/mL, (III) $500\times10^7$ NPs/mL and (IV) $1000\times10^7$ NPs/mL (scale bar = 100 $\mu$m). (C) Graph representing the delivery efficiency for eGFP-mRNA (left y-axis, black bars) and corresponding relative mean fluorescence intensity (right y-axis, bars with stripes) after photoporation with an increasing concentration of PD-BSA NPs. The corresponding viability was measured after 24 hours with the CellTiter Glo assay (left y-axis, light grey dots). UTC: untreated control, Ctrl: mock mRNA control, Photo ctrl: mock photoporation control. (D) Confocal microscopy images for photoporation without mRNA encoding for eGFP (photoporation control) and with mRNA encoding for eGFP (photoporation mRNA) into human T cells (PD-BSA NP concentration of respectively $250 \times 10^7$ NPs/mL). scale bar = 100 $\mu$m. (E) Graph representing the delivery efficiency for eGFP-mRNA (left y-axis, black bars) and corresponding relative mean fluorescence intensity (right y-axis, bars with stripes) after nucleofection of human T cells. The corresponding viability was measured after 24 hours with the metabolic Glo assay (left y-axis, light grey bars). (F) Comparison of the transfection yield (right y-axis) for respectively nucleofection and PD500-BSA NP-sensitized photoporation. Data are shown as mean $\pm$ SD. Statistical significance: Student's t-test, *** p $\leq$ 0.001.

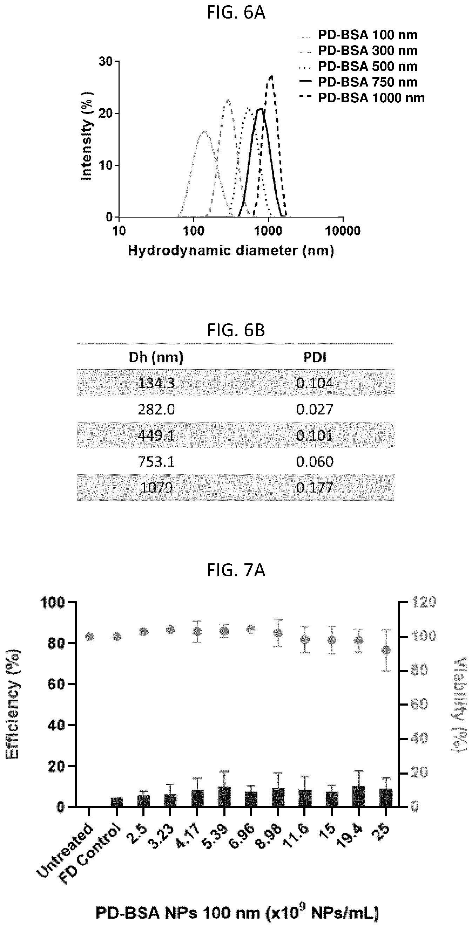

**FIG. 6:** Physicochemical characterization of PD-BSA NPs with different sizes. (A) Graph representing the hydrodynamic diameter of PD-BSA NPs with different sizes as measured in Hyclone water by DLS. (B) Table representing the hydrodynamic diameter of PD-BSA NPs with different sizes as measured in Hyclone water by DLS and polydispersity index.

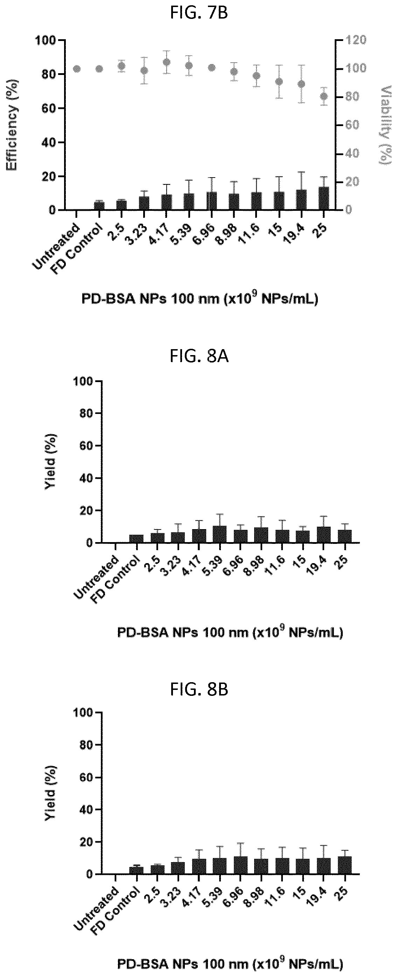

**FIG. 7:** Photoporation of HeLa cells with PD-BSA sensitizers with a hydrodynamic diameter of 100 nm. (A) Graph representing the delivery efficiency of FD500 (left y-axis, dark grey bars) and viability (2 hours, right y-axis, light grey dots) using the intensity setting of 5. (B) Graph representing the delivery efficiency of FD500 (left y-axis, dark grey bars) and viability (2 hours, right y-axis, light grey dots) using the intensity setting of 7.

**FIG. 8:** Graphs representing the yield percentage of viable FD500 positive cells for 100 nm PD-BSA NPs. (A) Graph representing the yield percentage of viable FD500 positive cells using the intensity setting of 5. (B) Graph representing the yield percentage of viable FD500 positive cells using the intensity setting of 7. The maximum yield in FIG. 8A = 11%; the maximum yield in FIG. 8B= 11%.

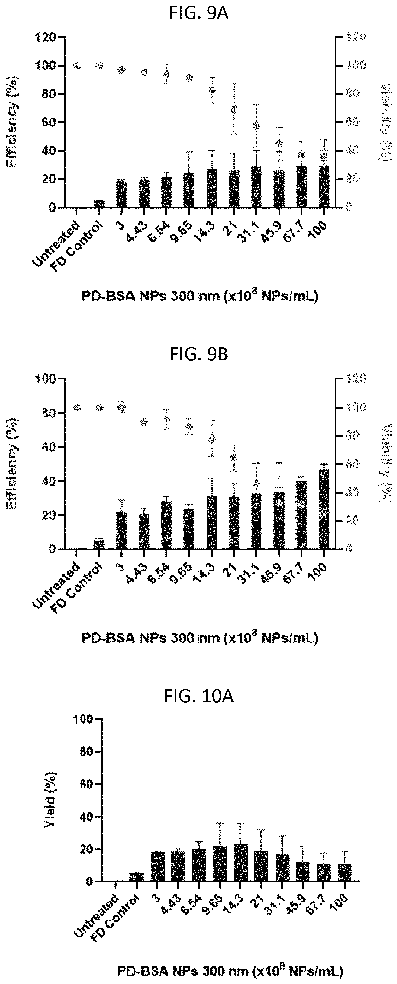

**FIG. 9:** Photoporation of HeLa cells with PD-BSA sensitizers with a hydrodynamic diameter of 300 nm. (A) Graph representing the delivery efficiency of FD500 (left y-axis, dark grey bars) and viability (2 hours, right y-axis, light grey dots) using the intensity setting of 5. (B) Graph representing the delivery efficiency of FD500 (left y-axis, dark grey bars) and viability (2 hours, right y-axis, light grey dots) using the intensity setting of 7.

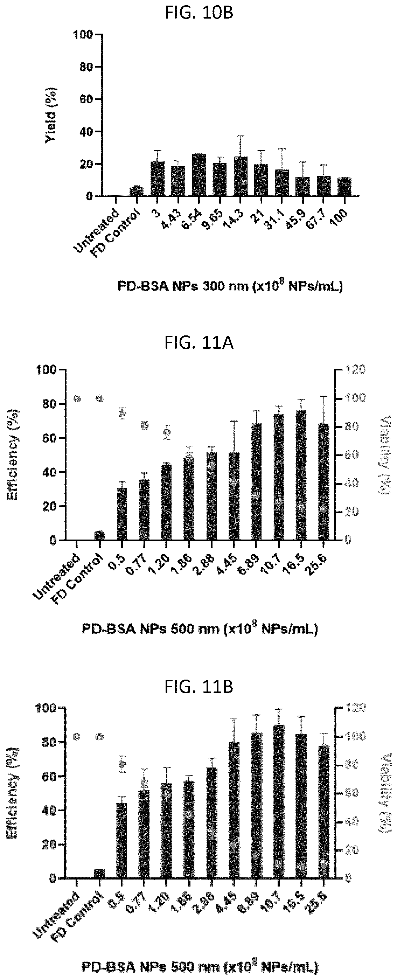

**FIG. 10:** Graphs representing the yield percentage of viable FD500 positive cells for 300 nm PD-BSA NPs. (A) Graph representing the yield percentage of viable FD500 positive cells using the intensity setting of 5. (B) Graph representing the yield percentage of viable FD500 positive cells using the intensity setting of 7. The maximum yield in FIG. 10A = 23%; the maximum yield in FIG. 10B= 26%.

**FIG. 11:** Photoporation of HeLa cells with PD-BSA sensitizers with a hydrodynamic diameter of 500 nm. (A) Graph representing the delivery efficiency of FD500 (left y-axis, dark grey bars) and viability (2 hours, right y-axis, light grey dots) using the intensity setting of 3. (B) Graph representing the delivery efficiency of FD500 (left y-axis, dark grey bars) and viability (2 hours, right y-axis, light grey dots) using the intensity setting of 5.

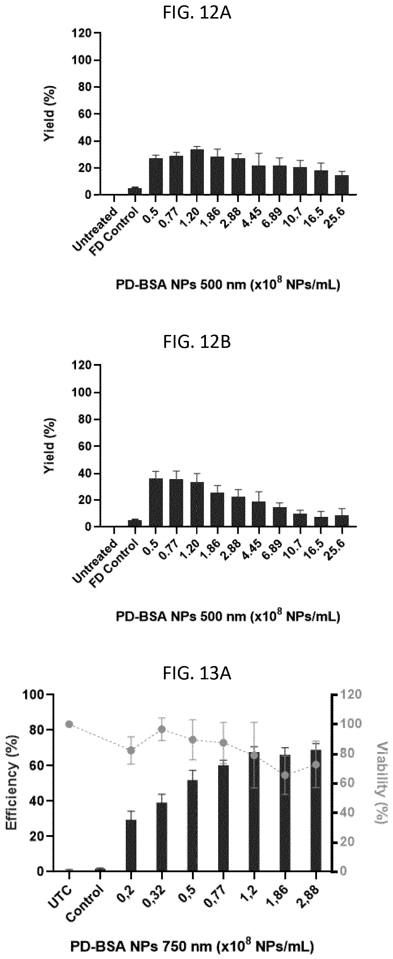

**FIG. 12:** Graphs representing the yield percentage of viable FD500 positive cells for 500 nm PD-BSA NPs. (A) Graph representing the yield percentage of viable FD500 positive cells using the intensity setting of 3. (B) Graph representing the yield percentage of viable FD500 positive cells using the intensity setting of 5. The maximum yield in FIG. 12A = 34%; the maximum yield in FIG. 12B= 36%.

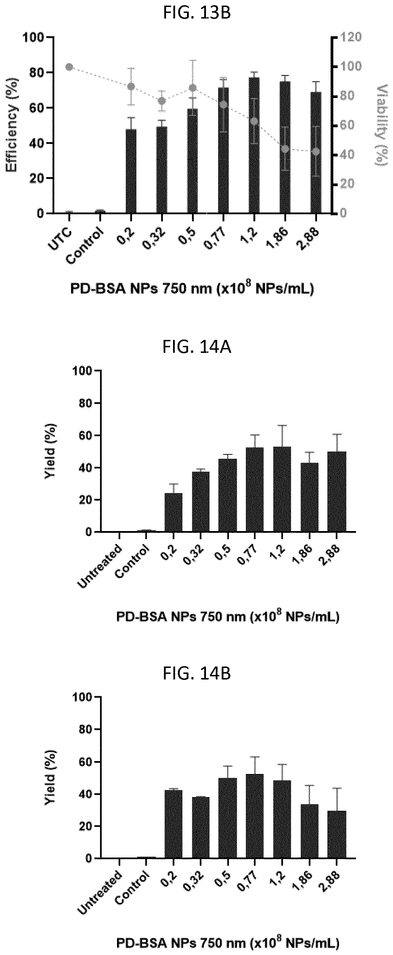

**FIG. 13:** Photoporation of HeLa cells with PD-BSA sensitizers with a hydrodynamic diameter of 750 nm. (A) Graph representing the delivery efficiency of FD500 (left y-axis, dark grey bars) and viability (2 hours, right y-axis, light grey dots) using the intensity setting of 3. (B) Graph representing the delivery efficiency of FD500 (left y-axis, dark grey bars) and viability (2 hours, right y-axis, light grey dots) using the intensity setting of 5.

**FIG. 14:** Graphs representing the yield percentage of viable FD500 positive cells for 750 nm PD-BSA NPs. (A) Graph representing the yield percentage of viable FD500 positive cells using the intensity setting of 3. (B) Graph representing the yield percentage of viable FD500 positive cells using the intensity setting of 5. The maximum yield in FIG. 14A = 53%; the maximum yield in FIG. 14B= 53%.

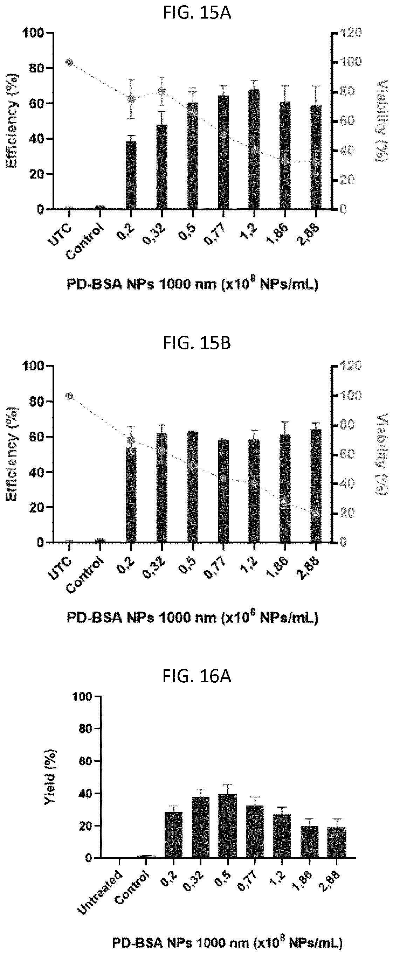

**FIG. 15:** Photoporation of HeLa cells with PD-BSA sensitizers with a hydrodynamic diameter of 1000 nm. (A) Graph representing the delivery efficiency of FD500 (left y-axis, dark grey bars) and viability (2 hours, right y-axis, light grey dots) using the intensity setting of 3. (B) Graph representing the delivery efficiency of FD500 (left y-axis, dark grey bars) and viability (2 hours, right y-axis, light grey dots) using the intensity setting of 4.

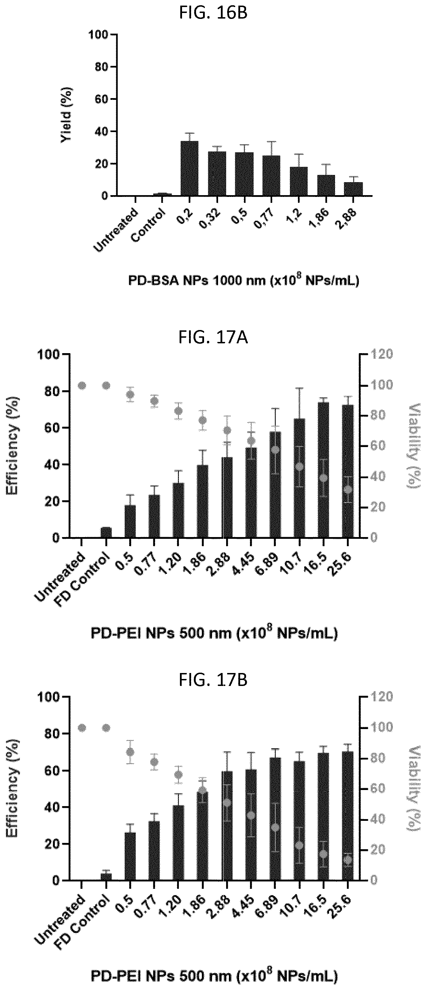

**FIG. 16:** Graph representing the yield percentage of viable FD500 positive cells for 1000 nm PD-BSA NPs. (A) Graph representing the yield percentage of viable FD500 positive cells using the intensity setting of 3. (B) Graph representing the yield percentage of viable FD500 positive cells using the intensity setting of 4. The maximum yield in FIG. 16A = 39%; the maximum yield in FIG. 16B= 38%.

**FIG. 17:** Photoporation of HeLa cells with PD-PEI sensitizers with a hydrodynamic diameter of 500 nm. (A) Graph representing the delivery efficiency of FD500 (left y-axis, dark grey bars) and viability (2 hours, right y-axis, light grey dots) using the intensity setting of 3. (B) Graph representing the delivery efficiency of FD500 (left y-axis, dark grey bars) and viability (2 hours, right y-axis, light grey dots) using the intensity setting of 5.

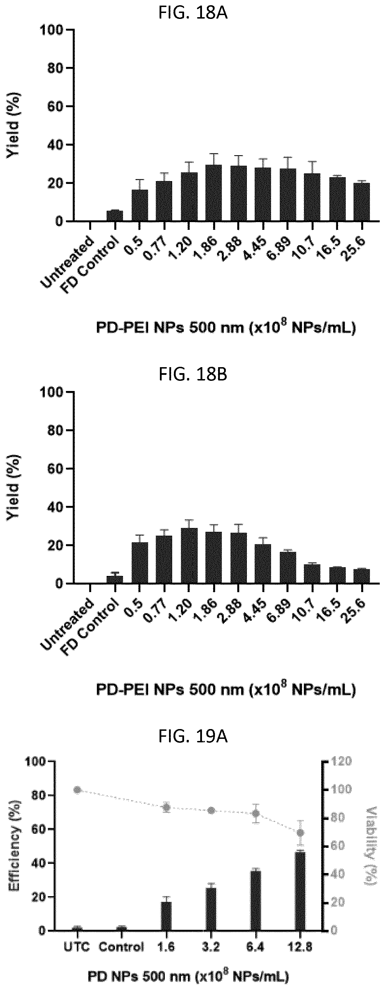

**FIG. 18:** Graphs representing the yield percentage of viable FD500 positive cells for 500 nm PD-PEI NPs. (A) Graph representing the yield percentage of viable FD500 positive cells using the intensity setting of 3. (B) Graph representing the yield percentage of viable FD500 positive cells using the intensity setting of 5. The maximum yield in FIG. 18A = 30%; the maximum yield in FIG. 18B= 29%.

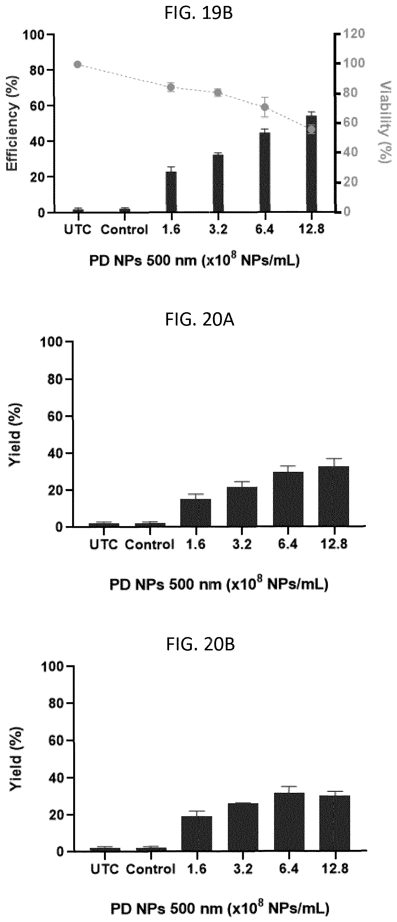

**FIG. 19:** Photoporation of HeLa cells with uncoated PD particles with a hydrodynamic diameter of 500 nm. (A) Graph representing the delivery efficiency of FD500 (left y-axis, dark grey bars) and viability (2 hours, right y-axis, light grey dots) using the intensity setting of 1. (B) Graph representing the delivery efficiency of FD500 (left y-axis, dark grey bars) and viability (2 hours, right y-axis, light grey dots) using the intensity setting of 2.

**FIG. 20:** Graphs representing the yield percentage of viable FD500 positive cells for 500 nm PD NPs. (A) Graph representing the yield percentage of viable FD500 positive cells using the intensity setting of 1. (B) Graph representing the yield percentage of viable FD500 positive cells using the intensity setting of 2. The maximum yield in FIG. 20A = 33%; the maximum yield in FIG. 20B= 32%.

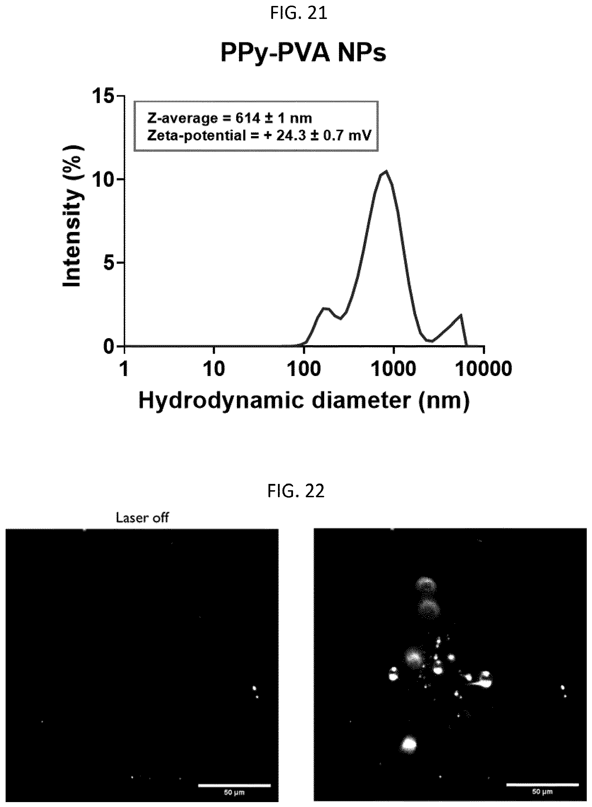

**FIG. 21:** Physicochemical characterization of PVA-coated polypyrrole NPs. Graph and table showing the hydrodynamic diameter and zeta-potential of PPy NPs as measured in Hyclone water by DLS.

**FIG. 22:** Dark field microscopy images of polypyrrole nanoparticles before (left image) and after (right image) the application of a laser pulse (7 ns pulse duration, 561 nm wavelength, 1.1 J/cm$^2$) (scale bar = 50 $\mu$m) showing the formation of laser-induced VNB.

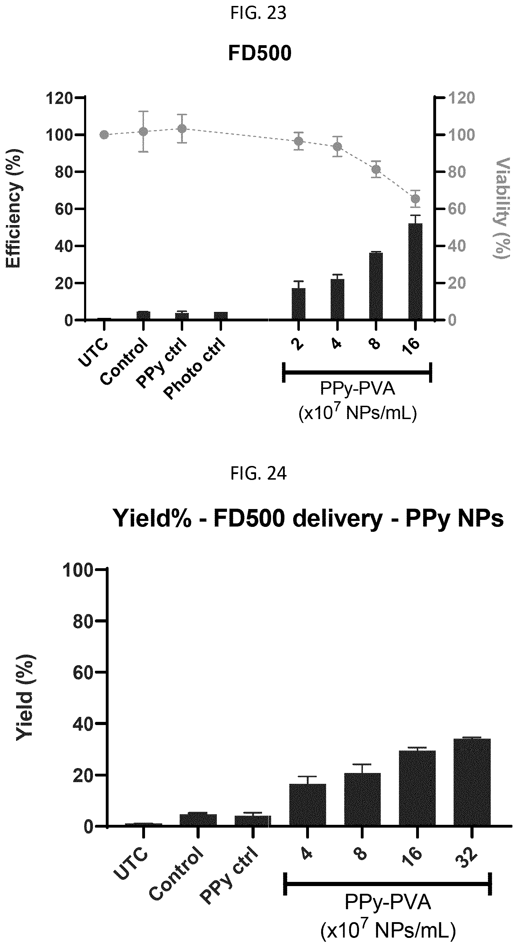

**FIG. 23:** Graph representing the delivery efficiency of 500 kDa FITC-dextran (FD500) (left y-axis, dark grey bars) and cell viability (2 hours after photoporation, right y-axis, light grey bars) via the mixing method in adherent HeLa cells. UTC: untreated control, Ctrl: FD500 incubation control, PPy ctrl: PPy NPs and FD500 incubation control, Photo ctrl: photoporation control with PPy NPs/mL (16x107 NPs/mL) and without FD500.

**FIG. 24:** Graphs representing the yield percentage of viable FD500 positive cells for PPy-sensitized photoporation.

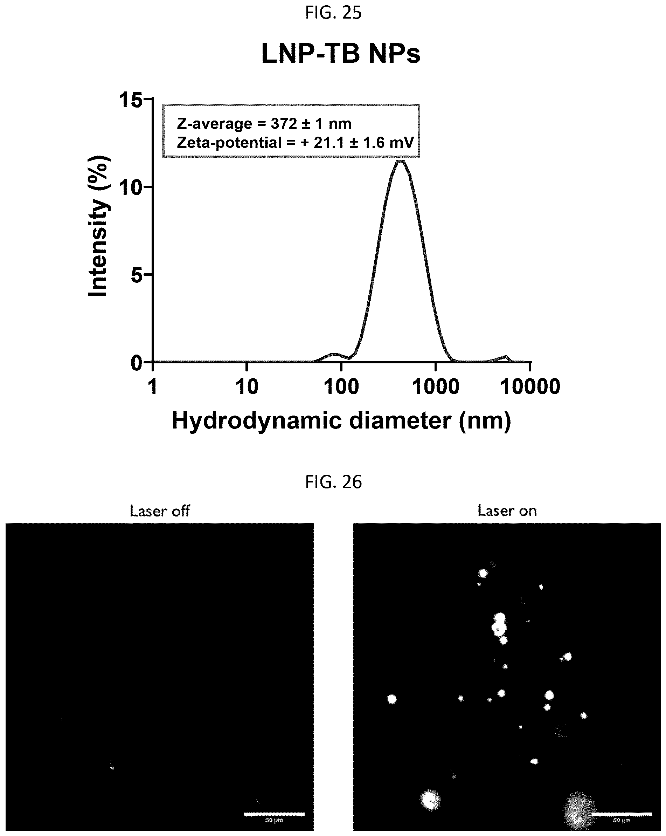

**FIG. 25:** Physicochemical characterization of trypan blue-loaded lipid nanoparticles as measured in Hyclone water. Graph and table showing the hydrodynamic diameter and zeta-potential of LNP-TB NPs as measured in Hyclone water by DLS.

**FIG. 26** : Dark field microscopy images of trypan blue-loaded lipid nanoparticles before (left image) and after (right image) the application of a laser pulse (7 ns pulse duration, 561 nm wavelength, 3.15 J/cm$^2$) (scale bar = 50 $\mu$m) showing the formation of laser-induced VNB.

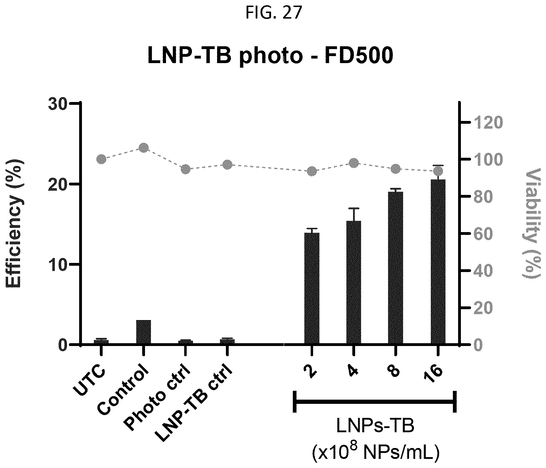

**FIG. 27:** Graph representing the delivery efficiency of 500 kDa FITC-dextran (FD500) (left y-axis, dark grey bars) and cell viability (2 hours after photoporation, right y-axis, light grey bars) via the mixing method in adherent HeLa cells. UTC: untreated control, Control: FD500 incubation control, LNP-TB ctrl: LNP-TB NPs and FD500 incubation control, Photo ctrl: photoporation control with LNP-TB NPs/mL (16x108 NPs/mL) and without FD500.

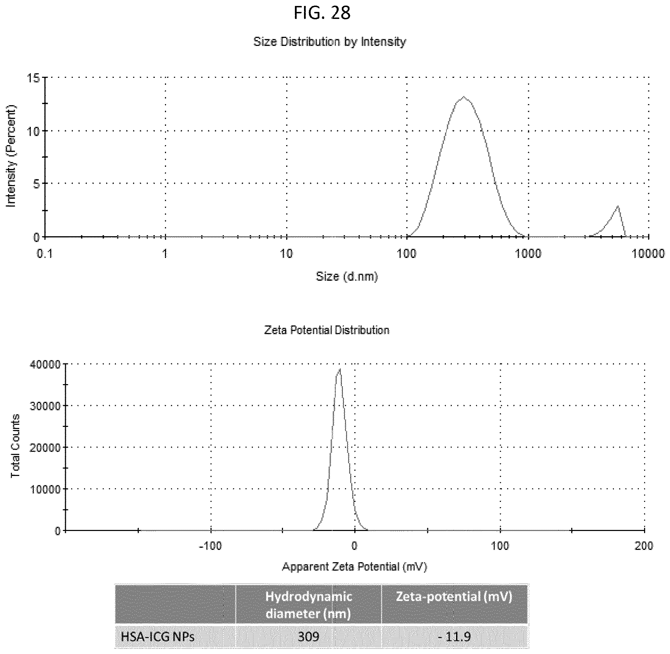

**FIG. 28:** Physicochemical characterization of Human serum albumin ICG nanoparticles (HSA-ICG NPs) as measured in distilled water. Graph and table showing the hydrodynamic diameter and zeta-potential of HSA-ICG NPs as measured in water by DLS.

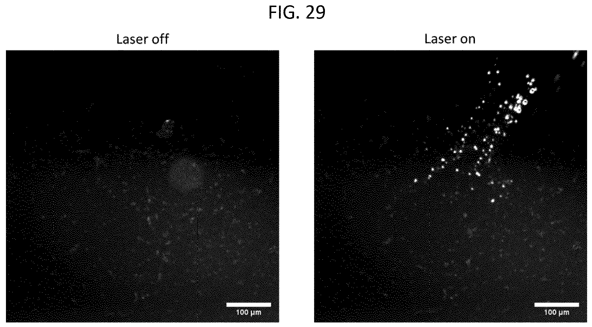

**FIG. 29:** Dark field microscopy images of HSA-ICG NPs before (left) and after (right) the application of a laser pulse (7 ns pulse duration, 561 nm wavelength, 4.5 J/cm$^2$) (scale bar = 100 $\mu$m) showing the formation of laser-induced VNB.

## DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any $\geq 3$, $\geq 4$, $\geq 5$, $\geq 6$ or $\geq 7$ etc. of said members, and up to all said members.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

Unless indicated otherwise, the references to "use" or "uses" as taught herein include a medical use or medical method.

By extensive experiment testing, the present inventors have found that photoresponsive organic particles selected from the group consisting of a polymer-based particle, a protein-based particle, a lipid-based particle (e.g. a liposome or a solid lipid particle), and a combination thereof allow to deliver efficiently and safely a cargo into cells, for instance mRNA into human T cells.

Accordingly, a first aspect of the invention relates to an *in vitro* or *ex vivo* method for delivering a cargo into a cell, the method comprising:

- contacting a cell with one or more photoresponsive organic particles, wherein the organic particle is selected from the group consisting of a polymer-based particle, a protein-based particle, a lipid-based particle, and a combination thereof, thereby obtaining a mixture of the cell and the one or more photoresponsive organic particles;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with a cargo, wherein the cargo is not bound to the one or more photoresponsive organic particles, thereby delivering the cargo into the cell; or

- contacting a cell with one or more photoresponsive organic particles and a cargo, wherein the cargo is not bound to the one or more photoresponsive organic particles, and wherein the organic particle is selected from the group consisting of a polymer-based particle, a protein-based particle, a lipid-based particle, and a combination thereof, thereby obtaining a mixture of the cell, the cargo, and the one or more photoresponsive organic particles; and

- irradiating the mixture of the cell, the cargo, and the one or more photoresponsive organic particles with electromagnetic radiation, thereby causing permeabilization of a barrier of the cell and delivering the cargo into the cell.

As indicated above, the methods as taught herein may be *in vitro* or *ex vivo* methods. The term *"in vitro"* as used herein is to denote outside, or external to, animal or human body. The term *"in vitro"* as used herein should be understood to include *"ex vivo".* The term *"ex vivo"* typically refers to tissues or cells removed from an animal or human body and maintained or propagated outside the body, e.g. in a culture vessel.

Alternatively, the methods as taught herein may be *in vivo* methods. The term *"in vivo"* as used herein is to denote inside, or internal to, animal or human body.

The recitation "delivering a cargo into a cell" as used herein refers to bringing (or providing or introducing) a cargo into a cell.

In embodiments, the cargo as taught herein is not bound to the one or more photoresponsive organic particles. In other words, in embodiments, the cargo and the one or more photoresponsive organic particles are not bound to each other. Hence, in embodiments of the methods as taught herein, the cargo and the one or more photoresponsive organic particles are added separately to the mixture of the cell, the cargo, and the one or more photoresponsive organic particles.

The terms "not bound" or "unbound" may be used interchangeably herein and denotes that a first component is not combined with or chemically bonded to a second component. For instance, the first component is not bound to the second component by a covalent binding or by a non-covalent interaction such as an electrostatic or hydrophobic interaction. The phrase "the cargo is not bound to the one or more photoresponsive organic particles" denotes that the cargo is not combined with or chemically bonded to the one or more photoresponsive organic particles, e.g. the cargo is not coupled (e.g. grafted) to or enclosed (e.g. encapsulated) in the one or more photoresponsive organic particles. For instance, the cargo is not bound to the one or more photoresponsive organic particles by a covalent binding or by a non-covalent interaction such as an electrostatic or hydrophobic interaction.

In embodiments, the cargo as taught herein is not covalently bound to the one or more photoresponsive organic particles. In other words, in embodiments, the cargo and the one or more photoresponsive organic particles are not covalently bound to each other.

As used herein, the terms "cargo" or "agent" broadly refer to any chemical (e.g., inorganic or organic), biochemical or biological substance, molecule or macromolecule (e.g., biological macromolecule), particle (e.g. a nanoparticle), a combination or mixture thereof, a sample of undetermined composition, or an extract made from biological materials such as bacteria, plants, fungi, or animal cells or tissues. Preferred "cargos" or "agents" include nucleic acids, oligonucleotides, ribozymes, proteins, polypeptides, peptides, peptidomimetics, peptide nucleic acids, antibodies, antibody fragments, antibody-like protein scaffolds, aptamers, photoaptamers, spiegelmers, chemical substances, lipids, carbohydrates, polysaccharides, etc., and any combinations thereof such as gene editing system, e.g. CRISPR/Cas. Depending on the context, the term "agent" may denote a "therapeutic agent" or "drug", useful for or used in the treatment, cure, prevention, or diagnosis of a disease. The cargo as taught herein includes but is not limited to a cargo in solution, and a dried or lyophilized cargo, such as a powder of the cargo.

In embodiments, the cargo may comprise or consist of two or more agents combined with or chemically bonded to each other. In embodiments, the cargo may comprise or consist of two or more agents conjugated (e.g. covalently bound) to each other. For instance, the cargo may be an agent comprising or consisting of two, three, four, five, six or more agents conjugated (e.g. covalently bound) to each other.

In embodiments, the cargo as taught herein may be bound to a particle such as a nanoparticle (which is not the photoresponsive organic particle). In embodiments, the cargo may be combined with or chemically bonded to a particle such as a nanoparticle, e.g. the cargo may be coupled (e.g. grafted) to or enclosed (e.g. encapsulated) in a particle such as a nanoparticle.

The term "nucleic acid" as used herein typically refers to a polymer (preferably a linear polymer) of any length composed essentially of nucleoside units. A nucleoside unit commonly includes a heterocyclic base and a sugar group. Heterocyclic bases may include *inter alia* purine and pyrimidine bases such as adenine (A), guanine (G), cytosine (C), thymine (T) and uracil (U) which are widespread in naturally-occurring nucleic acids, other naturally-occurring bases (e.g., xanthine, inosine, hypoxanthine) as well as chemically or biochemically modified (e.g., methylated), non-natural or derivatised bases. Exemplary modified nucleobases include without limitation 5-substituted pyrimidines, 6-azapyrimidines and N-2, N-6 and O-6 substituted purines, including 2-aminopropyladenine, 5- propynyluracil and 5-propynylcytosine. In particular, 5-methylcytosine substitutions have been shown to increase nucleic acid duplex stability and may be preferred base substitutions in for example antisense agents, even more particularly when combined with 2'-O-methoxyethyl sugar modifications. Sugar groups may include *inter alia* pentose (pentofuranose) groups such as preferably ribose and/or 2-deoxyribose common in naturally-occurring nucleic acids, or arabinose, 2-deoxyarabinose, threose or hexose sugar groups, as well as modified or substituted sugar groups (such as without limitation 2'-O-alkylated, e.g., 2'-O-methylated or 2'-O-ethylated sugars such as ribose; 2'-O-alkyloxyalkylated, e.g., 2'-O-methoxyethylated sugars such as ribose; or 2'-O,4'-C-alkylene-linked, e.g., 2'-O,4'-C-methylene-linked or 2'-O,4'-C-ethylene-linked sugars such as ribose; 2'-fluoro-arabinose, *etc.*). Nucleic acid molecules comprising at least one ribonucleoside unit may be typically referred to as ribonucleic acids or RNA. Such ribonucleoside unit(s) comprise a 2'-OH moiety, wherein -H may be substituted as known in the art for ribonucleosides (e.g., by a methyl, ethyl, alkyl, or alkyloxyalkyl). Preferably, ribonucleic acids or RNA may be composed primarily of ribonucleoside units, for example, $\geq 80\%$, $\geq 85\%$, $\geq 90\%$, $\geq 95\%$, $\geq 96\%$, $\geq 97\%$, $\geq 98\%$, $\geq 99\%$ or even 100% (by number) of nucleoside units constituting the nucleic acid molecule may be ribonucleoside units. Nucleic acid molecules comprising at least one deoxyribonucleoside unit may be typically referred to as deoxyribonucleic acids or DNA. Such deoxyribonucleoside unit(s) comprise 2'-H. Preferably, deoxyribonucleic acids or DNA may be composed primarily of deoxyribonucleoside units, for example, $\geq 80\%$, $\geq 85\%$, $\geq 90\%$, $\geq 95\%$, $\geq 96\%$, $\geq 97\%$, $\geq 98\%$, $\geq 99\%$ or even 100% (by number) of nucleoside units constituting the nucleic acid molecule may be deoxyribonucleoside units. Nucleoside units may be linked to one another by any one of numerous known inter-nucleoside linkages, including *inter alia* phosphodiester linkages common in naturally-occurring nucleic acids, and further modified phosphate- or phosphonate-based linkages such as phosphorothioate, alkyl phosphorothioate such as methyl phosphorothioate, phosphorodithioate, alkylphosphonate such as methylphosphonate, alkylphosphonothioate, phosphotriester such as alkylphosphotriester, phosphoramidate, phosphoropiperazidate, phosphoromorpholidate, bridged phosphoramidate, bridged methylene phosphonate, bridged phosphorothioate; and further siloxane, carbonate, sulfamate, carboalkoxy, acetamidate, carbamate such as 3'-N-carbamate, morpholino, borano, thioether, 3'-thioacetal, and sulfone internucleoside linkages. Preferably, inter-nucleoside linkages may be phosphate-based linkages including modified phosphate-based linkages, such as more preferably phosphodiester, phosphorothioate or phosphorodithioate linkages or combinations thereof. The term "nucleic acid" also encompasses any other nucleobase containing polymers such as nucleic acid mimetics, including, without limitation, peptide nucleic acids (PNA), peptide nucleic acids with phosphate groups (PHONA), locked nucleic acids (LNA), morpholino phosphorodiamidate-backbone nucleic acids (PMO), cyclohexene nucleic acids (CeNA), tricyclo-DNA (tcDNA), and nucleic acids having backbone sections with alkyl linkers or amino linkers (see, e.g., Kurreck 2003 (Eur J Biochem 270: 1628-1644)). "Alkyl" as used herein particularly encompasses lower hydrocarbon moieties, e.g., C1-C4 linear or branched, saturated or unsaturated hydrocarbon, such as methyl, ethyl, ethenyl, propyl, 1-propenyl, 2-propenyl, and isopropyl. Nucleic acids as intended herein may include naturally occurring nucleosides, modified nucleosides or mixtures thereof. A modified nucleoside may include a modified heterocyclic base, a modified sugar moiety, a modified inter-nucleoside linkage or a combination thereof.

The term "nucleic acid" preferably encompasses DNA, RNA and DNA/RNA hybrid molecules, specifically including hnRNA, pre-mRNA, mRNA, cDNA, genomic DNA (gDNA), plasmid DNA (pDNA), amplification products, oligonucleotides, and synthetic (e.g., chemically synthesised) DNA, RNA or DNA/RNA hybrids. RNA is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA). A nucleic acid can be naturally occurring, e.g., present in or isolated from nature, can be recombinant, i.e., produced by recombinant DNA technology, and/or can be, partly or entirely, chemically or biochemically synthesised. A "nucleic acid" can be double-stranded, partly double stranded, or single-stranded. Where single-stranded, the nucleic acid can be the sense strand or the antisense strand. In addition, nucleic acid can be circular or linear.

The term "oligonucleotide" as used throughout this specification refers to a nucleic acid (including nucleic acid analogues and mimetics) oligomer or polymer as defined herein. Preferably, an oligonucleotide, such as more particularly an antisense oligonucleotide, is (substantially) single-stranded. Oligonucleotides as intended herein may have a length of about 10 to about 100 nucleoside units (i.e., nucleotides or nucleotide analogues), preferably about 15 to about 50, more preferably about 20 to about 40, also preferably about 20 to about 30 nucleoside units (i.e., nucleotides or nucleotide analogues). Oligonucleotides as intended herein may comprise one or more or all non-naturally occurring heterocyclic bases and/or one or more or all non-naturally occurring sugar groups and/or one or more or all non-naturally occurring inter-nucleoside linkages, the inclusion of which may improve properties such as, for example, increased stability in the presence of nucleases and increased hybridization affinity, increased tolerance for mismatches, etc. Nucleic acid binding agents, such as oligonucleotide binding agents, are typically at least partly antisense to a target nucleic acid of interest. The term "antisense" generally refers to an agent (e.g., an oligonucleotide) configured to specifically anneal with (hybridize to) a given sequence in a target nucleic acid, such as for example in a target DNA, hnRNA, pre-mRNA or mRNA, and typically comprises, consist essentially of or consist of a nucleic acid sequence that is complementary or substantially complementary to said target nucleic acid sequence. Antisense agents suitable for use herein, such as hybridization probes or amplification or sequencing primers and primer pairs) may typically be capable of annealing with (hybridizing to) the respective target nucleic acid sequences at high stringency conditions, and capable of hybridizing specifically to the target under physiological conditions. The terms "complementary" or "complementarity" as used throughout this specification with reference to nucleic acids, refer to the normal binding of single-stranded nucleic acids under permissive salt (ionic strength) and temperature conditions by base pairing, preferably Watson-Crick base pairing. By means of example, complementary Watson-Crick base pairing occurs between the bases A and T, A and U or G and C. For example, the sequence 5'-A-G-U-3' is complementary to sequence 5'-A-C-U-3'.

The reference to oligonucleotides may in particular but without limitation include hybridization probes and/or amplification primers and/or sequencing primers, etc., as commonly used in nucleic acid detection technologies.

The terms "ribozyme" or "ribonucleic acid enzymes" as used herein refer to RNA molecules that have the ability to catalyse specific biochemical reactions, for example RNA splicing in gene expression. The function of ribozymes is similar to the action of protein enzymes. The most common activities of ribozymes are the cleavage or ligation of RNA and DNA and peptide bond formation. Within the ribosome, ribozymes function as part of the large subunit ribosomal RNA to link amino acids during protein synthesis. They also participate in a variety of RNA processing reactions, including RNA splicing, viral replication, and transfer RNA biosynthesis. Examples of ribozymes include the hammerhead ribozyme, the VS ribozyme, Leadzyme and the hairpin ribozyme.

The term "protein" as used herein generally encompasses macromolecules comprising one or more polypeptide chains, i.e., polymeric chains of amino acid residues linked by peptide bonds. The term may encompass naturally, recombinantly, semi-synthetically or synthetically produced proteins. The term also encompasses proteins that carry one or more co- or post-expression-type modifications of the polypeptide chain(s), such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes protein variants or mutants which carry amino acid sequence variations vis-à-vis a corresponding native protein, such as, e.g., amino acid deletions, additions and/or substitutions. The term contemplates both full-length proteins and protein parts or fragments, e.g., naturally occurring protein parts that ensue from processing of such full-length proteins.

The term "polypeptide" as used herein encompasses polymeric chains of amino acid residues linked by peptide bonds. Hence, especially when a protein is only composed of a single polypeptide chain, the terms "protein" and "polypeptide" may be used interchangeably herein to denote such a protein. The term is not limited to any minimum length of the polypeptide chain. The term may encompass naturally, recombinantly, semi-synthetically or synthetically produced polypeptides. The term also encompasses polypeptides that carry one or more co- or post-expression-type modifications of the polypeptide chain, such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes polypeptide variants or mutants which carry amino acid sequence variations vis-à-vis a corresponding native polypeptide, such as, e.g., amino acid deletions, additions and/or substitutions. The term contemplates both full-length polypeptides and polypeptide parts or fragments, e.g., naturally occurring polypeptide parts that ensue from processing of such full-length polypeptides.

The term "peptide" as used throughout this specification preferably refers to a polypeptide as used herein consisting essentially of 50 amino acids or less, e.g., 45 amino acids or less, preferably 40 amino acids or less, e.g., 35 amino acids or less, more preferably 30 amino acids or less, e.g., 25 or less, 20 or less, 15 or less, 10 or less or 5 or less amino acids.

The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134).

The term "peptide nucleic acid" or "PNA" refers to an artificially synthesized polymer comprising N-(2-aminoethyl)-glycine (AEG) units linked by peptide bonds. The various purine and pyrimidine bases are linked to the backbone by a methylene bridge (-CH$_2$-) and a carbonyl group (-(C=O)-). PNAs are depicted like peptides, with the N-terminus at the first (left) position and the C-terminus at the last (right) position.

As used herein, the term "antibody" is used in its broadest sense and generally refers to any immunologic binding agent. The term specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest, i.e., antigen-binding fragments), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced *in vitro* or *in vivo.*

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments, single domain (sd) Fv, such as VH domains, VL domains and VHH domains; diabodies; linear antibodies; single-chain antibody molecules, in particular heavy-chain antibodies; and multivalent and/or multi-specific antibodies formed from antibody fragment(s), e.g., dibodies, tribodies, and multibodies. The above designations Fab, Fab', F(ab')2, Fv, scFv etc. are intended to have their art-established meaning. The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius),* llama (e.g., *Lama paccos, Lama glama* or *Lama vicugna*) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g.,

glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

In certain embodiments, the agent may be a Nanobody®. The terms "Nanobody®" and "Nanobodies®" are trademarks of Ablynx NV (Belgium). The term "Nanobody" is well-known in the art and as used herein in its broadest sense encompasses an immunological binding agent obtained (1) by isolating the $V_{HH}$ domain of a heavy-chain antibody, preferably a heavy-chain antibody derived from camelids; (2) by expression of a nucleotide sequence encoding a $V_{HH}$ domain; (3) by "humanization" of a naturally occurring $V_{HH}$ domain or by expression of a nucleic acid encoding a such humanized $V_{HH}$ domain; (4) by "camelization" of a $V_H$ domain from any animal species, and in particular from a mammalian species, such as from a human being, or by expression of a nucleic acid encoding such a camelized $V_H$ domain; (5) by "camelization" of a "domain antibody" or "dAb" as described in the art, or by expression of a nucleic acid encoding such a camelized dAb; (6) by using synthetic or semi-synthetic techniques for preparing proteins, polypeptides or other amino acid sequences known *per se*; (7) by preparing a nucleic acid encoding a Nanobody using techniques for nucleic acid synthesis known *per se,* followed by expression of the nucleic acid thus obtained; and/or (8) by any combination of one or more of the foregoing. "Camelids" as used herein comprise old world camelids (*Camelus bactrianus* and *Camelus dromaderius*) and new world camelids (for example *Lama paccos, Lama glama* and *Lama vicugna*).

The term "antibody-like protein scaffolds" or "engineered protein scaffolds" broadly encompasses proteinaceous non-immunoglobulin specific-binding agents, typically obtained by combinatorial engineering (such as site-directed random mutagenesis in combination with phage display or other molecular selection techniques). Usually, such scaffolds are derived from robust and small soluble monomeric proteins (such as Kunitz inhibitors or lipocalins) or from a stably folded extra-membrane domain of a cell surface receptor (such as protein A, fibronectin or the ankyrin repeat). Such scaffolds have been extensively reviewed in Binz et al., Gebauer and Skerra, Gill and Damle, Skerra 2000, and Skerra 2007, and include without limitation affibodies, based on the Z-domain of staphylococcal protein A, a three-helix bundle of 58 residues providing an interface on two of its alpha-helices (Nygren); engineered Kunitz domains based on a small (ca. 58 residues) and robust, disulphide-crosslinked serine protease inhibitor, typically of human origin (e.g. LACI-D1), which can be engineered for different protease specificities (Nixon and Wood); monobodies or adnectins based on the 10th extracellular domain of human fibronectin III (10Fn3), which adopts an Ig-like beta-sandwich fold (94 residues) with 2-3 exposed loops, but lacks the central disulphide bridge (Koide and Koide); anticalins derived from the lipocalins, a diverse family of eight-stranded beta-barrel proteins (ca. 180 residues) that naturally form binding sites for small ligands by means of four structurally variable loops at the open end, which are abundant in humans, insects, and many other organisms (Skerra 2008); DARPins, designed ankyrin repeat domains (166 residues), which provide a rigid interface arising from typically three repeated beta-turns (Stumpp et al.); avimers (multimerized LDLR-A module) (Silverman et al.); and cysteine-rich knottin peptides (Kolmar). The term "aptamer" refers to single-stranded or double-stranded oligo-DNA, oligo-RNA or oligo-DNA/RNA or any analogue thereof that specifically binds to a target molecule such as a peptide. Advantageously, aptamers display fairly high specificity and affinity (e.g., $K_A$ in the order $1 \times 10^9$ M$^{-1}$) for their targets. Aptamer production is described *inter alia* in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592.

The term "photoaptamer" refers to an aptamer that contains one or more photoreactive functional groups that can covalently bind to or crosslink with a target molecule.

The term "spiegelmer" refers to an aptamer which includes L-DNA, L-RNA, or other left-handed nucleotide derivatives or nucleotide-like molecules. Aptamers containing left-handed nucleotides are resistant to degradation by naturally occurring enzymes, which normally act on substrates containing right-handed nucleotides.

In embodiments, the chemical substance is an organic molecule, preferably a small (organic) molecule. The term "small molecule" refers to compounds, preferably organic compounds, with a size comparable to those organic molecules generally used in pharmaceuticals. The term excludes biological macromolecules (e.g., proteins, peptides, nucleic acids, etc.). Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da. The chemical substance, in particular the small molecule may be an imaging agent, such as a contrast agent.

The term "lipid" as used herein refers to a macromolecule that is soluble in a nonpolar solvent. Lipids may be divided into eight categories: fatty acids; glycerolipids; glycerophospholipids; sphingolipids; saccharolipids; polyketides; sterol lipids or sterols; and prenol lipids or prenols.

The term "carbohydrate" generally refers to a biomolecule consisting of carbon (C), hydrogen (H) and oxygen (O) atoms. Usually, a carbohydrate has a hydrogen-oxygen atom ratio of 2:1 (as in water) and thus the empirical formula $C_m(H_2O)_n$ (where m may or may not be different from n). Carbohydrates encompass polyhydroxy aldehydes, ketones, alcohols, acids, their simple derivatives and their polymers having linkages of the acetal type. They may be classified according to their degree of polymerization, and may be divided initially into three principal groups, namely sugars, oligosaccharides and polysaccharides.

The term "polysaccharide" generally refers to a polymer or macromolecule consisting of monosaccharide units joined together by glycosidic bonds. Polysaccharides may be linear or branched.

The term "gene editing system" or "genome editing system" as used herein refers to a tool to induce one or more nucleic acid modifications, such as DNA or RNA modifications, into a specific DNA or RNA sequence within a cell. Targeted genome modification is a powerful tool for genetic manipulation of cells and organisms, including mammals. Genome modification or gene editing, including insertion, deletion or replacement of DNA in the genome, can be carried out using a variety of known gene editing systems. Gene editing systems typically make use of an agent capable of inducing a nucleic acid modification. In certain embodiments, the agent capable of inducing a nucleic acid modification may be a (endo) nuclease or a variant thereof having altered or modified activity. (endo)Nucleases typically comprise programmable, sequence-specific DNA- or RNA-binding modules linked to a nonspecific DNA or RNA cleavage domain. In DNA, these nucleases create site-specific double-strand breaks at desired locations in the genome. The induced double-stranded breaks are repaired through nonhomologous end-joining or homologous recombination, resulting in targeted mutations. In certain embodiments, said (endo)nuclease may be RNA-guided. In certain embodiments, said (endo)nuclease can be engineered nuclease such as a Clustered Regularly Interspaced Short Palindromic Repeats (CRISPR) associated (Cas) (endo)nuclease, such as Cas9, Cpf1, or C2c2, a (zinc finger nuclease (ZFN),a transcription factor-like effector nuclease (TALEN), a meganuclease, or modifications thereof. Methods for using TALEN technology, Zinc Finger technology and CRISPR/Cas technology are known by the skilled person.

In embodiments of the methods as taught herein (including the *in vitro* methods as taught herein and the *in vivo* methods or uses as taught herein), the cargo may be selected from the group consisting of a nucleic acid, a protein, a chemical substance, a polysaccharide, and combinations thereof, such as a gene editing system e.g. CRISPR/Cas system. Such cargos can be efficiently delivered into the cells by contacting the cells with the cargo and the photoresponsive organic particles as taught herein, and irradiating the mixture of the cells, the cargo, and the photoresponsive organic particles with electromagnetic radiation.

In embodiments of the methods as taught herein, the cargo may be a nucleic acid. In embodiments of the methods as taught herein, the cargo may be mRNA or plasmid DNA. Using nucleic acids such as mRNA or plasmid DNA as a cargo advantageously allows the delivery of desired genetic constructs into cells. Using mRNA as a cargo allows transient expression of a construct into cells without the need for genomic integration, thereby providing control over the duration of expression and avoiding any risks for unintended mutations by the genomic integration.

In embodiments, the cargo may be a nucleic acid, such as mRNA or plasmid DNA, having a size of at least 0.5 kilobase (kb). For example, the cargo may be a nucleic acid, such as mRNA or plasmid DNA, having a size of at least 0.6 kb, at least 0.7 kb, at least 0.8 kb, at least 0.9 kb, at least 1.0 kb, at least 1.5 kb, at least 2.0 kb, or more. For example, the cargo may be a nucleic acid, such as mRNA or plasmid DNA, having a size of at least 3.0 kb, at least 4.0 kb, at least 5.0 kb, at least 6.0 kb, at least 7.0 kb, at least 8.0 kb, at least 9.0 kb, at least 10.0 kb, or more. Such (large) nucleic acids can be efficiently delivery into cells by the present methods.

In embodiments of the methods as taught herein, the cargo may be a protein. The present methods advantageously allow the delivery of desired proteins into cells.

In embodiments, the cargo may be a negatively charged protein at physiological pH (e.g. pH of about 6 to about 8). (IEP). In embodiments, the cargo may be a neutral protein at physiological pH (e.g. pH of about 6 to about 8). The present methods advantageously allow efficient delivery of such proteins into the cells.

In embodiments of the methods as taught herein, the cargo may be a chemical substance. The present methods advantageously allow the delivery of desired chemical substances into cells.

In embodiments of the methods as taught herein, the cargo may be a molecular contrast agent. For instance, the cargo may be molecular contrast agent such as a gadolinium chelate, a fluorophore, or a chromophore.

In embodiments of the methods as taught herein, the cargo may be a polysaccharide. The present methods advantageously allow the delivery of desired polysaccharides into cells.

In embodiments of the methods as taught herein, the cargo may be a particle such as a nanoparticle, e.g. a luminescent or sensing nanoparticle. For instance, the cargo may be a luminescent or sensing nanoparticle such as a superparamagnetic iron-oxide nanoparticle, a plasmonic nanoparticle, e.g. gold nanoparticle, a quantum dot, an upconverting nanoparticle, a phosphorescent nanoparticle, a persistent luminescent nanoparticle, or a carbon dot.

In embodiments, the cargo may be a protein, a chemical substance, a polysaccharide, or combination thereof having a size of at least 200 kDa. In embodiments, the cargo may be a protein, a polysaccharide, a chemical substance, or combination thereof having a size of at least 250 kDa, at least 300 kDa, at least 350 kDa, at least 400 kDa, at least 450 kDa, at least 450 kDa, at least 500 kDa, at least 600 kDa, at least 700 kDa, at least 800 kDa at least 900 kDa, or at least 1000 kDa. In embodiments, the cargo may be a protein, a polysaccharide, a chemical substance, or combination thereof having a size of about 250 kDa to about 3500 kDa, about 300 kDa to about 3000 kDa, about 350 kDa to about 2500 kDa, about 400 kDa to about 2000 kDa, about 450 kDa to about 1500 kDa, or about 500 kDa to about 1000 kDa. Such (large) proteins, chemical substances, polysaccharides, or combinations thereof can be efficiently delivery into cells by the present methods.

In embodiments of the methods as taught herein, the cargo is in solution.

In embodiments, the concentration of the cargo in the mixture may be about 0.001 to about 100 mg/ml (i.e. $\mu g/\mu l$). For example, the concentration of the cargo in the mixture may be about 0.01 mg/ml to about 10 mg/ml or about 0.1 mg/ml to about 1.0 mg/ml. The cargo is preferably provided in the mixture in an aqueous solution, such as in water or a cell culture medium.

The cargo may be comprised in a composition or formulation such as a pharmaceutical formulation or kit of parts, as will be described further herein. The composition may comprise the cargo in a concentration ranging from about 0.005 mg/ml to 100 mg/ml, such as for example about 0.01 mg/ml to about 50 mg/ml.

The term "cell" refers to all types of biological cells, including eukaryotic cells and prokaryotic cells. As used herein, the terms "cells" and "biological cells" are interchangeably used.

In embodiments of the methods as taught herein, the cell may be a plant cell.

In embodiments of the methods as taught herein, the cell may be an animal cell. In embodiments of the methods as taught herein, the cell may be a human cell. As illustrated in the example section, the methods as taught herein advantageously allow high delivery yield into animal cells, in particular into human cells, e.g. human T cells.

In embodiments of the methods as taught herein, the cell may be a microorganism. In embodiments of the methods as taught herein, the cell may be a bacteria, a yeast, or a fungal cell.

In embodiments of the methods as taught herein, the cell may be a synthetic cell.

Cells may be obtained from (e.g., isolated from, derived from) a biological sample, preferably a biological sample of a human subject, e.g., blood, bone marrow, trabecular bone, umbilical cord, placenta, foetal yolk sac, skin (dermis), specifically foetal and adolescent skin, periosteum, dental pulp, tendon and adipose tissue. The term "biological sample" or "sample" as used herein refers to a sample obtained from a biological source, e.g., from an organism, such as a plant, an animal or human subject, cell culture, tissue sample, etc. A biological sample of a plant, an animal or human subject refers to a sample removed from a plant, an animal or human subject and comprising cells thereof. The biological sample of a plant, an animal or human subject may comprise one or more tissue types and may comprise cells of one or more tissue types. Methods of obtaining biological samples of a plant, an animal or human subject are well known in the art, e.g., tissue biopsy or drawing blood.

The biological sample can be derived from a biological origin, such as from plants, humans or non-human animals, preferably warm-blooded animals, even more preferably mammals, such as, e.g., non-human primates, rodents, canines, felines, equines, ovines, porcines, and the like. The term "non-human animals" includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. Preferably, the biological sample is derived from human origin.

The biological sample can be a biological fluid or a non-fluid biological sample. It should be understood that sample preparation before or during the method as taught herein can release the cells, for instance from a non-fluid biological sample. Hence, the method as taught herein may comprise the step of releasing the cells from a non-fluid biological sample such as a cell tissue.

In embodiments, the cell may be obtained from a biological sample of plant origin, plant tissue culture, or plant cell culture. For instance, the cell may be obtained from a plant cell suspension culture. In embodiments, the cell may be a plant cell of a living plant.

In embodiments, the cell may be obtained from a biological sample of animal origin, such as blood, bone marrow, trabecular bone, umbilical cord, placenta, foetal yolk sac, skin (dermis), specifically foetal and adolescent skin, periosteum, dental pulp, tendon and adipose tissue.

In embodiments, the cell may be obtained from whole blood. In embodiments, the cell may be obtained from a buffy coat. After centrifugation of whole blood, three layers can be distinguished: a layer of clear fluid (i.e. the plasma), a layer of red fluid containing red blood cells and granulocytes, and a thin layer in between, called the buffy coat. The buffy coat contains most of the white blood cells and platelets.

In embodiments, the cell may be obtained from bone marrow.

In embodiments, the cell may be a blood cell, a stem cell, or a cell derived thereof.

The terms "blood cell", "hematopoietic cell", "hemocyte" or "hematocyte" refer generally to a cell produced through hematopoiesis and found mainly in the blood. Major types of blood cells include red blood cells (erythrocytes), white blood cells (leukocytes), and platelets (thrombocytes).

The term "stem cell" refers generally to an unspecialized or relatively less specialized and proliferation-competent cell, which is capable of self-renewal, i.e., can proliferate without differentiation, and which or the progeny of which can give rise to at least one relatively more specialized cell type. The term encompasses stem cells capable of substantially unlimited self-renewal, i.e., wherein the progeny of a stem cell or at least part thereof substantially retains the unspecialized or relatively less specialized phenotype, the differentiation potential, and the proliferation capacity of the mother stem cell, as well as stem cells which display limited self-renewal, i.e., wherein the capacity of the progeny or part thereof for further proliferation and/or differentiation is demonstrably reduced compared to the mother cell. By means of example and not limitation, a stem cell may give rise to descendants that can differentiate along one or more lineages to produce increasingly relatively more specialized cells, wherein such descendants and/or increasingly relatively more specialized cells may themselves be stem cells as defined herein, or even to produce terminally differentiated cells, i.e., fully specialized cells, which may be post-mitotic.

In embodiments of the methods as taught herein, the cell may be an immune cell such as a T cell, a lymphocyte, a macrophage, a dendritic cell, a monocyte, a NK cell, a NKT cell, a B cell, a neutrophil, a granulocyte, a microglial cell, or a Langerhans cell. In embodiments, the immune cell is a lymphocyte such as a T cell. The present methods advantageously allow high delivery yield of a cargo, such as a mRNA, into immune cells, e.g. T cells.

Methods of obtaining immune cells, such as T cells, from an animal or human subject are well known in the art.

In embodiments, the cell may be an isolated cell or cultured cell.

The term "isolated" as used throughout this specification with reference to a particular component generally denotes that such component exists in separation from - for example, has been separated from or prepared and/or maintained in separation from - one or more other components of its natural environment. More particularly, the term "isolated" as used herein in relation to cells or tissues denotes that such cells or tissues do not or no longer form part of a plant, an animal or human body.

Isolated cells or tissues may be suitably cultured or cultivated *in vitro.* The terms "culturing" or "cell culture" are common in the art and broadly refer to maintenance of cells and potentially expansion (proliferation, propagation) of cells *in vitro.* Typically, plant cells or animal cells, such as mammalian cells, such as human cells, are cultured by exposing them to (i.e., contacting them with) a suitable cell culture medium in a vessel or container adequate for the purpose (e.g., a 96-, 24-, or 6-well plate, a T-25, T-75, T-150 or T-225 flask, or a cell factory), at art-known conditions conducive to *in vitro* cell culture.

In embodiments, the concentration of the cells in the mixture may be about $10^2$ to about $10^{10}$ cells per millilitre (ml) and more preferably about $10^3$ to about $10^9$ cells/ml, such as for example about $10^4$ to about $10^8$ cells/ml or about $10^5$ to about $10^8$ cells/ml. The cells are preferably present in the mixture in an aqueous solution such as in a cell culture medium or a suitable transfection buffer solution.

In embodiments, delivering (or the delivery of) a cargo into a cell may be transfecting (or the transfection of) a cargo into a cell. In embodiments, the methods as taught herein may thus be for transfecting or the transfection of a cargo into a cell.

The term "transfection" refers to the process of introducing a nucleic acid into an animal cell.

In embodiments, delivering (or the delivery of) a cargo into a cell may be transforming (or the transformation of) a cargo into a cell. In embodiments, the methods as taught herein may thus be for transforming or the transformation of a cargo into a cell.

The term "transformation" refers to the process of introducing a nucleic acid into an non-animal eucaryotic cell such as a plant cell.

In embodiments of the methods as taught herein, the organic particle is selected from the group consisting of a polymer-based particle, a protein-based particle, a lipid-based particle, e.g. a liposome or a solid lipid particle, and combinations thereof.

The term "based" as used in the context of the material of the organic particle as defined above is to be understood as a particle that predominantly comprises or is made of said material. In other words, said protein-based particle is to be understood as a particle that mainly comprises or completely consists of one or more proteins or peptides. A lipid-based or "lipid particle" may be used interchangeably herein and refer to particles comprising, consisting essentially of, or consisting of one or more lipids.

The terms "polymer-based particle" or "polymer particle" may be used interchangeably herein and refer to particles comprising, consisting essentially of, or consisting of one or more polymers.

As used herein, the term "polymer" refers to a macromolecule composed of repeating subunits or monomers. Preferably, each monomer comprises carbon and one or more additional elements such as hydrogen, oxygen or nitrogen. The term encompasses both synthetic and natural polymers but excludes biopolymers such as nucleic acids and proteins.

In embodiments, the organic particle may be a polymer-based particle.

In embodiments, the polymer-based particle is not a carbon-based particle such as a carbon-based microparticle or carbon-based nanoparticle. In embodiments, the polymer-based particle is not an allotrope of carbon. In embodiments, the polymer-based particle is not a particle selected from the group consisting of a graphene particle (e.g. nanoribbon), graphene oxide particle, carbon nanotube, carbon dot, fullerene, graphite, or diamond, or a combination thereof such as a carbon nanobud. In embodiments, the polymer is not consisting of carbon. In embodiments, the polymer is not an allotrope of carbon such as graphene. In embodiments, the polymer is not a solid form of carbon such as diamond. In embodiments, the polymer is not a crystalline form of carbon such as graphite.

In embodiments of the methods or products as taught herein, the polymer-based particle may comprise or consist of poly(DL-lactic-co-glycolic acid) (PLGA), poly(lactic acid) (PLA), polycaprolactone (PCL), ethyl cellulose, cellulose acetophthalate, cellulose, polyvinyl alcohol, polyethylene glycol, gelatine, collagen, silk, alginate, hyaluronic acid, dextran, starch, polycarbonate, polyacrylate, polystyrene, methoxy-PEG-polylactide, poly(alkyl cyanoacrylate) (PACA), poly(D,L-lactide-co-glycolide (PLGH), poly(allylamine hydrochloride), or a polyoxazoline. For example, PLGA-based ICG nanoparticles (PLGA-ICG NPs) may be prepared as described in Saxena et al., 2004, Int J Pharm, 278(2):293-301.

Suitable examples of PACA include poly(butyl cyanoacrylate) (PBCA), poly(octyl cyanoacrylate) (POCA), and poly (ethyl 2-cyanoacrylate) (PECA).

In embodiments, the polymer-based particle may comprise or consist of one or more clinically approved polymers, such as methoxy-PEG-polylactide, poly(alkyl cyanoacrylate) (PACA), poly(D,L-lactide-co-glycolide (PLGH), and poly(allylamine hydrochloride).

In embodiments, the organic particle may be a protein-based particle. Protein-based particles are highly stable, biocompatible, and biodegradable, thereby allowing the delivery of a cargo into a cell without any toxicity to the cell.

The terms "protein-based particle" or "protein particle" may be used interchangeably herein and refer to particles comprising, consisting essentially of, or consisting of one or more proteins.

Protein-based particles include protein-based nanoparticles (PNPs) such as non-viral particles based on ferritin, heat shock proteins (Hsp), DNA-binding proteins from starved cells (Dps), encapsulin, the E2 protein of pyruvate dehydrogenase, lumazine synthase, vault proteins; and virus-like particles (VLPs).

In embodiments, the protein may be an albumin such as human serum albumin (HSA). For example, human serum albumin ICG nanoparticles (HSA-ICG NPs) may be prepared as described in Sheng et al., 2014, ACS Nano, 8(12):12310-22.

In embodiments, the organic particle may be a protein-based particle comprising one or more clinically approved proteins such as albumin-bound nanoparticles (e.g. Abraxane). In embodiments, the organic particle may be a protein-based particle comprising or one or more clinically approved protein-based drugs, such as engineered protein combining IL-2 and diphtheria toxin (e.g. Ontak); PEG-asparaginase (e.g. Oncaspar); PEG-filgrastim (PEGylated granulocyte colony-stimulating factor (GCSF) protein) (e.g. Neulasta); PEGylated porcine-like uricase (e.g. Krystexxa); PEGylated factor VIII (e.g. ADYNOVATE); PEGylated IFN alpha-2a protein (e.g. PegIntron); PEGylated adenosine deaminase enzyme (e.g. Adagen/pegademase bovine); PEGylated antibody fragment (e.g. Cimzia/certolizumab pegol); random copolymer of L-glutamate, L-alanine, L-lysine and L-tyrosine (e.g. Copaxone/Glatopa); PEGylated anti-vascular endothelial growth factor (VEGF) aptamer (e.g. Macugen), methoxy polyethylene glycol-epoetin beta (e.g. Mircera); PEGylated GCSF protein (e.g. Neulasta); PEGylated IFN alpha-2a protein (e.g. Pegasys).

The terms "lipid-based particle" or "lipid particle" may be used interchangeably herein and refer to particles comprising, consisting essentially of, or consisting of one or more lipids. Lipid-based articles are biocompatible, and biodegradable, thereby allowing the delivery of a cargo into a cell without any toxicity to the cell.

The lipid-based particles may be liposomes or may be solid lipid particles.

In embodiments, the lipid-based particles may comprise a natural, a bacterial or a synthetic lipid. In embodiments, the lipid-based particles may comprise an anionic lipid, neutral lipid, cationic lipid, ionizable lipid or a sterol.

Suitable examples of anionic lipids include phosphatidylserine (PS) and phosphatidylglycerol (PG). Suitable examples of neutral lipids include prostaglandins, eicosanoids, glycerides, glycosylated diacyl glycerols, oxygenated fatty acids, very long chain fatty acids (VLCFA), palmitic acid esters of hydroxystearic acid (PAHSA), N-acylglycine (NAGly), and prenols.

Suitable examples of cationic lipids include multivalent cationic lipids; 1,2-di-O-octadecenyl-3-trimethylammonium propane (DOTMA); ethylphosphocholines (EPC); dimethyldioctadecylammonium (DDAB); N1-[2-((15)-1-[(3-aminopropyl)amino]-4-[di(3-aminopropyl)amino]butylcarboxamido)ethyl]-3,4-di[oleyloxy]-benzamide (MVL5); pH sensitive lipids; 1,2-dioleoyl-3-trimethylammonium propane (DOTAP); 3β-[N-(N',N'-dimethylaminoethane)-carbamoyl]cholesterol (DC-Cholesterol); N4-Cholesteryl-spermine (GL67); 1,2-dioleyloxy-3-dimethylaminopropane (DODMA); Dlin-MC3-DMA (MC3); DLinDAP; DLinDMA; DLinKDMA; or DLinKC2-DMA.

Such lipids are commercially available from Avanti Polar Lipids (Alabama, USA). For instance, a suitable multivalent cationic lipid is (N1-[2-((1S)-1-[(3-aminopropyl)amino]-4-[di(3-aminopropyl)amino]butylcarboxamido) ethyl]-3,4-di[oleyloxy]-benzamide). Examples of ethyl PC include 1,2-dilauroyl-sn-glycero-3-ethylphosphocholine (chloride salt) (12:0 EPC Cl salt); 1,2-dimyristoyl-sn-glycero-3-ethylphosphocholine (chloride salt) (14:0 EPC Cl salt); 1,2-dipalmitoyl-sn-glycero-3-ethylphosphocholine (chloride salt) (16:0 EPC Cl salt); 1,2-distearoyl-sn-glycero-3-ethylphosphocholine (chloride salt) (18:0 EPC Cl salt); 1,2-dioleoyl-sn-glycero-3-ethylphosphocholine (chloride salt) (18:1 EPC Cl salt); 1-palmitoyl-2-oleoyl-sn-glycero-3-ethylphosphocholine (chloride salt) (16:0-18:1 EPC Cl salt); and 1,2-dimyristoleoyl-sn-glycero-3-ethylphosphocholine (Tf salt) (14:1 EPC Tf salt).

Examples of pH sensitive lipids include N-(4-carboxybenzyl)-N,N-dimethyl-2,3-bis(oleoyloxy)propan-1-aminium (DOBAQ); 1,2-distearoyl-3-dimethylammonium-propane (18:0 DAP); 1,2-dipalmitoyl-3-dimethylammonium-propane (16:0 DAP); 1,2-dimyristoyl-3-dimethylammonium-propane (14:0 DAP); 1,2-dioleoyl-3-dimethylammonium-propane (18:1 DAP or DODAP))

Suitable examples of ionizable lipids include Dlin-MC3-DMA (MC3); DLinDAP; DLinDMA; DLinKDMA; or DLinKC2-DMA.

Suitable examples of sterols include oxysterols, natural sterols, bile acids, cholesteryl esters, glycosylated sterols, or oxidized sterols.

Methods for the preparation of lipid-based particles are known in the art. In embodiments, the lipid-based particles may be MC3-based lipid particles (Patel et al., 2019, J. Control. Release, 303, 91-100). Liposomes encapsulating ICG (Lip-ICG) may be prepared as described in Lajunen et al., 2018, J. Control. Release 284, 213-223.

In embodiments of the methods or product as taught herein, the liposomes may be prepared by methods known in the art such as thin film rehydration method (e.g. including extrusion or sonication for the incorporation of the light absorbing molecule into the core of the liposomes); microfluidic mixing; or injection method, e.g. including dropping a solvent containing lipids and light absorbing molecules in a physiological buffer.

For example, liposomes may be prepared by mixing 1,2-Dioleoyl-3-trimethylammonium propane (DOTAP), 1,2-dioleoyl-sn-glycero-3-phosphoethanolamine (DOPE), and 1,2-distearoyl-sn-glycero-3-phosphoethanolamine-N-[amino(polyethylene glycol)-2000] (DSPE-PEG(2000) Amine) at a certain molar ratio. A light absorbing molecule such as ICG may then be incorporated by an extrusion and/or sonication step (tip/bath sonication). Purification may be obtained by ultracentrifugation and/or dialysis.

In embodiments of the methods or product as taught herein, the solid lipid particles may be prepared by methods known in the art such as high shear homogenization (e.g. hot or cold homogenization); ultrasonication/high speed homogenization; solvent emulsification/evaporation; micro emulsion based solid lipid particles preparation; solid lipid particles preparation using supercritical fluid; spray drying method; and double emulsion method.

In embodiments, the photoresponsive organic particle may be a polymer-based particle, a protein-based particle, a lipid-based particle, or a combination of two or more of a polymer-based particle, a protein-based particle, a lipid-based particle. The polymer-based particle, protein-based particle, lipid-based particle, or combination thereof may itself have photoresponsive properties such as a polydopamine particle. Alternatively, the polymer-based particle, protein-based particle, lipid-based particle, or combination thereof may comprise a light absorbing molecule (having photoresponsive properties), thereby providing the polymer-based particle, protein-based particle, lipid-based particle, or combination thereof with photoresponsive properties. Both types of photoresponsive organic particles can be prepared using precursor molecules that are clinically approved.

The term "photoresponsive", "photosensitive", "light sensitising" may be used interchangeably and refer to the capacity to respond to electromagnetic radiation, such as e.g. visible light.

In embodiments of the methods as taught herein or products as taught herein (including the photoresponsive organic particles as taught herein), the photoresponsive organic particle may be a photoresponsive polymer-based particle, a photoresponsive protein-based particle, a photoresponsive lipid-based particle, or a combination thereof. Such photoresponsive organic particles may be prepared using clinically approved precursors, thereby facilitating clinical transition of the delivery methods as taught herein for the production of engineered therapeutic cell products, such as CAR-T cells.

In embodiments of the methods or products as taught herein, the photoresponsive organic particle may be a photoresponsive polymer-based particle. In embodiments, the photoresponsive organic particle may be a photoresponsive polymer-based particle selected from a polydopamine (PD) particle, a poly(N-phenylglycine) (PNPG) particle, a poly-2-phenyl-benzobisthiazole (PPBBT) particle, a porphyrin particle, a phthalocyanine particle, or a polypyrrole particle. In embodiments, the photoresponsive organic particle may comprise or consist of polydopamine, poly(N-phenylglycine), poly-2-phenyl-benzobisthiazole, porphyrin, phthalocyanine or polypyrrole. In embodiments, the photoresponsive organic particle may be prepared (produced or synthetised) from a clinically approved monomer, such as dopamine hydrochloride, thereby facilitating clinical transition of the methods as taught herein for the production of engineered therapeutic cell products, e.g. CAR-T cells.

In embodiments of the methods or products as taught herein, the photoresponsive organic particle may be a polymer-based particle, a protein-based particle, a lipid-based particle (e.g. liposome or solid lipid particle), or a combination thereof comprising a light absorbing molecule. In embodiments, the photoresponsive organic particle may be a polymer-based particle comprising a light absorbing molecule. In embodiments, the photoresponsive organic particle may be a protein-based particle comprising a light absorbing molecule. In embodiments, the photoresponsive organic particle may be a lipid-based particle comprising a light absorbing molecule. In embodiments, the photoresponsive organic particle may be a solid lipid particle comprising a light absorbing molecule. In embodiments, the photoresponsive organic particle may be a combination of two or more of a polymer-based particle, a protein-based particle, a lipid-based particle comprising a light absorbing molecule. Such photoresponsive organic particles may be prepared using clinically approved molecules, thereby facilitating clinical transition of the delivery methods as taught herein for the production of engineered therapeutic cell products, such as CAR-T cells.

In embodiments of the methods or products as taught herein, the photoresponsive organic particle may be a polymer-based particle, a protein-based particle, a lipid particle loaded with or functionalized with a light absorbing molecule.

In embodiments, the light absorbing molecule may be grafted on a particle, such as a nanoparticle or a microparticle. For instance, the light absorbing molecule may be grafted on a particle by 'click-chemistry' at the surface of the particle (e.g. at the end of polymer chains such as at the distal end of poly(ethylene) glycol chains or hyaluronic acid chains). For instance, the grafting of a light absorbing molecule may occur at the end of PEG chains that are grafted on the particles.

In embodiments, the light absorbing molecule may be encapsulated in a particle, such as a nanoparticle or a microparticle. In embodiments, the light absorbing molecule may be encapsulated in a particle by physical or chemical encapsulation. For instance, physical encapsulation of ICG in liposomes may be performed by adding ICG during the rehydration of the lipids. For human serum albumin-ICG particles, the chemical encapsulation may be performed by reacting ICG with the disulphide bonds of HSA.

Examples of photoresponsive protein-based particles include PLGA-based ICG particles.

Examples of photoresponsive protein-based particles include heptamethine dye (CySCOOH) coupled to human serum albumin (HSA) particles; HSA coupled to Ce6 (HSA-Ce6); HSA coupled to squaraine (SQ) dye (HAS-SQ); HSA-IR825 complexes; loading of zinc hexadecafluorophthalocyanine (ZnF16Pc) in Cys-Asp-Cys-Arg-Gly Asp-Cys-Phe-Cys (RGD4C)-modified ferritins (RFRTs); methylene blue encapsulation in apoferritin particles; IR820 dye-loaded ferritin particles; particles consisting of cowpea chlorotic mottle virus (CCMV) protein with a water soluble zinc Pc (ZnPc); albumin coupled to 5,10,15,20-tetrakis(m-hydroxyphenyl)porphyrine (mTHPP) and/or 5,10,15,20-tetrakis(m-hydroxyphenyl) chlorin (mTHPC); hematoporphyrin-linked albumin nanoparticles (HP-ANP); polypyrrole (PPy) complexed albumin nanoparticles; polypyrrole (PPy) complexed with albumin and Ce6 particles; Poly-L-Lysine (PLL) complexed with albumin particles containing Ce6; Poly-L-Lysine (PLL) complexed with albumin particles containing protoporphyrin IX; Poly-L-Lysine (PLL) complexed with albumin particles containing verteporfin; hydrogels containing meso-tetra-(N-methyl-4-pyridyl) porphine tetrachloride (TMPyP); and HSA-IR780 particles.

Examples of photoresponsive liposomes include for instance liposomes comprising ICG.

Examples of photoresponsive solid lipid particles include for instance solid lipid particles containing hydrophobic IR-780 Dye; solid lipid particles containing ICG; and solid lipid particles containing doxorubicin.

In embodiments of the methods or products as taught herein, the light absorbing molecule may be a molecule selected from the group consisting of a light absorbing dye, a naturally occurring light absorber, and a synthetic light absorber.

The light absorbing molecule may be lipophilic, hydrophilic, or amphiphilic. The light absorbing molecule may be a lipophilic compound embedded into the lipid membrane or lipid core of an organic particle. The light absorbing molecule may be a hydrophilic compound encapsulated into the aqueous core of an organic particle. The light absorbing molecule may be an amphiphilic compound embedded into the lipid membrane or lipid core of an organic particle and/or encapsulated into the aqueous core of the organic particle.

The light absorbing dye may be may be a natural dye or a synthetic dye.

The light absorbing dye may be selected from the group consisting of an ANEP dye; a 7-nitrobenz-2-oxa-1,3-diazole-4-yl-labeled phospholipid; 1-oleoyl-2-[6-[(7-nitro-2-1,3-benzoxadiazol-4-yl)amino]hexanoyl]-3-trimethylammonium propane (fluorescent DOTAP); bis-(1,3-dibutylbarbituric acid)trimethine oxonol (DiBAC4(3)); N-(3-Triethylammoniumpropyl)-4-(4-(dibutylamino) styryl) pyridinium dibromide; ethidium bromide; propidium iodide; chlorin e6 (Ce6); purpurin-18 (P18); and heptamethine dye (CySCOOH).

Examples of ANEP dyes include Di-4-ANEPPS; Di-4-ANEPPDHQ; Di-3-ANEPPDHQ; and Di-8-ANEPPS such dyes are commercially available from ThermoFisher Scientific.

Examples of 7-nitrobenz-2-oxa-1,3-diazole-4-yl-labeled phospholipids include [2-(4-nitro-2,1,3-benzoxadiazol-7-yl)aminoethyl]trimethylammonium (NBD-TMA); N-(7-Nitrobenz-2-Oxa-1,3-Diazol-4-yl)-1,2-Dihexadecanoyl-sn-Glycero-3-Phosphoethanolamine, Triethylammonium Salt) (NBD-PE); and NBD-DOPE.

The light absorbing dye may be selected from the group consisting of an azo dye, an arylmethane dye, a cyanine dye, a thiazine dye, a xanthene dye, a carbocyanine dye, and an aminostyryl dye. Examples of azo dyes include Trypan Blue (Membrane Blue, Vision Blue, CAS Number: 72-57-1) and Janus green B (Diazine Green S, Union Green B, CAS Number: 2869-83-2).

Examples of arylmethane dyes include Gentian violet (Crystal violet, Methyl violet 10B, Hexamethyl pararosaniline chloride, CAS Number: 548-62-9); Bromophenol Blue (CAS Number: 115-39-9); Patent blue (Blueron, CAS Number: 3536-49-0); Brilliant Blue (Acid Blue, Coomassie Brilliant Blue, Brilliant Peel, CAS Number: 6104-59-2); Light Green (Light Green SF, Light Green SF Yellowish, CAS Number: 5141-20-8); and Fast Green (Fast Green FCF, Food green 3, FD&C Green No. 3, Green 1724, Solid Green FCF, CAS Number: 2353-45-9).

Examples of cyanine dyes include Indocyanine Green (Cardiogreen, Foxgreen, Cardio-Green, Fox Green, IC Green, CAS Number: 3599-32-4) and Infracyanine Green. Infracyanine Green (IfCG) is a green dye with the same chemical formula and similar pharmacologic properties as ICG. IfCG dye possesses two pharmacologic differences when compared to ICG. First, If ICG contains no sodium iodine, which must be added to ICG during the dye synthesis. Second, the presence of the sodium iodine in the ICG solution necessitates dilution in water, resulting in a hypotonic solution.

Examples of thiazine dyes include Methylene blue (Methylthioninium chloride, CAS Number: 61-73-4) and Toluidine blue (CAS Number: 92-31-9).

Examples of xanthene dyes include Fluorescein Sodium (CAS Number: 518-47-8); Rose Bengal (CAS Number: 4159-77-7); and Rhodamine 6G (Rhodamine 590, Rh6G, C.I. Pigment Red 81, C.I. Pigment Red 169, Basic Rhodamine Yellow, C.I. 45160, CAS Number: 989-38-8).

Examples of carbocyanine dyes include Dil (Cat. no. D282, D3911, N22880, Invitrogen); DiO (Cat. no. D275, Invitrogen); DiD (Cat. no. D307, D7757, Invitrogen); DiR (Cat. no. D12731, Invitrogen), and derivatives thereof. Derivatives of carbocyanine dyes include $DiIC_{12}(3)$ (Cat. no. D383, Invitrogen); $DiIC_{16}(3)$ (Cat. no. D384, Invitrogen); $DiOC_{16}(3)$ (Cat. no. D1125, Invitrogen); unsaturated derivatives, e.g. mono-unsaturated or di-unsaturated derivatives, of Dil and DiO, such as $\Delta 9$ -Dil; (Cat. No. D3886, Invitrogen); sulfonated derivatives of Dil and DiO; and CM-Dil (a thiol-reactive Dil derivative).

Examples of aminostyryl dyes include DiA (Cat. no. D3883, Invitrogen), and 4-Di-10-ASP (Cat. no. D291, Invitrogen), and di-unsaturated derivatives of DiA (Cat. no. D7758, Invitrogen).

In embodiments of the methods or products as taught herein, the light absorbing dye may be selected from the group consisting of indocyanine green (ICG), trypan blue, Janus green B, gentian violet, bromophenol blue, patent blue, brilliant blue, light green, fast green, infracyanine green, methylene blue, toluidine blue, fluorescein sodium, rose Bengal, rhodamine 6G, DiD, DiO, Dil, DiA, DiR, and derivatives of Dil, DiO, or DiA. In embodiments of the methods or products as taught herein, the light absorbing dye may be selected from the group consisting of indocyanine green (ICG), trypan blue, Janus green B, gentian violet, bromophenol blue, patent blue, brilliant blue, light green, fast green, infracyanine green, methylene blue, toluidine blue, fluorescein sodium, rose Bengal, and rhodamine 6G. Such light absorbing dyes are advantageously approved for clinical use. Other light absorbing molecules which have been clinically approved include porfimer sodium (e.g. sold under commercial name Photofrin); $\delta$-Aminolevulinic acid (e.g. sold as Levulan); verteporfine (e.g. sold as Visudyne); temoporfin (e.g. sold as Foscan); sulfonated aluminum phthalocyanine (e.g. sold as Photosense); methyl aminolevulinate (MAL) (e.g. sold as Metvix); talaporfin or mono-L-aspartyl chlorin e6 (e.g. sold as Laserphyrin).

Other light absorbing molecules include for example pheophorbide; phthalocyanine derivatives (e.g. zinc (II) phthalocyanine, silicon phthalocyanine 4); cyanine IR-768; hypericin; hypocrellin A; C60 or C70 fullerene cage; and pheophorbide A (PheA).

The naturally occurring light absorber may be selected from the group consisting of hemoglobin, cytochrome C, porphyrin, and pigments. The pigment may be for example melanin, rhodopsin, photopsins, iodopsin, chlorophyll a, chlorophyll b, phycoerythrin, $\beta$-carotene, phycocyanin, or allophycocyanin.

The synthetic light absorber can be selected from the group consisting of polydopamine, poly(N-phenylglycine, poly-2-phenyl-benzobisthiazole, porphyrin, phthalocyanine, or polypyrrole.

The photoresponsive organic particle may have a size such that the largest distance between two points of the one or more photoresponsive organic particles is about 100 nm to about 2000 nm (2 $\mu$m). For instance, the largest distance between two points of the one or more photoresponsive organic particles may be about 200 nm to about 2000 nm, about 250 nm to about 2000 nm, about 250 nm to about 1750 nm, about 250 nm to about 1500 nm, or about 250 nm to about 1300 nm.

In embodiments of the methods or products as taught herein, the largest distance between two points of the one or more photoresponsive organic particles may be about 250 nm to about 1250 nm (1.25 $\mu$m). In embodiments, the largest distance between two points of the one or more photoresponsive organic particles may be about 300 nm to about 1200 nm(1.2 $\mu$m), about 400 nm to about 1100 nm (1.1 $\mu$m), about 300 nm to about 1000 nm(1.0 $\mu$m), or about 500 nm to about 1000 nm (1 $\mu$m). For instance, the largest distance between two points of the one or more photoresponsive organic particles may be about 550 nm to about 950 nm, about 600 nm to about 900 nm, about 650 nm to about 850 nm, or about 700 nm to about 800 nm. Such photoresponsive organic particles advantageously allow efficient delivery of a cargo, such as nucleic acids including mRNA, into a cell while maintaining sufficient cell viability, thereby resulting in high delivery yield of a cargo into the cell.

In embodiments of the methods as taught herein, the delivery yield when using photoresponsive organic particles having the largest dimensions of about 250 nm to about 1250 nm, such as of about 300 nm to about 1200 nm, about 400 nm to about 1100 nm, about 300 nm to about 1000 nm, or about 500 nm to about 1000 nm, may be at least 20%. For instance, the delivery yield when using photoresponsive organic particles having the largest dimensions of about 250 nm to about 1250 nm, such as of about 300 nm to about 1200 nm, about 400 nm to about 1100 nm, about 300 nm to about 1000 nm, or about 500 nm to about 1000 nm, may be at least 25%, at least 30%, at least 35%, at least 40%, at least 45%, or at least 50%.

The term "delivery yield" as used herein refers to the ratio of the quantity of living (viable) cells comprising the cargo after performing the method as taught herein (e.g. the quantity of living cells comprising the cargo as detected after the delivery method) relative to the quantity of living (viable) cells before performing the method as taught herein (e.g. the quantity of living cells as detected before the delivery method).

The delivery yield expressed as a percentage (%) can be determined by multiplying the viability of the cells after performing the method as taught herein (e.g. expressed as a percentage) with the efficiency of the method as taught herein (e.g. expressed as a percentage), followed by dividing the resulting value by 100.

$$\text{Delivery yield (\%)} = \{[\text{cell viability (\%)}] \times [\text{efficiency (\%)}]\}/100$$

The viability of cells after performing the method as taught herein (%) may be determined by dividing the quantity, such as number, of viable cells obtained after performing the method as taught herein by the quantity, such as number, of (total) viable cells before performing the method as taught herein, followed by multiplying the resulting value by 100.

The efficiency of the method as taught herein (%) may be determined by dividing the quantity, such as number, of viable cells comprising the cargo obtained after performing the method as taught herein by the quantity, such as number, of (total) viable cells obtained after performing the method as taught herein, followed by multiplying the resulting value by 100.

The delivery yield (%) = {[(number of viable cells after delivery method/number of viable cells before delivery method) x100] x [(number of viable cells comprising cargo after delivery method/number of viable cells after delivery method) x 100]}/100

In embodiments of the methods as taught herein, the delivery yield (e.g. maximal delivery yield) when using photoresponsive organic particles having the largest dimensions of about 250 nm to about 1250 nm, such as of about 300 nm to about 1200 nm, about 400 nm to about 1100 nm, about 300 nm to about 1000 nm, or about 500 nm to about 1000 nm, may be enhanced (i.e., increased) by at least about 5% relative to (i.e., compared with) the delivery yield (e.g. maximal delivery yield) when using photoresponsive organic particles having the largest dimensions outside this range (e.g. of about 100 nm or of about 2000 nm). In embodiments of the methods as taught herein, the delivery yield (e.g. maximal delivery yield) when using photoresponsive organic particles having the largest dimensions of about 250 nm to about 1250 nm, such as of about 300 nm to about 1200 nm, about 400 nm to about 1100 nm, about 300 nm to about 1000 nm, or about 500 nm to about 1000 nm, may be at least enhanced (i.e., increased) by at least about 10%, by at least about 15%, by at least about 20%, by at least about 25%, by at least about 30%, by at least about 35%, by at least about 40%, by at least about 45%, or by at least about 50%, or by at least about 60%, or by at least about 70%, or by at least about 80%, or by at least about 90%, or by at least about 100%, by at least about 200%, by at least about 300%, by at least about 400%, or by at least about 500%, relative to (i.e., compared with) the delivery yield (e.g. maximal delivery yield) when using photoresponsive organic particles having the largest dimensions outside this range (e.g. of about 100 nm or of about 2000 nm).

For example, an increase may encompass an increase of a first value of the delivery yield by, without limitation, at least about 5%, at least about 6%, at least about 7%, at least about 8%, at least about 9%, or at least about 10%, or by at least about 20%, or by at least about 30%, or by at least about 40%, or by at least about 50%, or by at least about 60%, or by at least about 70%, or by at least about 80%, or by at least about 90%, at least about 100%, at least about 200%, at least about 300%, at least about 400%, or at least about 500%, relative to a second value of the delivery yield with which a comparison is being made.

For instance, in a first example, an increase may encompass an increase of a first value of the delivery yield (e.g., 10% delivery yield) by 20% (i.e. 2%/10% x 100%) relative to a second value of the delivery yield (e.g., 12% delivery yield) with which a comparison is being made. In a second example, a deviation may encompass an increase of a first value of the delivery yield (e.g., 10% delivery yield) by 200% (i.e. 20%/10% x 100%) relative to a second value of the delivery yield (e.g., 30% delivery yield) with which a comparison is being made.

Accordingly, also provided herein is a photoresponsive organic particle, wherein the organic particle is selected from the group consisting of a polymer-based particle, a protein-based particle, a lipid-based particle, and a combination thereof, and wherein the largest distance between two points of the one or more photoresponsive organic particles may be about 250 nm to about 1250 nm (1.25 μm). As extensively demonstrated in the example section, such photoresponsive organic particles advantageously allow efficient delivery of a cargo such as nucleic acids including mRNA into a cell, while keeping satisfactory cell viability, thereby resulting in high delivery yield of a cargo into the cell.

The phrase "largest distance between two points of the one or more photoresponsive organic particles" refers to the largest dimension of the one or more photoresponsive organic particles.

The photoresponsive organic particles may comprise individual particles or a group, agglomerate, or cluster of two or more particles positioned close to or in contact with each other.

In embodiments, the photoresponsive organic particles may be present as individual particles, for instance in an aqueous solution, such as in a cell culture medium. In these embodiments, the phrase "largest distance between two points of the one or more photoresponsive organic particles" refers to the largest distance between two points of the individual particle.

In embodiments, the photoresponsive organic particles may comprise a group, agglomerate, or cluster of two or more particles, for instance in an aqueous solution, such as in a cell culture medium. In these embodiments, the phrase "largest distance between two points of the one or more photoresponsive organic particles" refers to the largest distance between two points of the group, agglomerate, or cluster of the two or more particles.

The largest distance may be an average largest distance calculated over a group of particles.

The term "particle" as used herein refers to a particle or a group, agglomerate, or cluster of two or more particles having dimensions (more particularly the largest dimensions of the particles) of about 1 nm to about 2000 nm (2 $\mu$m).

The term "microparticle" as used herein refers to a particle or a group, agglomerate, or cluster of two or more particles having dimensions (more particularly the largest dimensions of the particles) of more than 1000 nm (> 1 $\mu$m) and at most 2000 nm ($\leq$ 2 $\mu$m).

The term "nanoparticle" refers to a particle or a group, agglomerate, or cluster of two or more particles having dimensions (largest dimensions of the particles) of at least 1 nm ($\geq$ 1 nm) and at most 1000 nm ($\leq$ 1 $\mu$m).

The dimensions of a particle, for example a width, height or diameter of a particle, can be determined using Transmission Electron Microscopy (TEM), Scanning Electron Microscopy (SEM) or atomic force microscopy (AFM).

In embodiments, the methods as taught herein may comprise contacting the cell with one type of photoresponsive organic particles or a combination of different photoresponsive organic particles, for example photoresponsive organic particles having a different size, a different composition and/or a different shape.

The particles may have any shape. For example, the particles may be spherical, elliptical, rod-like shaped, pyramidal, branched, or may have an irregular shape.

In embodiments of the methods or products as taught herein, the photoresponsive organic particle may be an elliptical particle, rod-like shaped particle, pyramidal particle, branched particle, or may be an irregular shaped particle. The size of such particles is preferably defined by the equivalent spherical diameter (also referred to as the equivalent volume diameter). The equivalent spherical diameter (or ESD) of an irregularly shaped object is the diameter of a sphere of equivalent volume.

In embodiments, the photoresponsive organic particle may have an equivalent spherical diameter of about 250 nm to about 2000 nm. In embodiments, the photoresponsive organic particle may have an equivalent spherical diameter of about 250 nm to about 1250 nm. For instance, the photoresponsive organic particle may have an equivalent spherical diameter of about 300 nm to about 1200 nm, or about 500 nm to about 1000 nm. Such photoresponsive organic particle advantageously allow efficient delivery of a cargo such as large macromolecules into a cell.

The particles may have a spherical shape. In embodiments of the methods or products as taught herein, the photoresponsive organic particle may be a spherical particle. In embodiments, the photoresponsive organic particle may be a spherical particle having an average diameter of about 250 nm to about 2000 nm. In embodiments, the photoresponsive organic particle may be a spherical particle having an average diameter of about 250 nm to about 1250 nm. For instance, the photoresponsive organic particle may be a spherical particle having an average diameter of about 300 nm to about 1200 nm, or about 500 nm to about 1000 nm. Such photoresponsive organic particle advantageously allow efficient delivery of a cargo such as large macromolecules into a cell.

In embodiments of the methods or products as taught herein, the photoresponsive organic particle may be functionalized on the surface such as coated on the surface. In embodiments, the photoresponsive organic particle may be functionalized, such as coated, with one or more compounds selected from the group consisting of albumin, polyethyleneimine (PEI), polyvinylpyrrolidone (PVP), polyethylene glycol (PEG), poly(diallyldimethylammonium chloride) (PDDAC), poly(allylamine hydrochloride) (PAH), polyamidoamine (PAA), poly(amino-co-ester) (PAE), poly[2-(N,N-dimethylamino)ethyl methacrylate] (PDMAEMA), hyaluronic acid (HA), gelatin, polyglycerol, a cyclodextrin (CD), dextran, cellulose, silica, polyoxazoline, sulfobetaine-silane (SBS), a cationic lipid, a neutral lipid, an anionic lipid, chitosan, and poly-L-Lysine. Advantageously, photoresponsive organic particles which are further functionalized, such as polydopamine particles functionalized with albumin, allow to improve colloidal stability without interfering with or even improving the particles ability to adhere to a barrier of the cell, such as the cell membrane or cell wall.

In embodiments of the methods or products as taught herein, the photoresponsive organic particle may be a polydopamine particle. In embodiments of the methods or products as taught herein, the photoresponsive organic particle may be a polydopamine particle coated with albumin. Such photoresponsive organic particles may advantageously be biocompatible and biodegradable, and have sufficient colloidal stability, for example in a suitable cell medium or transfection buffer solution.

In embodiments, the photoresponsive organic particles are capable of binding to the cells. In embodiments, the photoresponsive organic particles may be capable of binding to (or accumulating at) the cells after contacting the cell with the photoresponsive organic particles. The binding of the photoresponsive organic particles to the cell may be binding by a covalent binding or a non-covalent interaction such as an electrostatic or hydrophobic interaction.

In embodiments, the methods as taught herein may comprise contacting the cell with the photoresponsive organic particle, thereby inducing binding of the photoresponsive organic particle to (accumulation of the photoresponsive organic particle at) the cell, in particular to a barrier of the cell, such as to the cell wall or cell membrane of the cell.

In embodiments of the methods and products as taught herein, the photoresponsive organic particle may be capable of forming vapour nanobubbles the cell, in particular at a barrier of the cell, when irradiated. When using the photoresponsive organic particle in accordance with embodiments of the invention, the photoresponsive organic particle may form vapour nanobubbles at the cell, in particular at a barrier of the cell, when irradiated.

In embodiments, the methods as taught herein may comprise irradiating the mixture of the cell, the cargo, and the photoresponsive organic particle with electromagnetic radiation, thereby causing permeabilization of a barrier of the cell and allowing influx of the cargo into the cell. In embodiments, the methods may comprise irradiating the photoresponsive organic particle bound to at least part of the cell, thereby forming vapour nanobubbles at a barrier of the cell and inducing permeabilization of the cellular barrier.

In embodiments of the methods or products as taught herein, the photoresponsive organic particle may be biodegradable. In embodiments, the photoresponsive organic particle may be biocompatible. In embodiments, the photoresponsive organic particle may be biodegradable and biocompatible. In embodiments, the photoresponsive organic particle may comprise or consist of clinically approved components, such as polymers, proteins, lipids, solid lipids, and/or light absorbing molecules. Advantageously, this renders the photoresponsive organic particles biocompatible and/or biodegradable.

In embodiments of the methods as taught herein, the photoresponsive organic particles are not coupled to a solid support, e.g. the photoresponsive organic particles are not coupled (e.g. grafted) to or enclosed (e.g. embedded) in a solid support or solid material. In embodiments of the methods as taught herein, the photoresponsive organic particles are not enclosed (e.g. embedded) in a nonporous structure such as a polymer sheet or polymer foil. In embodiments of the methods as taught herein, the photoresponsive organic particles are not enclosed (e.g. embedded) in a porous polymer structure such as structures comprising fibres (for example polymer fibres), structures comprising particulates (for example polymer particulates), structures comprising a combination of fibres and particulates (for example a combination of polymer fibres and/or polymer particulates) and structures comprising foam (for example polymer foam). In embodiments of the methods as taught herein, the photoresponsive organic particles are in solution.

In embodiments, the mixture may comprise the photoresponsive organic particles in a concentration of about $10^5$ to about $10^{11}$ particles per millilitre (ml) and more preferably about $10^6$ to about $10^{10}$ particles/ml, such as for example about $10^7$ to about $10^9$ particles/ml, e.g. to balance efficient permeabilization with cell viability. The mixture preferably comprises the photoresponsive organic particles in an aqueous solution, such as in a cell culture medium or a suitable transfection buffer solution, in which the photoresponsive organic particles have sufficient colloidal stability.

The photoresponsive organic particles may be comprised in a composition or formulation such as a pharmaceutical formulation or kit of parts, as will be described further herein. The composition may comprise the photoresponsive organic particles in a concentration ranging from about $10^8$ to about $10^{15}$ particles/ml, such as for example about $10^{10}$ to about $10^{12}$ particles/ml.

In embodiments of the methods as taught herein, the cell, the photoresponsive organic particle, and the cargo may be contacted in the mixture in a concentration of about $10^2$ to about $10^{10}$ cells/ml, about $10^5$ to about $10^{11}$ particles/ml, and about 0.001 to about 10 mg/ml (i.e. $\mu$g/$\mu$l) cargo/ml (with ml referring to the volume of the mixture).

In embodiments of the methods as taught herein, the cargo may be a nucleic acid such as a plasmid DNA encoding a chimeric antigen receptor (CAR) or a mRNA encoding a CAR, and the cells may be T cells. In embodiments of the methods as taught herein, the cargo may be a nucleic acid such as a plasmid DNA encoding a CAR or a mRNA encoding a CAR; the photoresponsive organic particle may be a polydopamine particle, preferably a polydopamine particle coated with albumin; and the cells may be T cells. The present methods advantageously allow the delivery of desired genetic constructs, e.g. encoding a chimeric antigen receptor, into human T cells to generate chimeric antigen receptor T cells.

The term "chimeric antigen receptor" or "CAR" (also known as chimeric immunoreceptors, chimeric T cell receptors or artificial T cell receptors) refers to a receptor protein that has been engineered to give T cells the new ability to target a specific protein. The receptors are chimeric because they combine both antigen-binding and T-cell activating functions into a single receptor.

In embodiments of the methods as taught herein, the cargo may be a mRNA encoding a CAR and the cells may be T cells. In embodiments of the methods as taught herein, the cargo may be a mRNA encoding a CAR; the photoresponsive organic particle may be a polydopamine particle, preferably a polydopamine particle coated with albumin; and the cells may be T cells. The present methods advantageously allow delivery and transient expression of mRNA into T cells, thereby providing control over the duration of expression without any risks for unintended mutations by genomic integration.

In embodiments, the methods comprise contacting the cell with the one or more photoresponsive organic particles. In embodiments, the methods comprise contacting the cell with the one or more photoresponsive organic particles and the cargo.

The term "contact" or "contacting" as used herein means bringing one or more first components (such as one or more molecules, biological entities, cells, or materials) together with one or more second components (such as one or more molecules, biological entities, cells, or materials) in such a manner that the first component(s) can - if capable thereof - interact with such as bind or modulate the second component(s) or that the second component(s) can - if capable thereof - interact with such as bind or modulate the first component(s). Such modulation may occur either directly, i.e., by way of direct interaction between the first and second component(s); or indirectly, e.g., when the first component(s) interact with or modulate one or more further component(s), one or more of which in turn interact with or modulate the second component(s), or vice versa. The term "contacting" may depending on the context be synonymous with "exposing", "bringing together", "mixing", "reacting", "treating", or the like.

In embodiments, the contacting step (e.g. in suspension) may comprise pipetting or microfluidics.

In the methods as taught herein, the cells may be contacted with the photoresponsive organic particles in such a manner that the photoresponsive organic particles can interact with - such as bind - the cells.

In embodiments of the methods as taught herein, the cells and the one or more photoresponsive particles are contacted, and the mixture of the cells and the one or more photoresponsive organic particles is irradiated before the mixture of the cells and the one or more photoresponsive organic particles is contacted with the cargo (i.e. before the cargo is added to the mixture of the cells and the one or more photoresponsive organic particles).

In embodiments, the methods as taught herein may comprise:

- contacting a cell with one or more photoresponsive organic particles, wherein the organic particle is selected from the group consisting of a polymer-based particle, a protein-based particle, a lipid-based particle, and a combination thereof, thereby obtaining a mixture of the cell and the one or more photoresponsive organic particles;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with a cargo (e.g. adding the cargo to the mixture of the cell and the one or more photoresponsive organic particles), thereby delivering the cargo into the cell.

In embodiments of the methods as taught herein, the cells, the one or more photoresponsive particles, and the cargo are contacted with each other before the mixture of the cells, the one or more photoresponsive organic particles, and the cargo is irradiated.

In embodiments, the methods as taught herein may comprise:

- contacting a cell with one or more photoresponsive organic particles and a cargo, wherein the cargo is not bound to the one or more photoresponsive organic particles, and wherein the organic particle is selected from the group consisting of a polymer-based particle, a protein-based particle, a lipid-based particle, and a combination thereof, thereby obtaining a mixture of the cell, the cargo, and the one or more photoresponsive organic particles; and

- irradiating the mixture of the cell, the cargo, and the one or more photoresponsive organic particles with electromagnetic radiation, thereby causing permeabilization of a barrier of the cell and delivering the cargo into the cell.

In embodiments, the cells, the one or more photoresponsive particles, and the cargo may be contacted with each other in any order. In embodiments, the cargo may be contacted with the cells before contacting the mixture of the cells and the cargo with the photoresponsive organic particles. In preferred embodiments, the cargo may be contacted with the cells after contacting the cells with the photoresponsive organic particles. Further, in embodiments, the cargo may be contacted with the photoresponsive organic particles before contacting the mixture of the photoresponsive organic particles and the cargo with the cells.

Accordingly, in preferred embodiments, the methods as taught herein may comprise:

- contacting a cell with one or more photoresponsive organic particles, wherein the organic particle is selected from the group consisting of a polymer-based particle, a protein-based particle, a lipid-based particle, and a combination thereof, thereby obtaining a mixture of the cell and the one or more photoresponsive organic particles;

- contacting the mixture of the cell and the one or more photoresponsive organic particles with the cargo (e.g. adding the cargo to the mixture of the cell and the one or more photoresponsive organic particles); and

- irradiating the mixture of the cell, the cargo, and the one or more photoresponsive organic particles with electromagnetic radiation, thereby causing permeabilization of a barrier of the cell and delivering the cargo into the cell.

In embodiments, the methods as taught herein may comprise:

- contacting the cargo with the cell (e.g. adding the cargo to a cell suspension), thereby obtaining a mixture of the cell and the cargo;

- contacting the mixture with one or more photoresponsive organic particles, wherein the organic particle is selected from the group consisting of a polymer-based particle, a protein-based particle, a lipid-based particle , and a combination thereof; and

- irradiating the mixture with electromagnetic radiation, thereby causing permeabilization of a barrier of the cell and delivering the cargo into the cell.

In embodiments, the methods as taught herein may comprise:

- contacting the cargo with one or more photoresponsive organic particles, wherein the cargo is not bound to the one or more photoresponsive organic particles, and wherein the organic particle is selected from the group consisting of a polymer-based particle, a protein-based particle, a lipid-based particle , and a combination thereof, thereby obtaining a mixture of the cargo and the one or more photoresponsive organic particles;

- contacting the mixture with a cell; and

- irradiating the mixture with electromagnetic radiation, thereby causing permeabilization of a barrier of the cell and delivering the cargo into the cell.

In embodiments, the methods as taught herein may comprise contacting the cell with the one or more photoresponsive organic particles, thereby allowing (at least part of) the one or more photoresponsive organic particles to become associated to the cells, e.g. by binding to the cell membrane or cell wall or by active internalization by endocytic processes. **In** embodiments, the methods as taught herein may comprise washing the unbound photoresponsive organic particles from the cells.

In embodiments, the methods as taught herein may comprise contacting a cell with one or more photoresponsive organic particles, thereby obtaining a mixture of the cell and the one or more photoresponsive organic particles; and optionally washing the unbound photoresponsive organic particles from the cells.

In embodiments, the methods as taught herein may comprise:

- contacting a cell with one or more photoresponsive organic particles, thereby obtaining a mixture of the cell and the one or more photoresponsive organic particles;

- optionally washing the unbound photoresponsive organic particles from the cells;

- contacting the mixture of the cell and the one or more photoresponsive organic particles with the cargo (e.g. adding the cargo to the mixture of the cell and the one or more photoresponsive organic particles); and

- irradiating the mixture of the cell, the cargo, and the one or more photoresponsive organic particles with electromagnetic radiation, thereby causing permeabilization of a barrier of the cell and delivering the cargo into the cell.

In embodiments, the methods as taught herein may comprise:

- contacting a cell with one or more photoresponsive organic particles, thereby obtaining a mixture of the cell and the one or more photoresponsive organic particles;

- optionally washing the unbound photoresponsive organic particles from the cells;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with the cargo (e.g. adding the cargo to the mixture of the cell and the one or more photoresponsive organic particles), thereby delivering the cargo into the cell.

In embodiments, the methods as taught herein may comprise incubating the mixture of the cells and the photoresponsive organic particles. Incubation may increase the binding of the one or more photoresponsive organic particles to the cells.

In embodiments, the methods as taught herein may comprise contacting a cell with one or more photoresponsive organic particles, thereby obtaining a mixture of the cell and the one or more photoresponsive organic particles; and optionally incubating the mixture of the cells and the photoresponsive organic particles.

In embodiments, the methods as taught herein may comprise:

- contacting a cell with one or more photoresponsive organic particles, thereby obtaining a mixture of the cell and the one or more photoresponsive organic particles;

- incubating the mixture of the cells and the photoresponsive organic particles;

- optionally washing the unbound photoresponsive organic particles from the cells;

- contacting the mixture of the cell and the one or more photoresponsive organic particles with the cargo (e.g. adding the cargo to the mixture of the cell and the one or more photoresponsive organic particles); and

- irradiating the mixture of the cell, the cargo, and the one or more photoresponsive organic particles with electromagnetic radiation, thereby causing permeabilization of a barrier of the cell and delivering the cargo into the cell.

In embodiments, the methods as taught herein may comprise:

- contacting a cell with one or more photoresponsive organic particles, thereby obtaining a mixture of the cell and the one or more photoresponsive organic particles;

- incubating the mixture of the cells and the photoresponsive organic particles;

- optionally washing the unbound photoresponsive organic particles from the cells;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with the cargo (e.g. adding the cargo to the mixture of the cell and the one or more photoresponsive organic particles), thereby delivering the cargo into the cell.

In embodiments of the methods as taught herein, the cells and the photoresponsive organic particles may be contacted immediately before performing the next step. This advantageously allows to perform rapid delivery of a cargo into cells. In embodiments, after contacting (e.g. mixing) the cells and the photoresponsive organic particles, the cells and the photoresponsive organic particles may be incubated at least 1 min. In embodiments, after contacting (e.g. mixing) the cells and the photoresponsive organic particles, the cells and the photoresponsive organic particles may be incubated at least 5 min, such as at least 10 min, at least 15 min, at least 30 min, at least 1 hour, at least 2 hours, at least 3 hours, at least 4 hours, or at least 6 hours.

In embodiments of the methods as taught herein, after contacting (e.g. mixing) the cells and the photoresponsive organic particles, the cells and the photoresponsive organic particles may be incubated before (i.e. prior to) contacting the cargo with the mixture of the cells and the photoresponsive organic particles. In embodiments, after contacting (e.g. mixing) the cells and the photoresponsive organic particles, the cells and the photoresponsive organic particles may be incubated at least 1 min before (i.e. prior to) contacting the cargo with the mixture of the cells and the photoresponsive organic particles. In embodiments, after contacting (e.g. mixing) the cells and the photoresponsive organic particles, the cells and the photoresponsive organic particles may be incubated at least 5 min, such as at least 10 min, at least 15 min, at least 30 min, at least 1 hour, at least 2 hours, at least 3 hours, at least 4 hours, or at least 6 hours before (i.e. prior to) contacting the cargo with the mixture of the cells and the photoresponsive organic particles.

In embodiments, the methods as taught herein may comprise contacting the cell with a photoresponsive organic particle and the cargo in an aqueous solution, such as in a cell culture medium, commonly a liquid cell culture medium. Typically, the cell culture medium will comprise a basal medium formulation as known in the art. Many basal media formulations (available, e.g., from the American Type Culture Collection, ATCC; or from Thermo Fisher Scientific, Waltham, Massachusetts, USA) can be used to culture the cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), BGJb, F-12 Nutrient Mixture (Ham), Iscove's Modified Dulbecco's Medium (IMDM), or Opti-MEM (reduced serum medium), available from Thermo Fisher Scientific (Waltham, Massachusetts, USA), and modifications and/or combinations thereof. Compositions of the above basal media are generally known in the art and it is within the skill of one in the art to modify or modulate concentrations of media and/or media supplements as necessary for the cells cultured. Such basal media formulations contain ingredients necessary for cell development, which are known *per se.* By means of illustration and not limitation, these ingredients may include inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g., glucose, sodium pyruvate, sodium acetate), etc.

In embodiments, the methods as taught herein may comprise contacting the cell with a photoresponsive organic particle and the cargo in an aqueous solution, such as a suitable transfection buffer solution, such as Dulbecco's Phosphate Buffered Saline (DPBS).

In embodiments, the methods as taught herein may comprise the prior step of providing the cells, the cargo, and the photoresponsive organic particles. Each of the cells, the cargo and the photoresponsive organic particles may be provided in an aqueous solution as taught herein.

In embodiments, the methods as taught herein may comprise providing the cells. In embodiments, the methods as taught herein may comprise providing the cells in an aqueous solution, such as in a cell culture medium, commonly a liquid cell culture medium.

In embodiments, the methods as taught herein may comprise providing the cargo. In embodiments, the methods as taught herein may comprise providing the cargo in an aqueous solution, such as in water.

In embodiments, the methods as taught herein may comprise providing the photoresponsive organic particles. In embodiments, the methods as taught herein may comprise providing the photoresponsive organic particles in an aqueous solution, such as in a cell culture medium, commonly a liquid cell culture medium.

In embodiments, the methods as taught herein may comprise providing the cells as adherent cells. In embodiments, the methods as taught herein may comprise contacting adherent cells with one or more photoresponsive organic particles. In embodiments, the methods as taught herein may comprise contacting adherent cells with one or more photoresponsive organic particles and the cargo.

In embodiments, the methods as taught herein may comprise providing the cells in suspension. In embodiments, the methods as taught herein may comprise contacting the cells in suspension with a photoresponsive organic particle and the cargo.

The terms "suspension" and "cell suspension" generally refer to a heterogenous mixture comprising cells dispersed in a liquid phase. As the mixture is generally liquid, the cells may in principle be able to, but need not, settle or sediment from the mixture.

Cells such as animal cells including human cells may be "adherent", i.e., require a surface for growth, and typically grow as an adherent monolayer on said surface (i.e., adherent cell culture), rather than as free-floating cells in a culture medium (suspension culture). Adhesion of cells to a surface, such as the surface of a tissue culture plastic vessel, can be readily examined by visual inspection under inverted microscope. Cells grown in adherent culture require periodic passaging, wherein the cells may be removed from the surface enzymatically (e.g., using trypsin), suspended in growth medium, and re-plated into new culture vessel(s). In general, a surface or substrate which allows adherence of cells thereto may be any substantially hydrophilic substrate. As known in the art, tissue culture vessels, e.g., culture flasks, well plates, dishes, or the like, may be usually made of a large variety of polymeric materials, suitably surface treated or coated after moulding in order to provide for hydrophilic substrate surfaces.

In embodiments, the methods as taught herein may thus comprise the prior step of suspending the cells in an aqueous solution, such as a cell culture medium. In embodiments, adherent cells may be first removed from a surface by a method as known in the art, for instance by tapping the cell culture vessel, by scraping the surface, or enzymatically (e.g., using trypsin or Accutase®, followed by washing).

In embodiments of the methods as taught herein, the method may be performed: in an aqueous solution; at physiological conditions; at a temperature ranging from 15°C to 40°C, preferably at a temperature ranging from 20°C to 25°C or from 32°C to 37°C; and/or at a pH ranging from about 3 to about 11, preferably from about 5 to about 7.

In embodiments, the methods as taught herein may comprise irradiating the mixture of the cell and the one or more photoresponsive organic particles with electromagnetic radiation, thereby causing permeabilization of a barrier of the cell. In embodiments, the methods as taught herein may comprise irradiating the mixture of the cell, the cargo, and the photoresponsive organic particle with electromagnetic radiation.

By irradiating the mixture, the photoresponsive organic particles capable of absorbing electromagnetic radiation may be optically activated. Upon absorption of the electromagnetic radiation by the photoresponsive organic particles, photothermal or photochemical effects can be induced. Of particular interest is the generation of a thermal or plasma induced vapour bubble, for example a vapour microbubble or a vapour nanobubble. By the generation of a vapour bubble, local high pressure waves may be induced which may alter a nearby cell, for example a cellular barrier of a nearby cell.

The phrase "generation of a vapour bubble" includes expansion of the vapour bubble, collapse of the vapour bubble, or a combination of expansion and collapse of the vapour bubble, and secondary effects that can be the result of the bubble expansion and collapse, such as pressure waves and flow of the surrounding medium. The terms "vapour bubble" or "bubble" as used herein refer to vapour nanobubbles and vapour microbubbles. Preferably, a vapour bubble may have a diameter in the range of 10 nm to 100 $\mu$m. Vapour bubbles may comprise water vapour bubbles.

The phrases "cellular barrier" or "barrier of the cell" refer to cell membranes (plasma membranes) or cell walls of eukaryotic and prokaryotic cells, and intracellular membranes, such as endosomal membranes, nuclear envelopes, mitochondrial membranes.

The terms "alter", "altering" or "alteration" refer to any way to change one or more properties of a cell, for example at least locally, such as a barrier of a cell. Altering includes but is not limited to inducing a local change in a cell's composition, for example in the composition of a barrier of the cell by adding, removing, destroying or reorganizing constituents. Altering comprises for example changing one or more physicochemical properties, such as its viscosity, porosity, density, rigidity, elasticity etc. Altering also includes local destruction or rearrangement of cellular barrier constituents, resulting in a change of the composition and/or physicochemical properties of the cellular barrier. Altering includes amongst other deforming, permeabilizing and perforating.

The terms "deform", "deforming" and "deformation" refer to any way to alter the spatial organization or structure of a cell, in particular of the cellular barrier, at least partially, for example at least locally. Examples of deforming comprise providing a cellular barrier with indentations or invaginations.

The terms "permeabilize", "permeabilizing" and "permeabilization" refer to any way to alter the permeability of a cell, in particular of the permeability of the cellular barrier, at least partially, for example at least locally. Examples of permeabilizing comprise altering the barrier composition or structure so that it becomes more permeable to a cargo.

The terms "perforate", "perforating" or "perforation" refer to any way to provide a cell, in particular the cellular barrier, at least partially, for example at least locally, with one or more openings, holes or pores. By perforating a cellular barrier, openings are created into the barrier allowing the transport of a cargo across or into that barrier.

The terms "perforate", "perforating", "perforation" and the terms "increase the permeability of", "permeabilize", "permeabilizing", "permeabilization" are interchangeably used. Similarly, the terms "opening", "hole" and "pore" may be used interchangeably herein.

The mixture is preferably irradiated by a laser such as a pulsed radiation source, although irradiation by a continuous wave radiation source can also be considered. The mixture can be irradiated by one or more pulses.

The terms "radiation" and "electromagnetic radiation" may be used interchangeably herein.

The wavelength of the radiation source may range from the ultraviolet region to the infrared region. In preferred methods, the wavelength range of the radiation used is in the visible to the infrared region, including the near infrared region.

In embodiments of the methods as taught herein, the electromagnetic radiation may be generated by a laser, such as a pulsed laser. Laser irradiation, such as irradiation by pulsed lasers, e.g. pico-, femto- and/or nanosecond pulsed lasers, can be combined with a photoresponsive organic particle in accordance with embodiments of the present invention to efficiently permeabilize a cell's barrier, e.g. by laser-induced vapour nanobubble generation. While laser irradiation may be advantageous, irradiation by another (intense) light source is not necessarily excluded, e.g. a Xenon flash lamp, to achieve the same or similar effects.

The laser pulses may each have a power density or intensity in the range of $10^4$ to $10^{17}$ W/cm$^2$, e.g. in the range of $10^6$ to $10^{15}$ W/cm$^2$, $10^7$ to $10^{14}$ W/cm$^2$, or $10^8$ to $10^{13}$ W/cm$^2$.

The laser pulses may each have a fluence (electromagnetic energy delivered per unit area) in the range of 0.01 J/cm$^2$ to 100 J/cm$^2$, 0.05 J/cm$^2$ to 50 J/cm$^2$, 0.1 J/cm$^2$ to 10 J/cm$^2$, or 0.5 J/cm$^2$ to 10 J/cm$^2$, e.g. in the range of 1 J/cm$^2$ to 10 J/cm$^2$.

The laser pulses may consist of 1 to 1000 laser pulses, such as 1 to 500 laser pulses, 1 to 100 laser pulses, 1 to 20 laser pulses, or 1 to 10 laser pulses (per cell or per cell sample). The number of laser pulses may be depending on the photoresponsive organic particle, the type of cargo, and the type of cells.

The laser pulses may have a duration in the range of 1 fs to 100 seconds (s), for instance in the range of 100 fs to 1 s, e.g. in the range of 1 ps to 0.1 s or in the range of 1 ns to 100 $\mu$s.

In embodiments of the methods as taught herein, the electromagnetic radiation may be generated by a laser, such as a pulsed laser, wherein:

- the intensity of the pulses of the laser may be at least $10^4$ W/cm$^2$, such as $10^4$ to $10^{17}$ W/cm$^2$;

- the fluence of the pulses of the laser may be at least 0.01 mJ/cm$^2$, such as 0.01 J/cm$^2$ to 100 J/cm$^2$;

- the number of pulses of the laser may be at least 1 laser pulse; such as 1 to 1000 laser pulses; and/or

- the duration of the pulses of the laser may be at least 1 fs, such as 1 fs to 100 s.

In embodiments of the methods as taught herein, the electromagnetic radiation may be generated by a laser, such as a pulsed laser, wherein the intensity of the pulses of the laser may be $10^8$ to $10^{13}$ W/cm$^2$; the fluence of the pulses of the laser may be 1 J/cm$^2$ to 10 J/cm$^2$; the number of pulses of the laser may be 1 to 10 laser pulses; and/or the duration of the pulses of the laser may be 1 ns to 100 $\mu$s.

In embodiments of the methods as taught herein, the cells, the photoresponsive organic particles, and optionally the cargo, may be contacted immediately before irradiating the mixture of the cells, the photoresponsive organic particles, and optionally the cargo. This advantageously allows to perform rapid delivery of a cargo into cells.

In embodiments of the methods as taught herein, after contacting (e.g. mixing) the cells, the photoresponsive organic particles, and optionally the cargo, the mixture of the cells, the photoresponsive organic particles, and optionally the cargo, may be incubated before (i.e. prior to) irradiating the mixture. In embodiments, after contacting (e.g. mixing) the cells, the photoresponsive organic particles, and optionally the cargo, the mixture of the cells, the photoresponsive organic particles, and optionally the cargo, may be incubated at least 1 min before (i.e. prior to) irradiating the mixture. In embodiments, after contacting (e.g. mixing) the cells, the photoresponsive organic particles, and optionally the cargo, the mixture of the cells, the photoresponsive organic particles, and optionally the cargo, may be incubated at least 5 min, such as at least 10 min, at least 15 min, at least 30 min, at least 1 hour, at least 2 hours, at least 3 hours, at least 4 hours, or at least 6 hours before (i.e. prior to) irradiating the mixture.

In embodiments, the mixture of the cells and the one or more photoresponsive organic particles is contacted with the cargo (i.e. the cargo is added to the mixture) immediately before irradiating (e.g. applying laser irradiation to) the mixture. In embodiments, the mixture of the cells and the one or more photoresponsive organic particles is contacted with the cargo (i.e. the cargo is added to the mixture) at most 15 min, at most 10 min, at most 5 min , at most 2 min, at most 1 min, at most 30 seconds, or at most 10 seconds before irradiating the mixture. Thereby, the cargo is present in the mixture to enter into the cells upon permeabilization of a barrier of the cell.

In embodiments, the mixture of the cells and the one or more photoresponsive organic particles is contacted with the cargo (i.e. the cargo is added to the mixture) immediately after irradiating the mixture. In embodiments, the mixture of the cells and the one or more photoresponsive organic particles is contacted with the cargo (i.e. the cargo is added to the mixture) at most 15 min, at most 10 min, at most 5 min , at most 2 min, at most 1 min, at most 30 seconds, or at most 10 seconds after irradiating the mixture. Thereby, the permeabilized cells allow entry of the cargo into the cells before the pores that are created in the cells will be closed again.

The methods as taught herein may be suitable to alter, manipulate and/or treat cells. In embodiments, the methods may be in particular suitable to perforate or permeabilize cells, in particular cellular barriers. By the methods as taught herein, transient pores can be formed into a cellular barrier, such as a cell membrane or cell wall, allowing the delivering of a cargo in the cells. In embodiments, the methods as taught herein may be in particular suitable for use in drug delivery, in intracellular delivery of cargo, in cell therapy, in immunotherapy, in gene therapy and in transfection of cells for example stem cells or T cells.

In embodiments, the methods as taught herein may be suitable for use in intracellular delivery of nucleic acids, including oligonucleotides, siRNA, mRNA or pDNA. In embodiments, the methods as taught herein may be also suitable for use in the intracellular delivery of nucleoproteins, including ribonucleoproteins, such as Cas9/gRNA. Furthermore, the methods as taught herein may be suitable for use in the intracellular delivery of peptides and proteins, such as nanobodies or antibodies. In addition, the methods may be suitable for use in the intracellular delivery of polysaccharides, such as labelled polysaccharides allowing detection of the cells. The methods as taught herein furthermore allow to alter, treat and/or manipulate cells with high throughput.

The methods as taught herein may as well be useful for the delivery of a cargo into a cell of a human or animal body.

A further aspect provides a photoresponsive organic particle as defined herein and a cargo as defined herein, for use in an *in vivo* method of delivering a cargo into a cell of a subject. For instance, photoresponsive organic particles and a cargo may be administered *in vivo* to a subject, for instance in the proximity of cells of the subject, e.g. by injection into a tissue such as subcutaneous injection, and the cells may be irradiated with electromagnetic radiation, thereby causing permeabilization of a barrier of the cells and delivering the cargo into the cells. The cargo may be a therapeutic agent. The method may advantageously be a method of treatment.

Hence, a further aspect provides a photoresponsive organic particle as defined herein and a cargo as defined herein, for use in a method of therapy or treatment of a disease in a subject, wherein a cargo is delivered to a cell of a subject.

An aspect provides one or more photoresponsive organic particles as defined herein and a cargo as defined herein, for use in an *in vivo* method of delivering a cargo into a cell of a subject, the method comprising:

- administering the one or more photoresponsive organic particles to the surrounding (i.e. in the proximity) of the cell of the subject;

- irradiating at least part of the surrounding of the cell of the subject, thereby causing permeabilization of a barrier of the cell; and

- administering the cargo to the surrounding of the cell of the subject, thereby delivering the cargo into the cell;

or the method comprising:

- administering the one or more photoresponsive organic particles and the cargo to the surrounding of the cell of the subject; and

- irradiating at least part of the surrounding of the cell of the subject, thereby causing permeabilization of a barrier of the cell and delivering the cargo into the cell.

A further aspect relates to one or more photoresponsive organic particles as defined herein and a cargo as defined herein, for use in a method of therapy or treatment of a disease in a subject, wherein a cargo is delivered to a cell of a subject, the method comprising:

- administering the one or more photoresponsive organic particles to the surrounding (i.e. in the proximity) of the cell of the subject;

- irradiating at least part of the surrounding of the cell of the subject, thereby causing permeabilization of a barrier of the cell; and

- administering the cargo to the surrounding of the cell of the subject, thereby delivering the cargo into the cell;

or the method comprising:

- administering the one or more photoresponsive organic particle and the cargo to the surrounding of the cell of the subject; and

- irradiating at least part of the surrounding of the cell of the subject, thereby causing permeabilization of a barrier of the cell and delivering the cargo into the cell.

Related aspects provide:

- a method of treatment of a disease in a subject, wherein a cargo is delivered *in vivo* to a cell of a subject in need of such a treatment, the method comprising administering a therapeutically effective amount of one or more photoresponsive organic particles as defined herein and a cargo as defined herein to the subject.
- the use of one or more photoresponsive organic particles as defined herein and a cargo as defined herein for the manufacture of a medicament for therapy or treatment of a disease, wherein a cargo is delivered to a cell of a subject.
- the use of one or more photoresponsive organic particles as defined herein and a cargo as defined herein for therapy or treatment of a disease in a subject, wherein a cargo is delivered *in vivo* to a cell of a subject.

In embodiments, the photoresponsive organic particles as taught herein and the cargo as taught herein such as the therapeutic agent may be administered separately. In embodiments, the photoresponsive organic particles as taught herein and the cargo as taught herein such as the therapeutic agent may be dosed independently, i.e. are present in a kit of parts in different unit doses or dosage forms. Said separate dosage forms can be administered simultaneously and/or at different time points, such as chronologically staggered, that is at different time points. The photoresponsive organic particle as taught herein and the cargo as taught herein can be administered by the same route or by different routes.
In embodiments, the photoresponsive organic particles as taught herein and the cargo as taught herein such as the therapeutic agent may be administered sequentially. In embodiments, the photoresponsive organic particles as taught herein and the cargo as taught herein such as the therapeutic agent may be administered separately and sequentially. In embodiments, the photoresponsive organic particles may be administered before the cargo such as the therapeutic agent. In embodiments, the photoresponsive organic particles may be administered immediately before the cargo such as the therapeutic agent.
In embodiments, the photoresponsive organic particles may be administered to the surrounding of the cells at least 1 min before administration of the cargo, such as the therapeutic agent. In embodiments, the photoresponsive organic particles may be administered to the surrounding o the cell at least 5 min, such as at least 10 min, at least 15 min, at least 30 min, at least 1 hour, at least 2 hours, at least 3 hours, at least 4 hours, or at least 6 hours before administration of the cargo, such as the therapeutic agent.

In embodiments, the irradiation step is performed after the administration of the one or more photoresponsive organic particles. In embodiments, the irradiation step is performed before or after the administration of the cargo.

In embodiments of the methods or uses as taught herein, the method may comprise:

- administering the one or more photoresponsive organic particle to the surrounding (i.e. in the proximity) of the cell of the subject;

- irradiating at least part of the surrounding of the cell of the subject, thereby causing permeabilization of a barrier of the cell; and

- administering the cargo to the surrounding of the cell of the subject, thereby delivering the cargo into the cell;

In embodiments, the photoresponsive organic particles as taught herein and the cargo as taught herein such as the therapeutic agent may be administered simultaneously. In embodiments, the photoresponsive organic particles as taught herein and the cargo as taught herein such as the therapeutic agent may be administered separately and simultaneously.

In embodiments of the methods or uses as taught herein, the method may comprise:

- administering the one or more photoresponsive organic particles and the cargo to the surrounding of the cell of the subject; and

- irradiating at least part of the surrounding of the cell of the subject, thereby causing permeabilization of a barrier of the cell and delivering the cargo into the cell.

In embodiments, the photoresponsive organic particles as taught herein and the cargo as taught herein such as the therapeutic agent may be comprised in a composition for simultaneous administration or in a kit of parts for simultaneous or sequential administration.

As used herein, the phrases "administering the one or more photoresponsive organic particles to the surrounding of the cells" or "administering the one or more photoresponsive organic particles in the proximity of the cells" refer to administering the photoresponsive organic particles within a distance of the cell that allow inducing permeabilization of a barrier of the cell after irradiation with electromagnetic radiation.

In certain embodiments of the methods as taught herein, the distance between the photoresponsive organic particle and the cell may be at most 10 $\mu$m, such as from about 0 $\mu$m to about 10 $\mu$m. For instance, the distance between the photoresponsive organic particle and the cell may be at most 1 $\mu$m, at most 100 nm, at most 10 nm, or at most 1 nm.

In embodiments of the photoresponsive organic particle and cargo for use as taught herein: the largest distance between two points of the one or more photoresponsive organic particles may be about 100 nm to about 2000 nm, e.g. about 250 nm to about 1250 nm; preferably about 300 nm to about 1200 nm, about 400 nm to about 1100 nm, or about 500 nm to about 1000 nm.

In embodiments of the photoresponsive organic particle and cargo for use as taught herein: the cell may be an animal cell; the cell may be a human cell; and/or the cell may be an immune cell; preferably wherein the immune cell is a T cell, a lymphocyte, a macrophage, a dendritic cell, a monocyte, a NK cell, a NKT cell, a B cell, a neutrophil, a granulocyte, a microglial cell, or a Langerhans cell.

In embodiments of the photoresponsive organic particle and cargo for use as taught herein, the electromagnetic radiation is generated by a laser, such as a pulsed laser.

In embodiments, the treatment may comprise performing laser irradiation of the photoresponsive organic particles as taught herein, in particular pulsed laser irradiation of the photoresponsive organic particles as taught herein. Accordingly, in embodiments, the treatment as taught herein comprises laser-assisted treatment.

In embodiments of the photoresponsive organic particle and cargo for use as taught herein, administering the photoresponsive organic particle and the cargo to the surrounding of the cell may comprise injection, such as subcutaneous injection or intravascular injection. In embodiments, administering the photoresponsive organic particle and the cargo to the surrounding of the cell may comprise intratumoral injection. Injection allows delivery of the photoresponsive organic particles and/or the cargo such as the therapeutic agent directly to the surrounding of the cells by a minimally invasive technique, thereby reducing the risks and pain for the patient and increasing the patient's well-being.

In embodiments, the *in vivo* methods may be useful for vaccination purpose. For instance, cells may be transfected *in vivo* with a cargo, such as mRNA and/or proteins, for vaccination purposes.

The photoresponsive organic particles as taught herein and the cargo as taught herein such as the therapeutic agent allow treatment, such as laser-assisted treatment, of a disease or condition in a subject.

The terms "subject", "individual" or "patient" can be used interchangeably herein, and typically and preferably denote humans, but may also encompass reference to non-human animals, preferably warm-blooded animals, even more preferably mammals, such as, e.g., non-human primates, rodents, canines, felines, equines, ovines, porcines, and the like. The term "non-human animals" includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In certain embodiments, the subject is a non-human mammal. In certain embodiments, the subject is a human subject. The term does not denote a particular age or sex. Thus, adult and newborn subjects, as well as fetuses, whether male or female, are intended to be covered. Examples of subjects include humans, dogs, cats, cows, goats, and mice. The term subject is further intended to include transgenic species.

Suitable subjects may include without limitation subjects presenting to a physician for a screening for a disease or condition, subjects presenting to a physician with symptoms and signs indicative of a disease or condition, subjects diagnosed with a disease condition, and subjects who have received an alternative (unsuccessful) treatment for a disease or condition.

As used herein, a phrase such as "a subject in need of treatment" includes subjects that would benefit from treatment of a given condition. Such subjects may include, without limitation, those that have been diagnosed with said condition, those prone to develop said condition and/or those in who said condition is to be prevented.

The terms "treat" or "treatment" encompass both the therapeutic treatment of an already developed disease or condition, such as the therapy of an already developed disease or condition, as well as prophylactic or preventive measures, wherein the aim is to prevent or lessen the chances of incidence of an undesired affliction, such as to prevent occurrence, development and progression of a disease or condition. Beneficial or desired clinical results may include, without limitation, alleviation of one or more symptoms or one or more biological markers, diminishment of extent of disease, stabilized (i.e., not worsening) state of disease, delay or slowing of disease progression, amelioration of the disease state, and the like. The term may encompass *ex vivo* or *in vivo* treatments.

The uses and methods as taught herein allow to administer a therapeutically effective amount of the active compound, such as the photoresponsive organic particle as taught herein and/or the cargo as taught herein, in subjects having a disease which will benefit from such treatment. The term "therapeutically effective amount" as used herein, refers to an amount of active compound that elicits the biological or medicinal response in a subject that is being sought by a surgeon, researcher, veterinarian, medical doctor or other clinician, which may include inter alia alleviation of the symptoms of the disease or condition being treated.

The term "therapeutically effective dose" refers to an amount of an active compound, such as the photoresponsive organic particle as taught herein and/or the cargo as taught herein, that when administered brings about a positive therapeutic response with respect to treatment of a patient having the disease or condition being treated.

Appropriate therapeutically effective doses of an active compound, such as the photoresponsive organic particle as taught herein and/or the cargo as taught herein, may be determined by a qualified physician with due regard to the nature of the agent, the disease condition and severity, and the age, size and condition of the patient.

In certain embodiments, the active compound, such as the photoresponsive organic particle as taught herein and/or the cargo as taught herein, e.g. the therapeutic agent, may be formulated into and administered as pharmaceutical formulations or pharmaceutical compositions.

In certain embodiments, the active compound, such as the photoresponsive organic particle as taught herein and/or the cargo as taught herein, e.g. the therapeutic agent, may be formulated into a kit of parts and administered simultaneously or sequentially.

In embodiments, the photoresponsive organic particles may be comprised in a pharmaceutical formulation. In embodiments, the cargo such as the therapeutic agent may be comprised in a pharmaceutical formulation.

In embodiments, the photoresponsive organic particle and the cargo such as the therapeutic agent may be comprised in a pharmaceutical formulation.

The photoresponsive organic particles or pharmaceutically acceptable salts thereof, and/or the cargo such as the therapeutic agent or pharmaceutically acceptable salts thereof can be formulated as an aqueous solution.

Accordingly, an aspect relates to a pharmaceutical formulation comprising a photoresponsive organic particles as taught herein. A further aspect provides a pharmaceutical formulation comprising a photoresponsive organic particles as taught herein and a cargo as taught herein such as a therapeutic agent.

A further aspect relates to a pharmaceutical formulation as taught herein for use in a method of therapy or treatment in a subject. Preferably, the subject is a human subject.

The terms "pharmaceutical composition", "pharmaceutical formulation" or "pharmaceutical preparation" may be used interchangeably herein and refer to a mixture comprising an active ingredient. The terms "composition" or "formulation" may likewise be used interchangeably herein.

The terms "active ingredient", "active compound" or "active component" can be used interchangeably and broadly refer to a compound or substance which, when provided in an effective amount, achieves a desired outcome. The desired outcome may be therapeutic and/or prophylactic. Typically, an active ingredient may achieve such outcome(s) through interacting with and/or modulating living cells or organisms.

The term "active" in the recitations "active ingredient" or "active component" refers to "pharmacologically active" and/or "physically active".

In embodiments, the pharmaceutical formulations as taught herein may comprise in addition to the photoresponsive organic particles and the cargo such as the therapeutic agent one or more pharmaceutically acceptable excipients.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic compositions may be contemplated.

Pharmaceutical compositions as intended herein may be formulated for essentially any route of administration, such as without limitation, oral administration (such as, e.g., oral ingestion), parenteral administration (such as, e.g., subcutaneous, intravenous or intramuscular injection or infusion), and the like.

For example, for oral administration, pharmaceutical compositions may be formulated in the form of pills, tablets, lacquered tablets, coated (e.g., sugar-coated) tablets, granules, hard and soft gelatin capsules, aqueous, alcoholic or oily solutions, syrups, emulsions or suspensions. In an example, without limitation, preparation of oral dosage forms may be is suitably accomplished by uniformly and intimately blending together a suitable amount of the active compound in the form of a powder, optionally also including finely divided one or more solid carrier, and formulating the blend in a pill, tablet or a capsule. Exemplary but non-limiting solid carriers include calcium phosphate, magnesium stearate, talc, sugars (such as, e.g., glucose, mannose, lactose or sucrose), sugar alcohols (such as, e.g., mannitol), dextrin, starch, gelatin, cellulose, polyvinylpyrrolidine, low melting waxes and ion exchange resins. Compressed tablets containing the pharmaceutical composition can be prepared by uniformly and intimately mixing the active ingredient with a solid carrier such as described above to provide a mixture having the necessary compression properties, and then compacting the mixture in a suitable machine to the shape and size desired. Moulded tablets maybe made by moulding in a suitable machine, a mixture of powdered compound moistened with an inert liquid diluent. Suitable carriers for soft gelatin capsules and suppositories are, for example, fats, waxes, semisolid and liquid polyols, natural or hardened oils, etc.

Preferably the pharmaceutical formulation may be formulated for parenteral administration, e.g. by injection.

In embodiments, the pharmaceutical composition may be formulated as an aqueous solution. For example, for parenteral administration, pharmaceutical compositions may be advantageously formulated as solutions, suspensions or emulsions with suitable solvents, diluents, solubilisers or emulsifiers, etc. Suitable solvents are, without limitation, water, physiological saline solution or alcohols, e.g. ethanol, propanol, glycerol, in addition also sugar solutions such as glucose, invert sugar, sucrose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid. The photoresponsive organic particles and/or the cargo or pharmaceutically acceptable salts thereof can also be lyophilized. The obtained lyophilizates can be used, for example, for injection or infusion preparation or for the production of injection or infusion preparations.

In embodiments, the photoresponsive organic particles may be comprised in a kit of parts.

In embodiments, the photoresponsive organic particles and the cargo such as the therapeutic agent may be comprised in a kit of parts.

A further aspect relates to a kit of parts comprising a photoresponsive organic particles as taught herein. A further aspect provides a kit of parts comprising a photoresponsive organic particles as taught herein and a cargo such as a therapeutic agent as taught herein.

A further aspect thus relates to a kit of parts as taught herein for use in a method of therapy or treatment in a subject. Preferably, the subject is a human subject.

The terms "kit of parts", "kit-of-parts" or "kit" as used herein refer to a product containing components necessary for carrying out the specified uses or methods, packed so as to allow their transport and storage. Materials suitable for packing the components comprised in a kit include crystal, plastic (e.g., polyethylene, polypropylene, polycarbonate), bottles, flasks, vials, ampules, paper, envelopes, or other types of containers, carriers or supports. Where a kit comprises a plurality of components, at least a subset of the components (e.g., two or more of the plurality of components) or all of the components may be physically separated, e.g., comprised in or on separate containers, carriers or supports. The components comprised in a kit may be sufficient or may not be sufficient for carrying out the specified uses or methods, such that external reagents or substances may not be necessary or may be necessary for performing the methods, respectively. Typically, kits are employed in conjunction with standard laboratory equipment, such as liquid handling equipment, environment (e.g., temperature) controlling equipment, analytical instruments, etc. In addition to the photoresponsive organic particles as taught herein and/or the cargo as taught herein such as the therapeutic agent, optionally provided on arrays or microarrays, the present kits may also include excipients such as solvents useful in the specified uses or methods. Typically, the kits may also include instructions for use thereof, such as on a printed insert or on a computer readable medium. The terms may be used interchangeably with the term "article of manufacture", which broadly encompasses any man-made tangible structural product, when used in the present context.

The present application also provides aspects and embodiments as set forth in the following Statements:

1. An *in vitro* or *ex vivo* method for delivering a cargo into a cell, the method comprising:

- contacting a cell with one or more photoresponsive organic particles and a cargo, wherein the cargo is not bound to the one or more photoresponsive organic particles, and wherein the organic particle is selected from the group consisting of a polymer-based particle, a protein-based particle, a lipid-based particle, and a combination thereof, thereby obtaining a mixture of the cell, the cargo, and the one or more photoresponsive organic particles; and

- irradiating the mixture of the cell, the cargo, and the one or more photoresponsive organic particles with electromagnetic radiation, thereby causing permeabilization of a barrier of the cell and delivering the cargo into the cell; or

- contacting a cell with one or more photoresponsive organic particles, wherein the organic particle is selected from the group consisting of a polymer-based particle, a protein-based particle, a lipid-based particle, and a combination thereof, thereby obtaining a mixture of the cell and the one or more photoresponsive organic particles;

- irradiating the mixture of the cell and the one or more photoresponsive organic particles with electromagnetic radiation, thereby causing permeabilization of a barrier of the cell; and

- contacting the mixture of the cell and the one or more photoresponsive organic particles with a cargo, thereby delivering the cargo into the cell.

2. The method according to statement 1, wherein the largest distance between two points of the one or more photoresponsive organic particles is about 250 nm to about 1250 nm; preferably about 300 nm to about 1200 nm, about 400 nm to about 1100 nm, or about 500 nm to about 1000 nm.

3. The method according to statement 1 or 2, wherein the cargo is selected from the group consisting of a nucleic acid, a protein, a chemical substance, a polysaccharide, and combinations thereof; preferably wherein the cargo is a nucleic acid; more preferably wherein the cargo is mRNA or plasmid DNA.

4. The method according to any one of statements 1 to 3, wherein the photoresponsive organic particle is a photoresponsive polymer-based particle; preferably wherein the photoresponsive organic particle is a photoresponsive polymer-based particle selected from a polydopamine (PD) particle, a poly(N-phenylglycine) (PNPG) particle, a poly-2-phenyl-benzobisthiazole (PPBBT) particle, a porphyrin particle, a phthalocyanine particle, or a polypyrrole particle.

5. The method according to any one of statements 1 to 3, wherein the photoresponsive organic particle is a polymer-based particle, a protein-based particle, or a lipid-based particle comprising a light absorbing molecule; preferably wherein the photoresponsive organic particle is a polymer-based particle, a protein-based particle, or a lipid-based particle loaded with or functionalized with a light absorbing molecule.

6. The method according to statement 5, wherein the light absorbing molecule is a molecule selected from the group consisting of a light absorbing dye, a naturally occurring light absorber, and a synthetic light absorber.

7. The method according to any one of statements 1 to 6, wherein the polymer-based particle comprises poly(DL-lactic-co-glycolic acid) (PLGA), poly(lactic acid) (PLA), polycaprolactone (PCL), ethyl cellulose, cellulose acetophthalate, cellulose, polyvinyl alcohol, polyethylene glycol, gelatine, collagen, silk, alginate, hyaluronic acid, dextran, starch, polycarbonate, polyacrylate, polystyrene, methoxy-PEG-polylactide, poly(alkyl cyanoacrylate) (PACA), poly(D,L-lactide-co-glycolide (PLGH), poly(allylamine hydrochloride), or a polyoxazoline.

8. The method according to any one of statements 1 to 7, wherein the photoresponsive organic particle is functionalized on the surface; preferably wherein the photoresponsive organic particle is coated with one or more compounds selected from the group consisting of albumin, polyethyleneimine (PEI), polyvinylpyrrolidone (PVP), polyethylene glycol (PEG), poly(diallyldimethylammonium chloride) (PDDAC), poly(allylamine hydrochloride) (PAH), polyamidoamine (PAA), poly(amino-co-ester) (PAE), poly[2-(N,N-dimethylamino)ethyl methacrylate] (PDMAEMA), hyaluronic acid (HA), gelatin, polyglycerol, a cyclodextrin (CD), dextran, cellulose, silica, polyoxazoline, sulfobetaine-silane (SBS), a cationic lipid, a neutral lipid, an anionic lipid, chitosan, and poly-L-Lysine.

9. The method according to any one of statements 1 to 8, wherein the photoresponsive organic particle is biodegradable.

10. The method according to any one of statements 1 to 9, wherein the photoresponsive organic particle is a polydopamine particle; preferably wherein the photoresponsive organic particle is a polydopamine particle coated with albumin.

11. The method according to any one of statements 1 to 10, wherein the cell is an animal cell; preferably wherein the cell is a human cell.

12. The method according to any one of statements 1 to 11, wherein the cell is an immune cell; preferably wherein the immune cell is T cell, a lymphocyte, a macrophage, a dendritic cell, a monocyte, a NK cell, a NKT cell, a B cell, a neutrophil, a granulocyte, a microglial cell, or a Langerhans cell.

13. The method according to any one of statements 1 to 12, wherein the electromagnetic radiation is generated by a laser, such as a pulsed laser; preferably wherein:

- the intensity of the pulses of the laser may be at least $10^4$ W/cm$^2$, such as $10^4$ to $10^{17}$ W/cm$^2$;

- the fluence of the pulses of the laser may be at least 0.01 mJ/cm$^2$, such as 0.01 J/cm$^2$ to 100 J/cm$^2$;

- the number of pulses of the laser may be at least 1 laser pulse; such as 1 to 1000 laser pulses; and/or

- the duration of the pulses of the laser may be at least 1 fs, such as 1 fs to 100 s.

14. One or more photoresponsive organic particle as defined in any one of statements 1 to 10 and a cargo as defined in any one of statements 1 to 10, for use in a method of therapy in a subject, wherein a cargo is delivered to a cell of a subject, the method comprising:

- administering the one or more photoresponsive organic particles to the surrounding of the cell of the subject;

- irradiating at least part of the surrounding of the cell of the subject, thereby causing permeabilization of a barrier of the cell; and

- administering the cargo to the surrounding of the cell of the subject, thereby delivering the cargo into the cell;

or the method comprising:

- administering the one or more photoresponsive organic particle and the cargo to the surrounding of the cell of the subject; and

- irradiating at least part of the surrounding of the cell of the subject, thereby causing permeabilization of a barrier of the cell and delivering the cargo into the cell.

15. The one or more photoresponsive organic particles and cargo for use according to statement 14, wherein the largest distance between two points of the one or more photoresponsive organic particles is about 250 nm to about 1250 nm; preferably about 300 nm to about 1200 nm, about 400 nm to about 1100 nm, or about 500 nm to about 1000 nm.

16. The one or more photoresponsive organic particles and cargo for use according to any one of statements 14 or 15, wherein:

- the cell is an animal cell;

- the cell is a human cell;

- the cell is an immune cell; preferably wherein the immune cell is a T cell, a lymphocyte, a macrophage, a dendritic cell, a monocyte, a NK cell, a NKT cell, a B cell, a neutrophil, a granulocyte, a microglial cell, or a Langerhans cell; and/or

- wherein the electromagnetic radiation is generated by a laser, such as a pulsed laser.

The above aspects and embodiments are further supported by the following non-limiting examples.

## EXAMPLES

### Example 1: Method according to an embodiment of the invention for delivery of mRNA or dextran in both adherent (HeLa cells) and suspension cells (Jurkat and human T cells)

### Materials and methods

#### Synthesis of polydopamine nanoparticles (NPs)

The synthesis of polydopamine nanoparticles was performed according to a previously described protocol with some slight modifications (Ju et al., Biomacromolecules, 2011, 12, 625). Briefly, 70 mg of dopamine hydrochloride (Sigma-Aldrich) was dissolved into 20 mL HyClone water (VWR, HyPure, Cell Culture Grade) of 45-50 °C. Next, 295 μL of a 1 M NaOH solution was added under vigorous stirring (molar ratio: 1/0.8). The colour of the solution immediately turned yellowish and then changed after some time into a dark brown colour. The solution was allowed to stir for about 7 hours. The size of the particles was carefully measured every hour by DLS. The solution was collected and washed for several times with HyClone water by centrifugation (4.000 rcf, 10 minutes). Next, to reduce potential aggregation, tip sonication was applied for 30 seconds (10% A, Branson digital sonifier, Danbury, USA).

#### Functionalization of polydopamine nanoparticles with bovine serum albumin

To increase the colloidal stability of 500 nm PD NPs, functionalization with a bovine serum albumin (BSA, VWR Chemicals, Biotechnology grade, USA) layer was performed. Briefly, PD NPs were mixed with a solution prepared from mixing DPBS with 10 mg/mL BSA solution at a volume ratio of 1:1. The mixture was then allowed to react by vigorous stirring overnight and the remaining unbound BSA was removed by several washing steps with HyClone water (4000 rcf, 5 minutes). Finally, the BSA coated 500 nm PD NP dispersed in HyClone water were stored at 4°C.

#### Physicochemical characterization of polydopamine nanoparticles (PD NPs)

SEM images were acquired by using an FEI Quanta 200F (Thermo Scientific), operating at an accelerating voltage of 20 kV. The PD NPs were dried on a silicon wafer one day prior to the SEM measurements. The dried samples were additionally coated with a gold layer.

Dynamic light scattering (DLS, Zetasizer Nano ZS, Malvern instruments Co., Ltd.) and Nanoparticle tracking analysis (NTA, NanoSight LM10, Malvern Panalytical, UK) were used to measure the hydrodynamic diameter. NTA was performed in scattering mode with a 488 nm laser. NTA was also used to determine the nanoparticle concentration. The UV/VIS spectrum was measured with a Nanodrop 2000c spectrophotometer (Thermofischer, Rockford, USA).

*Vapour nanobubble generation and threshold*

The VNB generation from PD NPs was visualized by dark field microscopy upon the application of a pulsed nanosecond laser (OpoletteTM HE 355 LD, OPOTEK Inc, CA, 7 ns pulse duration, 561 nm wavelength). The laser pulse energy was measured with an energy meter (J-25MBHE&LE, Energy Max-USB/RS sensors, Coherent) which was synchronized with the pulsed laser. An EMCCD camera (Cascade II: 512, Photometrics, Tucson, USA) was used to record short movies which were processed with NIS elements software (Nikon, Japan). The bubble threshold, which is the 90% probability that the PD NPs in the irradiated area will form VNBs, was determined after fitting the data with a Boltzmann sigmoid function.

*Cell cultures*

HeLa cells (ATTC® CCL-2™) were cultured in DMEM/F-12 medium (Gibco-Invitrogen) which was further supplemented with 10% fetal bovine serum (FBS), 100 U/mL Penicillin/Streptomycin (Gibco-Invitrogen) and 2 mM L-glutamine (Gibco-invitrogen). The cells were frequently passaged upon reaching confluency and kept in a humidified incubator (37 °C, 5% $CO_2$).

Jurkat cells (American Type Culture Collection, ATCC® TIB-152) were cultured in RPMI 1640 medium which was additionally supplemented with 10% fetal bovine serum (FBS), 100 U/mL Penicillin/Streptomycin (Gibco-Invitrogen) and 2 mM L-glutamine (Gibco-invitrogen). During the experiments, Jurkat cells were kept in culture in a humidified incubator (37 °C, 5% $CO_2$) and frequently passaged.

The human T cells were generated from buffy coats from blood donors after obtaining informed consent (Biobank Red Cross Flanders) and were used following the guidelines of the Medical Ethical Committee of the Ghent University Hospital (Belgium, Ghent). First, the isolation of peripheral blood mononuclear cells (PBMCs) from buffy coats was performed via density centrifugation using Lymphoprep (Alere Technologies AS, Oslo, Norway). The isolated PBMCs were then stimulated with Immunocult Human CD3/CD28 T cell Activator (Stemcell Technologies, Vancouver, Canada). The PBMCs were kept in culture for 7 days in IMDM medium (Invitrogen, Merelbeke, Belgium) supplemented with 10% fetal calf serum (FCS, Bovogen, Melbourne, Australia), 100U/ml penicillin (Invitrogen, Merelbeke, Belgium), 100 $\mu$g/ml streptomycin, 2 mM glutamine and 5 ng/ml IL-2 (Roche) for 7 days. Next, the expanded T cells were collected and restimulated with 1 $\mu$g/ml phytohemagglutinin (Remel Europe, KENT, UK) in the presence of a feeder mixture consisting of irradiated PBMCs (40 Gy) and JY cells (50Gy). After 4 days, 5 ng/ml IL-2 (Roche Diagnostics, Mannheim, Germany) was added. Ten to 14 days later, these culture expanded T cells were used and referred to as human T cells in the manuscript.

***Incubation and mixing photoporation of dextran into adherent and suspension HeLa cells***

*Incubation photoporation of dextran on adherent HeLa cells*

HeLa cells were seeded in a 96-well plate at 20.000 cells per well and incubated overnight in an incubator (37 °C, 5% $CO_2$). Next, the adherent HeLa cells were washed once with DPBS and new HeLa cell culture medium containing PD-BSA NPs with an increasing concentration was added which were allowed to incubate for 30 minutes. After 30 minutes of incubation, the cells were washed once with Opti-MEM medium to remove the excess of unbound PD-BSA NPs. After washing, 50 $\mu$L of fresh Opti-MEM medium containing 1 mg/mL 500 kDa Fluorescein isothiocyanate-dextran (FD500) was added to the cells. Photoporation was then performed with a fluence of 1.6 J/cm$^2$ on a custom built photoporation setup (pulse duration: 3 ns, 532 nm wavelength). Mock photoporation was performed with the PD-BSA NPs but without any cargo. The cells were then washed several times with cell culture medium to remove the excess of FD500 so as to avoid further spontaneous uptake of the marker by pinocytosis.

*Mixing photoporation of dextran on adherent HeLa cells*

HeLa cells were seeded in a 96-well plate at 20.000 cells per well and incubated overnight in an incubator (37 °C, 5% $CO_2$). Next, the adherent HeLa cells were washed once with Opti-MEM medium and 25 $\mu$L of Opti-MEM medium containing an increasing concentration of PD500-BSA NPs was added. An additional 25 $\mu$L of Opti-MEM containing FD500 was added such that the final concentration of FD500 in the mixture was 1 mg/mL. Photoporation was then performed with a fluence of 1.6 J/cm$^2$ on the photoporation setup (pulse duration: 3 ns, 532 nm wavelength). The cells were then washed several times with cell culture medium to remove the excess of FD500.

*Mixing photoporation of dextran on suspension HeLa cells*

The suspension of HeLa cells was first collected after performing trypsinization. The cells were dispersed in cell culture medium, counted and washed twice with Opti-MEM medium. Next, 24 $\mu$L of Opti-MEM medium containing 40.000 cells (doubling time of HeLa cells approximately 24 hour) was added per well in a 96-well plate. The cells were then supplemented with another 25 $\mu$L of Opti-MEM medium containing an increasing concentration of PD500-BSA NPs and FD500. The final concentration of FD500 in the 50 $\mu$L mixture was 1 mg/mL. All the conditions had a final volume of 50 $\mu$L and a quick spin-down (5-10 seconds, 500 rcf) of the 96-well plate was performed such that the cells were sedimented on the bottom of the 96-well plate. Note: a quick spin-down was performed for all the different methods. The cells were then washed several times with cell culture medium to remove the excess of FD500.

### Mixing photoporation of suspension HeLa cells for mRNA transfection

A similar protocol as described above was followed. Briefly, 20 $\mu$L of Opti-MEM medium containing 40.000 cells was added per well in a 96-well plate. The cells were then supplemented with another 25 $\mu$L of Opti-MEM medium containing $16 \times 10^7$ NPs/mL. Next, the mixture was supplemented with 5 $\mu$L of mRNA encoding for enhanced green fluorescent protein (eGFP-mRNA, 353 kDa, 996 nucleotides), resulting in a final mRNA concentration of 0.3 $\mu$M or 0.1 $\mu$g/$\mu$L. All the conditions had a final volume of 50 $\mu$L and a quick spin-down (5-10 seconds, 500 rcf) of the 96-well plate was performed such that the cells were on the bottom of the 96-well plate. Photoporation was performed at a fluence of 1.6 J/cm$^2$ and the cells were supplemented with 200 $\mu$L complete DMEM/F-12 medium. After 24 hours of incubation the cells were ready to be harvested for eGFP read-out (by flow cytometry or confocal microscopy) and viability measurement after 24 hours (see details below).

### Photoporation of Jurkat cells and human T cells for the delivery of dextran

Photoporation of Jurkat and human T cells was performed in a similar manner as described before. Briefly, the cells were first washed several times with Opti-MEM (3 min, 500 rcf) in order to remove the medium. Next, 24 $\mu$L of Opti-MEM containing 250.000 Jurkat cells or 1 million human T cells was put into a single well of a 96-well plate (VWR, plastic bottom). Next, 25 $\mu$L of Opti-MEM containing the PD-BSA NPs at the desired concentration was immediately added to the cells. Finally, 1 $\mu$L of Opti-MEM containing FD500 (stock concentration 50 mg/mL) was added to the 49 $\mu$L mixture. For the untreated cells (UTC) which served as a negative control, cells were dispersed in a total volume of 50 $\mu$L Opti-MEM medium. An additional 'FD500 control' was included to correct for any spontaneous uptake of FD500 by cells, for which cells were first dispersed in 49 $\mu$L of Opti-MEM and 1 $\mu$L of FD500 (stock concentration of 50 mg/mL) was added to the mixture. Also a 'photoporation control' condition was included to check the effect of performing the photoporation procedure in the absence of cargo. Here, cells were dispersed in 25 $\mu$L of Opti-MEM and supplemented with another 25 $\mu$L of Opti-MEM containing PD-BSA NPs of the desired concentration. The photoporation control was performed for every tested PD-BSA NPs concentration such that FD500 delivery could be corrected for a shift in the background fluorescence in the presence of PD-BSA NPs. All the conditions had a final volume of 50 $\mu$L and a quick spin-down (5-10 seconds, 500 rcf) of the 96-well plate was performed such that the cells were on the bottom of the 96-well plate. Photoporation was then performed operating at a fluence of 1.6 J/cm$^2$ and only took 3-4 seconds per well. The wells were then supplemented with 200 $\mu$L fresh complete cell culture medium. In order to assess the cytotoxicity of the photoporation procedure, the exact same steps were performed but with the exception that FD500 was left out during the photoporation process. After the photoporation, the cells were supplemented with 200 $\mu$L cell culture medium and left to incubate for the indicated amount of time (i.e. Jurkat cells: 24 hours, Human T cells: 4 hours, 24 hours and 48 hours).

### Photoporation of Jurkat cells and human T cells for the mRNA transfection

Transfection with mRNA was performed similarly to the photoporation of FD500s. Briefly, cells were first washed several times with Opti-MEM medium. Both Jurkat (250.000 cells) and human T cells (1 million cells), suspended in 20 $\mu$L Opti-MEM, were transferred into a single well of a 96-well plate and were mixed with 25 $\mu$L of Opti-MEM containing PD-BSA NPs at the desired concentration. Next 5 $\mu$L of eGFP-mRNA (stock concentration of 1 $\mu$g/$\mu$L, CleanCap, Trilink Biotechnologies, San Diego, CA, USA) was quickly added to the 45 $\mu$L mixture and the cells were quickly spun-down (5 seconds centrifugation at 500 rcf) such that the cells were at the bottom of the 96-well plate. Photoporation was then performed as mentioned before. For the untreated cells (UTC), the cells were dispersed in a total volume of 50 $\mu$L Opti-MEM medium. For the mRNA control, the cells were first dispersed in 45 $\mu$L of Opti-MEM and 5 $\mu$L of mRNA eGFP (stock concentration of 1 $\mu$g/$\mu$L) was added to the cells. For the photoporation control, the cells were dispersed in 25 $\mu$L of Opti-MEM and supplemented with another 25 $\mu$L of Opti-MEM containing PD-BSA NPs at the desired concentration. Following photoporation treatment, another 200 $\mu$L of full cell culture medium was added to the cells and incubated for 24 hours in an incubator (37 °C, 5% CO$_2$).

*Flow cytometry*

Flow cytometry was used to assess the delivery efficiency (FD500) or transfection efficiency (eGFP-mRNA) of the photoporation procedure. Following photoporation, the cells were washed several times (3 minutes, 500 rcf) with DPBS (-/-) to remove the excess of FD500 or cell debris. After the washing steps, flow buffer containing DPBS, 1% bovine serum albumin (BSA) and 0.1% NaN3 was added to the cells. Furthermore, 0.5 $\mu$M TO-PRO-3 iodide was used to stain the dead cells. The samples were then measured with a CytoFLEX flow cytometry (Beckman Coulter, Krefeld, Germany) using respectively a 488 nm and 637 nm excitation laser. eGFP fluorescence was detected with a 525/40 nm bandpass filter, while the signal of the TO-PRO-3 probe was detected with a 660/20 nm bandpass filter. The TO-PRO-3 positive cells (i.e. dead cells) were excluded for quantification of the FD500 and eGFP signal.

*Confocal microscopy*

The Nikon A1R HD laser scanning confocal microscope, operating at room temperature, was used to record confocal images with respectively a 10x (CFI Plan Apo Lambda 10X, 0.45 NA), 20x (CFI Plan Apochromat VC 20X, 0.75 NA) and 60x (CFI SR Plan Apo IR 60XAC WI, NA 1.27) objective. Excitation was performed with a 488 nm laser and the fluorescent signal was detected at 525/50nm (MHE57030, A1 Filter Cube 525/50) by A1-DUG-2 GaAsP Multi Detector Unit (this means following detectors/channel: GaAsp PMT for 488 nm).

*Viability assay*

Cell viability was determined by using the CellTiter Glo® metabolic viability assay according to the manufacturer's recommendations. Briefly, for both the adherent and suspension (i.e. trypsinized) HeLa cells, 100 $\mu$L of HeLa cell culture medium was supplemented with 100 $\mu$L CellTiter Glo solution in each well of the 96-well plate. For the suspension cells (i.e. Jurkat and human T cells), 50.000 cells were diluted in 100 $\mu$L cell culture medium and another 100 $\mu$L of CellTiter Glo solution was added to the 96-well plate. The plates were then allowed to stir on a shaker for about 10 minutes (100 rpm) and 100 $\mu$L of the mixture was transferred into a white opaque 96-well plate (Greiner Bio-One, Belgium). The read-out of the luminescent signal was performed by a luminometer (plate reader, GloMax, Promega).

*mRNA transfection by nucleofection*

Activated primary human T cells were transfected with eGFP-mRNA by a 4D-Nucleofector™ according to the manufacturer's instructions with a P3 Primary Cell 4D-Nucleofector™ X Kit (V4XP-3032) (Lonza, Breda, The Netherlands), Briefly, 1x10⁶ cells were resuspended in P3 nucleofector solution, combined with 2$\mu$g eGFP-mRNA (total concentration: 0.3 $\mu$M) and transferred a 16-well Nucleocuvette™ strip. Transfection was obtained using pulse program EO-115 (High functionality). Immediately after transfection, the cells were supplemented with 80$\mu$L pre-heated culture medium and 40$\mu$L was transferred to a 96-well plate containing 160$\mu$L pre-heated culture medium (total mRNA concentration: 0.1 $\mu$g/$\mu$L or 0.3 $\mu$M). Cells were incubated at 37°C, 5% $CO_2$ for 24 hours prior analysis of cell viability and mRNA expression by flow cytometry.

**Results**

*Synthesis, functionalization and physicochemical characterization of polydopamine nanoparticles*

The experiments started with the synthesis of polydopamine nanoparticles (PD NPs). A relatively large particle size of 0.5 $\mu$m was aimed for so as to generate large enough VNBs and membrane pores for efficient entry of large mRNA molecules into cells (FIG. 1). FIG. 1 provides a schematical representation of polydopamine-sensitized photoporation for the delivery of a cargo such as mRNA into cells: First, polydopamine (PD) nanoparticles (NPs) and mRNA are added to cells after which an intense nanosecond laser pulse is applied. This leads to the creation of VNBs around the PD NPs, which eventually collapse and transiently permeabilize the cells, allowing mRNA molecules to enter into the cells. mRNA transfected cells are indicated in dark grey.

The synthesis of the PD NPs involves spontaneous oxidation of dopamine hydrochloride (dopamine.HCl) followed by polymerization. Several parameters were optimized, such as the temperature and ratio of dopamine.HCl : NaOH. When a fixed ratio of dopamine.HCl : NaOH (1:0.8) and temperature (45-50°C) were used, a gradual growth of the nanoparticles could be observed in function of the reaction time (mixture becomes yellow and eventually turns into a dark-brown solution). After 7 hours of stirring, PD NPs with a hydrodynamic diameter (Z-average) of $636 \pm 41$ nm were obtained (FIG. 2A). Subsequently the PD NPs were sonicated with a tip sonicator for 30 seconds to remove any agglomerated PD NPs which led to PD NPs with a final hydrodynamic diameter of $465 \pm 6$ nm (FIG. 2A, arrow). For simplicity we will refer to these particles as 0.5 $\mu$m PD NPs from here on. The synthesis of spherical PD NPs was confirmed by scanning electron microscopy (SEM) (FIG. 2B). The corresponding size distribution showed a core size of 454 $\pm$ 96 nm (FIG. 2C) which is very similar to the hydrodynamic diameter as measured with DLS. The PD NPs had a slightly positive zeta-potential of +17.9 $\pm$ 2.1 mV in HyClone water which is likely due to the protonation of amine groups. Next, the colloidal stability of these uncoated PD NPs was assessed in Opti-MEM, which was shown before to be a suitable medium for mRNA transfections with limited mRNA degradation. A fast size increase in Opti-MEM medium ($\leq$ 10 minutes incubation time) was observed (FIG. 2D). This is in line with previous reports where uncoated PD NPs were found to have an excellent colloidal stability in water but not in phosphate buffered saline (PBS). Therefore, to maintain colloidal stability of the PD NPs in Opti-MEM as transfection medium, an extra functionalization step with bovine serum albumin (BSA) was performed. The functionalization is based on the reaction between amine groups of BSA and catechol/quinine of polydopamine, which can be achieved through overnight incubation at room temperature. After several washing steps with HyClone water, the particle's hydrodynamic diameter slightly increased to 513 $\pm$ 6 nm which gave a first indication that coating with BSA was successful. In addition, the zeta-potential turned slightly negative after the coating procedure (-19.4 $\pm$ 0.8 mV in HyClone water), providing further proof that the BSA coating was successful. Most importantly, BSA coated PD NPs did no longer aggregate in Opti-MEM (FIG. 2E). This improved colloidal stability can be attributed to respectively the electrostatic and steric repulsion of the BSA molecules.

As the PD-BSA NPs will be used for photoporation, for which laser light absorption is an important requirement, we additionally performed UV/VIS measurements. The starting solution of dopamine.HCl had a sharp extinction peak in the UV region (FIG. 2F, striped line). Instead, 500 nm PD NPs had a broad extinction spectrum ranging from the UV up to the near-infrared (NIR) region (FIG. 2F, dark grey dots), which remained virtually unaltered after BSA coating (FIG. 2F, light grey dots). In a next step, we tested the possibility to create VNBs by irradiation of the PD-BSA NPs with pulsed laser light (7 ns pulse duration, 561 nm wavelength). The occurrence of VNBs was visualized by dark field microscopy as they intensely scatter light during their lifetime. While before laser irradiation no VNBs can be seen (FIG. 2G), several VNBs appeared upon illumination with a laser pulse (FIG. 2H). By counting the number of VNBs as a function of the applied laser pulse fluence and by fitting the plotted data with a Boltzmann sigmoidal function, the bubble threshold could be determined, which is defined as the laser fluence at which 90% of the particles in the irradiated region generate VNBs. This resulted in a bubble threshold of 1.06 J/cm$^2$ (FIG. 2I, dashed line on the left of the grey area), which falls within the range of fluences that have been reported before to generate VNBs from AuNP. For cell experiments it is preferred that all PD NPs create VNBs so that a fluence of 1.60 J/cm$^2$ was selected for further photoporation experiments.

### Comparison between incubation and mixing method according to embodiments of the invention for photoporation of dextran or mRNA into HeLa cells

Next, we investigated the potential of PD-BSA NPs as photoporation sensitizers for the delivery of macromolecules into cells. To deliver proof-of-concept, this was first done on HeLa cells as an often used model cell line for photoporation, in which FITC-dextran of 500 kDa (FD500) was delivered as a large model macromolecule with a comparable molecular weight as eGFP-mRNA (353 kDa, 996 nucleotides). We tested two types of protocols to expose cells to the nanoparticles. In the first protocol, the photosensitive particles were incubated for 30 minutes with HeLa cells followed by a washing step and laser exposure. This protocol is referred to herein as the 'incubation method'. This method is identical to how photoporation with AuNPs is normally performed. In the second protocol, PD NPs were simply added to the cell medium and irradiated immediately without further incubation or washing. This protocol is referred to herein as the 'mixing method'. Since the final aim is to transfect T-cells, which are suspension cells, the second protocol was additionally tested on trypsinized HeLa cells in suspension.

The percentage of FD500 positive cells was determined by flow cytometry after gating for viable cells (i.e. negative for TO-PRO-3 iodide) ('% Efficiency') and gradually increased for increasing concentrations of PD-BSA NPs in all three cases (FIG. 3A-C). This was accompanied by a gradual decrease in cell viability, measured two hours after treatment with the CellTiter-Glo assay. This assay was chosen since it provides similar results as trypan blue counting.

By multiplying the percentage of viable cells with the percentage of FD500 positive cells, the delivery yield can be calculated, which is the percentage of living FD500 positive cells relative to the starting population. The highest yield values were obtained for 8x10$^7$ NPs/mL, 8x10$^7$ NPs/mL and 16x10$^7$ NPs/mL for the incubation method, mixing method on adherent cells and mixing method on suspension cells, respectively. The differences between the corresponding yield values were, however, not statistically significant (FIG. 3D). Also the relative mean fluorescence per cell was virtually the same for those conditions, with an rMFI value of about 15. Together these results show that incubating cells for 30 minutes with the PD-BSA NPs gives no additional advantage and that the simpler and faster 'mixing method' works equally well for BSA-PD NPs, both on adherent and suspension cells.

As there is no washing step included in the mixing method to remove unbound PD-BSA NPs, we wanted to clarify to which extent bound vs. unbound nanoparticles contributed to the delivery effect. To test this, 8x10$^7$ NPs/mL PD-BSA NPs were added to adherent HeLa cells similar to the mixing protocol. Next, cells were immediately washed twice with Opti-MEM to remove any unbound PD-BSA NPs. The PD NPs that remain must then be adsorbed to the cells. Upon laser irradiation the percentage of FD500 positive cells decreased only slightly, while there was no significant difference in rMFI. This result proves that the cell permeabilizing effect in the mixing method still comes from cell-associated BSA-coated PD NPs which apparently can bind quite rapidly to cells. While the unbound PD NPs that remain present in solution still create vapour bubbles, they do not contribute to the delivery process.

Next we wanted to verify successful mRNA delivery in HeLa's. In this experiment, HeLa cells were trypsinized and transferred to Opti-MEM containing 0.3 $\mu$M eGFP-mRNA and 16x10$^7$ NPs/mL PD-BSA NP/mL. According to the mixing protocol the sample was subsequently immediately irradiated with pulsed laser light. After photoporation with mRNA, the cells were again supplemented with complete cell culture medium for 24 hours after which transfection efficiency and viability was quantified. A transfection efficiency of 46 $\pm$ 1% GFP positive cells (FIG. 3E, black bar) was obtained with a 24 hour viability of 88 $\pm$ 13% (FIG. 3E, light grey bar). Combined this gives a transfection yield percentage of 41 $\pm$ 7% living transfected cells (Fig. 3E, bar with stripes). Confocal microscopy images gave further support that photoporation of HeLa cells resulted in bright eGFP expressing cells (FIG. 3F). This result is markedly better as compared to results where AuNPs were used as sensitizers resulting a eGFP-mRNA transfection yield in HeLa cells of 16% (20 % transfection efficiency with a cell viability of 80%) (results not shown). The better results for PD-BSA NPs could potentially be explained by their larger size (i.e., approximately 500 nm PD-BSA NPs versus approximately 70 nm AuNPs), which may result in bigger vapour bubbles and bigger membrane pores which could be beneficial for more efficient entry of large mRNA molecules. In addition, larger pores may take a longer time to repair, which gives more time for molecules to enter the cells. Another reason that could explain the enhanced transfection efficiency could be the generation of liquid jets from large PD NP induced vapour bubbles, as has previously been proposed for AuNP clusters as well.

### *Method according to an embodiment of the invention for photoporation of dextran or mRNA into Jurkat cells*

Following successful proof-of-concept experiments on HeLa cells, we moved on to Jurkat cells as an immortalized human lymphocyte suspension cell-line which is frequently used as a model for primary human T cells. Jurkat cells dispersed in Opti-MEM were mixed with an increasing concentration of PD-BSA NPs together with FD500 and immediately (i.e. < 1 minute) photoporated with a laser fluence of 1.60 J/cm$^2$ (3 ns pulse duration, 532 nm wavelength), similar to the mixing procedure performed before on HeLa cells in suspension. It was found that both the percentage of FD500 positive cells ('% Efficiency') as well as the relative mean fluorescence intensity (rMFI) increased with an increasing concentration of PD-BSA NPs (FIG. 4A). As usual this was accompanied by a gradual drop in cell viability, here measured after 24 hours by CellTiter Glo metabolic assay (FIG. 4A). Starting from $16 \times 10^7$ PD-BSA NPs/mL the delivery yield remained rather constant at 30-35%. Together this shows that one can increase the percentage of FD500 positive cells by using higher nanoparticle concentrations, but that the overall delivery yield does not increase further due to a loss in cell viability. In any case, a delivery yield of 30-35% is again substantially higher than what was previously obtained with photoporation using AuNPs for which the yield was limited to approximately 20% (delivery efficiency of about 50% with a 2 hours viability of about 40%, results not shown). Even more remarkably, the rMFI ($\pm$38) obtained with PD-BSA NPs is about 8-fold higher compared to the rMFI with AuNP ($\pm$5), which can again be explained by the fact that bigger pores are created, allowing more efficient influx of large macromolecules in the cytosol.

Next, the potential to deliver eGFP-mRNA into Jurkat cells was assessed. Therefore, similar as described above, Jurkat cells (250.000 cells/well) dispersed in Opti-MEM were mixed with PD-BSA NPs (64x10$^7$ NPs/mL) together with mRNA (0.3 $\mu$M). Photoporation resulted in a transfection efficiency of 45 $\pm$ 14% (FIG. 4B, black bars) with a viability of 50 $\pm$ 15% (FIG. 4B, light grey bars), amounting to a cell transfection yield of about 21% (FIG. 4B, bars with stripes). The transfection of Jurkat cells was apparent from flow cytometry results (not shown) and also from confocal microscopy images (FIG. 4C). This result was better than what was previously reported for eGFP-mRNA photoporation of Jurkat cells with AuNP for which a transfection yield of 16% was obtained. Most importantly, it was much better as compared to nucleofection, i.e. a state-of-the-art electroporation platform, for which a eGFP-mRNA transfection yield of only 4% could be obtained in Jurkat cells.

### *Method according to an embodiment of the invention for photoporation of dextran or mRNA into human T cells*

Finally, we proceeded to the *ex vivo* modification of hard-to-transfect human T cells using the mixing method. Experiments were performed in an identical manner as for Jurkat cells, with the only exception that 1 million T cells were seeded per well. First, the optimal concentration of PD-BSA NPs was screened for by FD500 delivery. Similar as for Jurkat cells, an increase in the delivery efficiency could be observed for both the percentage of FD500 positive cells and rMFI values (FIG. 5A). The corresponding viability after 24 hours decreased with an increasing concentration of PD-BSA NPs (FIG. 5A, black dots). Both 250 and 500x10$^7$ NPs/mL performed equally well in terms of delivery yield, reaching ~30% living and FD500 positive cells, quite similar to what was obtained for Jurkat cells. For 1000x10$^7$ NPs/mL the delivery yield was slightly but significantly less (~25%), mainly due to higher cytotoxicity. Confocal microscopy images confirmed successful delivery of FD500 into human T cells for increasing PD-BSA NP concentrations (FIG. 5B, II-IV) in comparison to the control (FIG. 5B, I).

Next, the potential to transfect human T cells with mRNA was assessed. An increased transfection efficiency could again be observed for an increasing concentration of PD-BSA NPs.

Indeed, transfection efficiencies of 22 $\pm$ 2%, 29 $\pm$ 3% and 30 $\pm$ 6% could be obtained for a concentration of respectively 250x10$^7$, 500x10$^7$ and 1000x10$^7$ PD-BSA NPs/mL (FIG. 5C, black bars). The corresponding cell viabilities after 24 hours were respectively around 87 $\pm$ 4%, 63 $\pm$ 2% and 44 $\pm$ 9% (FIG. 5C, black dots). The rMFI were 4 $\pm$ 1, 5 $\pm$ 2 and 6 $\pm$ 3 for the same increasing NP concentration (FIG. 5C). Exemplary confocal images are shown in FIG. 5D and confirmed by flow cytometry (results not shown). Again, these results showed that the percentage of transfected cells increased by using higher nanoparticle concentrations, but that at a certain NP concentration, the overall transfection yield may deteriorate again due to a loss in cell viability.

In a final experiment, mRNA was delivered into human T cells via photoporation and compared to nucleofection as a benchmarking technology. Nucleofection of human T cells (using the optimized pulse program EO-115) in the presence of 0.3 $\mu$M mRNA resulted in a transfection efficiency of 62.4 $\pm$ 27.9 % (FIG. 5E, black bar). However, a strong decrease in cell viability after 24 hours was observed as measured with the metabolic CellTiter Glo assay. Indeed, 24 hours after nucleofection only 11.5 $\pm$ 3.6 % of the electroporated human T cells remained viable (FIG. 5E, light grey bar). This is also in line with previous studies where nucleofection was shown to induce a very high level of acute cell death in primary T cells. This resulted in a transfection yield of 18.7 $\pm$ 1.0% for photoporation, thus clearly outperforming nucleofection with a transfection yield of only 7.5 $\pm$ 4.8 % (FIG. 5F). In addition the transfection results were much more variable in case of nucleofection, while this was much less for photoporation.

In conclusion, polydopamine nanoparticles with a size of around 500 nm were successfully synthesized. These PD NPs were further functionalized with BSA to improve colloidal stability without interfering with the particles ability to adhere to the cell membrane. The results demonstrated successful vapour bubble formation from PD NPs upon irradiation with ns pulsed laser light, which is the first demonstration of vapour bubble formation from organic nanoparticles. Interestingly, polydopamine-sensitized laser-assisted photoporation could be applied on both adherent and suspension cells by simply mixing the nanoparticles with the cells and immediately applying laser irradiation. With this method, 500 kDa FITC-dextran and eGFP encoding mRNA were successfully delivered into HeLa cells, as well as into hard-to-transfect Jurkat and human T cells. Importantly, about 2 times more living transfected primary human T cells were obtained after photoporation as compared to nucleofection. Considering that PD-BSA NPs were prepared from a clinically approved precursor, these methods open up the possibility to allow the clinical translation of photoporation for the production of engineered therapeutic cell products, such as CAR-T cells.

**Example 2: Methods according to embodiments of the invention to investigate the influence** of **the size of the photoresponsive organic particles on delivery yield of a cargo into cells**

### Materials and methods

BSA-functionalized polydopamine particles with different sizes were generated as described above (Example 1, Synthesis & functionalization of PD NPs) by using different reaction times. The hydrodynamic diameter of the different PD-BSA NPs was performed via the DLS. The photoporation experiments were performed on adherent HeLa cells using the mixing photoporation method (i.e., without any incubation step) and the to-be-delivered cargo was FD500.

### Results

The synthesis of PD-BSA NPs with a different size was performed in order to assess the influence of the increasing pore size on the delivery efficiency. FIG. 6A shows an increasing size distribution (intensity %) for PD-BSA NPs with an increased size. The Z-average, as measured by the DLS, for the different PD-BSA NPs in Hyclone water was respectively 134$\pm$2 nm (PDI: 0.104), 282$\pm$1 nm (PDI: 0.027), 449$\pm$2 nm (PDI: 0.101), 753$\pm$5 nm (PDI: 0.060) and 1079 $\pm$24 nm (PDI: 0.177) (FIG. 6B).

The photoporation experiments with the different sizes of polydopamine particles were performed via the mixing method on adherent HeLa cells (i.e., without any incubation step) and FD500 was delivered as model macromolecule. The following settings were used for photoporation (starting from FIG. 7): Intensity 3 = 0.88 J/cm$^2$; Intensity 5 = 1.8 J/cm$^2$; Intensity 7 = 2.72 J/cm$^2$. These are the fluences per laser pulse. To convert it to W/cm$^2$, these values must be divided by the duration of a single laser pulse, i.e. 5 ns: Intensity 3 = 1.8 10$^8$ W/cm$^2$; Intensity 5 = 3.6 10$^8$ W/cm$^2$; Intensity 7 = 5.4 10$^8$ W/cm$^2$.

For 100 nm PD-BSA NPs, only a very small increase in delivery efficiency could be observed for increasing concentrations of PD-BSA NPs and this for both intensities (intensity setting of 5 and 7, FIG. 7A and 7B respectively). The viability, measured two hours after treatment with the CellTiter-Glo assay, also remained excellent for both fluences (i.e., >80%) and this even for very high concentrations (FIG. 7A, 7B). The lack of delivery efficiency could potentially be attributed to the size of the generated pores which may not be large enough to allow the passage of the FD500 macromolecules. The saturation of the delivery yield for both fluences also hints at the limited effect on the delivery efficiency of the 100 nm PD-BSA NPs (FIG. 8A, 8B).

Photoporation with PD-BSA NPs of around 300 nm showed a clear increase of the delivery efficiency with an increasing concentration of polydopamine and this for both laser intensities (intensity setting 5 and 7, FIG. 9A and 9B respectively). The increase for intensity 7 was slightly higher compared to intensity 5 which could attributed to the creation of bigger pores. Moreover, the viability started to drop under 80% for higher concentrations of PD-BSA NPs starting from a concentration of $14.3 \times 10^8$ PD-BSA NPs/mL. The highest yield value of 26% was obtained using the intensity 7 and a concentration of $6.54 \times 10^8$ PD-BSA NPs/mL (FIG. 10A, 10B).

By increasing the PD-BSA NP size to about 500 nm, high delivery efficiencies could be achieved for lower polydopamine concentrations but were also accompanied with higher toxicities (FIG. 11A, 11B). The laser intensity had to be lowered to intensity 3 and 5. Moreover, lower concentration of polydopamine had to be used compared to 100 and 300 nm particles possibly due to the much larger created bubbles. The most optimal delivery yield of around 36% could be obtained using the intensity 5 and a concentration of $0.77 \times 10^8$ PD-BSA NPs/mL (FIG. 12A, 12B).

Again by further increasing the PD-BSA NP size (i.e., 750 nm), high delivery efficiencies could be obtained (FIG. 13A, 13B). The most optimal delivery yield of around 53% could be obtained using both intensities (i.e., 3 and 5) and a concentration of respectively $1.2 \times 10^8$ and $0.77 \times 10^8$ PD-BSA NPs/mL (FIG. 14A, 14B). The experiment was also performed for 750 nm particles for an intensity of 2 and an intensity of 4 and high delivery yields were obtained as well (results not shown).

Upon further increasing the polydopamine size up to 1000 nm, a much higher toxicity could be observed for similar laser intensities (intensity setting of 3 and 4, FIG. 15A, 15B respectively). This may suggest that for PD-BSA NPs of 1000 nm the size of the generated pores became too large in order to be repaired. The best results, where the viability still remained above 80%, could be obtained for a concentration of $0.32 \times 10^8$ PD-BSA NPs/mL (FIG. 16A, 16B). In that case, the delivery yield was around 39% which was lower in comparison to PD-BSA NPs of around 750 nm. The experiment was also performed for 1000 nm particles for an intensity of 2 and an intensity of 5 (results not shown).

In conclusion, the most optimal size which showed the most optimal delivery yield in adherent HeLa cells was the PD-BSA NPs with a hydrodynamic diameter of around 750 nm.

**Example 3: Methods according to embodiments of the invention to investigate the influence of the coating of the photoresponsive organic particles on delivery yield of a cargo into cells**

**Materials and methods**

In order to show that the delivery efficiency and hence the binding of the PD NPs with the cell membrane is independent of the surface charge, we functionalized the 500 nm PD NPs with a cationic layer of branched polyethyleneimine (bPEI, 25 kDa, Sigma Aldrich, Saint Louis, United states of America). Briefly, 5 mL of bPEI 25 kDa 10% m/V solution was added to 5 mL of PD NPs in Hyclone water. The mixture was then sonicated with a tip sonicator (Branson ultrasonic corporation, Danbury, United States of America) for 20-30 seconds (10% A). The suspension was vigorously shaken overnight in the IKA® MS 3 basic shaker (IKA, Staufen im Breisgau, Germany). Finally, the unbound bPEI was removed through washing the suspension for at least 3 times with Hyclone water (4000 rcf, 5 minutes).

Similarly as to the photoporation experiment with PD-BSA particles, the photoporation experiments with PD-PEI NPs were performed on adherent HeLa cells using the mixing photoporation method (i.e., without any incubation step) and the to-be-delivered cargo was FD500.

**Results**

The hydrodynamic diameter of the PD-PEI NPs was $522 \pm 24$ nm as measured by the DLS in Hyclone water. The zeta-potential of the PD-PEI NPs was around $+30 \pm 1$ mV, which confirmed the successful coating with bPEI. Similarly as observed with the PD-BSA NPs, high delivery efficiencies could be achieved for an increasing concentration of PD-PEI NPs (intensity setting of 3 and 5, FIG. 17A, 17B respectively). This was also accompanied with higher toxicities for an increasing concentration of PD-PEI NPs (FIG. 17A, 17B). In comparison to the delivery yield of PD-BSA NPs PD-BSA 500 nm (FIG. 12A: maximum yield of 34%; FIG. 12B: maximum yield of 36%), PD-PEI particles showed only a slightly lower delivery yield of around 30% for both fluences (FIG. 18A, 18B).

**Example 4: Method according to an embodiment of the invention for the delivery of a cargo into a cell with uncoated photoresponsive organic particles**

**Materials and methods**

This experiment was used to test whether uncoated polydopamine NPs could also be used in the photoporation procedure. As a proof of concept, we performed traditional photoporation on adherent HeLa cells with the uncoated PD particles (i.e., with incubation). As the uncoated PD NPs were unstable in biological media such as Opti-MEM, we opted to use complete cell culture medium as this could enhance the colloidal stability of the PD NPs through the formation of a protein corona.

**Results**

The percentage of FD500 positive cells gradually increased for increasing concentrations of uncoated PD NPs. This was accompanied by a gradual decrease in cell viability, measured two hours after treatment with the CellTiter-Glo assay (FIG. 19A, 19B). The maximum yield obtained for both intensity 1 and 2 were around 33 and 32% showing the possibility to perform photoporation without any coating (FIG. 20A, 20B).

**Example 5: Method according to embodiments of the invention for delivery of dextran in adherent HeLa cells**

**Materials and methods**

*Synthesis of polyvinyl alcohol coated polypyrrole nanoparticles (PPy NPs)*

Synthesis of polyvinyl alcohol (PVA)-coated polypyrrole NPs was performed according to a previous protocol with slight modifications (S. E. Zayan et al., AIP Conference Proceedings, 2019, 2190, 020025). Briefly, 250 mg of FeCl3 (Sigma-Aldrich) and 20 mg of PVA were dissolved in 20 mL pure water (Hyclone, VWR). The mixture was stirred for 1 hour via magnetic stirring. Next, 100 $\mu$L of pyrrole monomer (TCI Chemical) was mixed with 1.4 mL Hyclone water and then added dropwise to the 18.5 mL mixture at room temperature. The successful polymerization reaction could be visualized by the change in color of the mixture (gradual change of color into a black solution). After 3 hours, the mixture was washed several times via centrifugation and finally dispersed into Hyclone water. The solution was sonicated with a tip sonication for 30 seconds (10% A).

*Physicochemical characterization of polyvinyl alcohol coated polypyrrole nanoparticles (PPy NPs)*

Dynamic light scattering (DLS, Zetasizer Nano ZS, Malvern instruments Co., Ltd.) and Nanoparticle tracking analysis (NTA, NanoSight LM10, Malvern Panalytical, UK) were used to measure the hydrodynamic diameter and zeta-potential. NTA was performed in scattering mode with a 488 nm laser. NTA was also used to determine the nanoparticle concentration.

*Vapour nanobubble generation*

The vapour nanobubble generation from PPy NPs upon the application of a pulsed nanosecond laser (OpoletteTM HE 355 LD, OPOTEK Inc, CA, 7 ns pulse duration, 561 nm wavelength) was visualized by dark field microscopy. The laser pulse energy was measured with an energy meter (J-25MBHE&LE, Energy Max-USB/RS sensors, Coherent) which was synchronized with the pulsed laser. A sCMOS camera (Prime RM16C, Photometrics, Tucson, USA) was used to record short movies which were processed with NIS elements software (Nikon, Japan).

*Cell cultures*

HeLa cells (ATTC® CCL-2™) were cultured in DMEM/F-12 medium (Gibco-Invitrogen) which was further supplemented with 10% fetal bovine serum (FBS), 100 U/mL Penicillin/Streptomycin (Gibco-Invitrogen) and 2 mM L-glutamine (Gibco-invitrogen). The cells were cultured in a humidified incubator (37 °C, 5% $CO_2$) and frequently passaged upon reaching confluency.

*Flow cytometry*

Flow cytometry was used to assess the delivery efficiency (FD500) of the photoporation procedure. Following photoporation, the cells were washed several times (3 minutes, 500 rcf) with DPBS (-/-) to remove the excess of FD500 or cell debris. After the washing steps, flow buffer containing DPBS, 1% bovine serum albumin (BSA) and 0.1% NaN3 was added to the cells. Furthermore, 0.5 $\mu$M TO-PRO-3 iodide was used to stain the dead cells. The samples were then measured with a CytoFLEX flow cytometry (Beckman Coulter, Krefeld, Germany) using respectively a 488 nm and 637 nm excitation laser. eGFP fluorescence was detected with a 525/40 nm bandpass filter, while the signal of the TO-PRO-3 probe was detected with a 660/20 nm bandpass filter. The TO-PRO-3 positive cells (i.e. dead cells) were excluded for quantification of the FD500 and eGFP signal.

### Viability assay

Cell viability was determined by using the CellTiter Glo® metabolic viability assay according to the manufacturer's recommendations. Briefly, adherent HeLa cells were trypsinized and 100 μL of the HeLa cell suspension was transferred to a 96-well plate and supplemented with 100 μL CellTiter Glo solution. The plates were then allowed to stir on a shaker for about 10 minutes (100 rpm) and 100 μL of the mixture was transferred into a white opaque 96-well plate (Greiner Bio-One, Belgium). The read-out of the luminescent signal was performed by a luminometer (plate reader, GloMax, Promega).

### Incubation and mixing photoporation of dextran into adherent HeLa cells

### Incubation photoporation of dextran on adherent HeLa cells

HeLa cells were seeded in a 96-well plate at 20.000 cells per well and incubated overnight in an incubator (37 °C, 5% $CO_2$). Next, the adherent HeLa cells were washed once with DPBS and new HeLa cell culture medium containing PPY NPs with an increasing concentration was added which were allowed to incubate for 30 minutes. After 30 minutes of incubation, the cells were washed once with Opti-MEM medium to remove unbound PPy NPs. After washing, 50 μL of fresh Opti-MEM medium containing 1 mg/mL 500 kDa Fluorescein isothiocyanate-dextran (FD500) was added to the cells. Photoporation was then performed with a fluence of 1.6 J/cm$^2$ on a custom built photoporation setup (pulse duration: 3 ns, 532 nm wavelength). Mock photoporation was performed with the PPy NPs but without any cargo. The cells were then washed several times with cell culture medium to remove excess FD500 so as to avoid further uptake of the marker by pinocytosis.

### Mixing photoporation of dextran on adherent HeLa cells

HeLa cells were seeded in a 96-well plate at 20.000 cells per well and incubated overnight in an incubator (37 °C, 5% $CO_2$). Next, the adherent HeLa cells were washed once with Opti-MEM medium and 25 μL of Opti-MEM medium containing an increasing concentration of FD500-PPy NPs was added. An additional 25 μL of Opti-MEM containing FD500 was added such that the final concentration of FD500 in the mixture was 1 mg/mL. Photoporation was then performed with a fluence of 1.6 J/cm$^2$ on the photoporation setup (pulse duration: 3 ns, 532 nm wavelength). The cells were then washed several times with cell culture medium to remove excess FD500.

### Results

The synthesis of polyvinyl alcohol (PVA)-coated polypyrrole NPs was performed in order to demonstrate the ability of PPy NPs to form vapour nanobubbles upon excitation by a pulsed laser. FIG. 21 shows physicochemical characterization of the synthesized PVA-coated PPy NPs. The Z-average and zeta-potential of the PPy NPs, as measured by DLS in Hyclone water was respectively 614±1 nm and +24.3±0.7 mV. The zeta-potential of +24.3±0.7 mV confirmed successful coating with PVA. The occurrence of VNBs was visualized by dark field microscopy. While before laser irradiation no VNBs can be seen (FIG. 22, left image), several VNBs appeared upon illumination with a laser pulse (FIG. 22, right image).

Next, the potential of PPy NPs as photoporation sensitizers for the delivery of macromolecules into cells was investigated. To deliver proof-of-concept, this was done on HeLa cells as an often used model cell line for photoporation, in which FITC-dextran of 500 kDa (FD500) was delivered as a large model macromolecule with a comparable molecular weight as eGFP-mRNA (353 kDa, 996 nucleotides).

FIG. 23 shows the delivery efficiency of 500 kDa FITC-dextran (FD500) and cell viability (2 hours after photoporation) via the mixing method in adherent HeLa cells. The percentage of FD500 positive cells was determined by flow cytometry after gating for viable cells (i.e. negative for TO-PRO-3 iodide) ('% Efficiency') and gradually increased for increasing concentrations of PPy-PVA NPs. This was accompanied by a gradual decrease in cell viability, measured two hours after treatment with the CellTiter-Glo assay.

By multiplying the percentage of viable cells with the percentage of FD500 positive cells, the delivery yield can be calculated, which is the percentage of living FD500 positive cells relative to the starting population. FIG. 24 shows the yield percentage of viable FD500 positive cells for PPy-sensitized photoporation. The highest yield value was obtained for 32x10$^7$ NPs/mL.

**Example 6: Method according to embodiments of the invention for delivery of dextran in adherent HeLa cells**

**Materials and methods**

*Synthesis of trypan blue-loaded lipid nanoparticles (LNP-TB)*

The synthesis of trypan blue-loaded lipid nanoparticles was based on a previous protocol with some modifications (D.Miranda et al., Biomater. Sci. 2019, 7, 3158). Briefly, 486 $\mu$L of DOTAP in chloroform (25 mg/ml, Avanti Lipids) and 215 $\mu$L of DSPE-PEG2000 in chloroform (25 mg/ml, Avanti Lipids) were transferred into a round-bottom flask. Next, 7.46 mg cholesterol powder (Avanti Lipids) was added along with 800 $\mu$L of excess chloroform. The final ratio of DOTAP:DSPE-PEG:cholesterol was 10:9:1. The mixture was then transferred to the rotary evaporator. The solution was rotated for 20-30 minutes under vacuum condition at 250 rpm and 40°C to form a dry lipid film. A trypan blue working solution was prepared by diluting 500 $\mu$L of the trypan blue stock solution (0.4%, Gibco) into 2 mL of deionized water. The resulting lipid film was then rehydrated with 2 mL of 1 mg/ml trypan blue working solution and vortexed for approximately 30 seconds. Overnight dialysis was performed with a 50 kDa MWCO cassette (Spectra/Por, Float-A-Lyzer G2) to remove unconjugated trypan blue.

*Physicochemical characterization of trypan blue-loaded lipid nanoparticles (LNP-TB)*

Dynamic light scattering (DLS, Zetasizer Nano ZS, Malvern instruments Co., Ltd.) and Nanoparticle tracking analysis (NTA, NanoSight LM10, Malvern Panalytical, UK) were used to measure the hydrodynamic diameter and zeta-potential. NTA was performed in scattering mode with a 488 nm laser. NTA was also used to determine the nanoparticle concentration.

*Vapour nanobubble generation*

The vapour nanobubble generation from LNP-TB was visualized by dark field microscopy upon the application of a pulsed nanosecond laser (OpoletteTM HE 355 LD, OPOTEK Inc, CA, 7 ns pulse duration, 561 nm wavelength). The laser pulse energy was measured with an energy meter (J-25MBHE&LE, Energy Max-USB/RS sensors, Coherent) which was synchronized with the pulsed laser. An EMCCD camera (Prime RM16C, Photometrics, Tucson, USA) was used to record short movies which were processed with NIS elements software (Nikon, Japan).

*Cell cultures*

HeLa cells (ATTC® CCL-2™) were cultured in DMEM/F-12 medium (Gibco-Invitrogen) which was further supplemented with 10% fetal bovine serum (FBS), 100 U/mL Penicillin/Streptomycin (Gibco-Invitrogen) and 2 mM L-glutamine (Gibco-invitrogen). The cells were cultured in a humidified incubator (37 °C, 5% $CO_2$) and frequently passaged upon reaching confluency.

*Flow cytometry*

Flow cytometry was used to assess the delivery efficiency (FD500) of the photoporation procedure. Following photoporation, the cells were washed several times (3 minutes, 500 rcf) with DPBS (-/-) to remove excess FD500 or cell debris. After the washing steps, flow buffer containing DPBS, 1% bovine serum albumin (BSA) and 0.1% NaN3 was added to the cells. Furthermore, 0.5 $\mu$M TO-PRO-3 iodide was used to stain the dead cells. The samples were then measured with a CytoFLEX flow cytometry (Beckman Coulter, Krefeld, Germany) using a 488 nm and 637 nm excitation laser. eGFP fluorescence was detected with a 525/40 nm bandpass filter, while the signal of the TO-PRO-3 probe was detected with a 660/20 nm bandpass filter. The TO-PRO-3 positive cells (i.e. dead cells) were excluded for quantification of the FD500 and eGFP signal.

*Viability assay*

Cell viability was determined by using the CellTiter Glo® metabolic viability assay according to the manufacturer's recommendations. Briefly, adherent HeLa cells were trypsinized and 100 $\mu$L of the HeLa cell suspension was transferred to a 96-well plate and supplemented with 100 $\mu$L CellTiter Glo solution. The plates were then allowed to stir on a shaker for about 10 minutes (100 rpm) and 100 $\mu$L of the mixture was transferred into a white opaque 96-well plate (Greiner Bio-One, Belgium). The read-out of the luminescent signal was performed by a luminometer (plate reader, GloMax, Promega).

*Incubation and mixing photoporation of dextran into adherent HeLa cells*

*Incubation photoporation of dextran on adherent HeLa cells*

HeLa cells were seeded in a 96-well plate at 20.000 cells per well and incubated overnight in an incubator (37 °C, 5% $CO_2$). Next, the adherent HeLa cells were washed once with DPBS and new HeLa cell culture medium containing LNP-TB with an increasing concentration was added which were allowed to incubate for 30 minutes. After 30 minutes of incubation, the cells were washed once with Opti-MEM medium to remove unbound LNP-TB. After washing, 50 $\mu$L of fresh Opti-MEM medium containing 1 mg/mL 500 kDa Fluorescein isothiocyanate-dextran (FD500) was added to the cells. Photoporation was then performed with a fluence of 1.6 $J/cm^2$ on a custom built photoporation setup (pulse duration: 3 ns, 532 nm wavelength). Mock photoporation was performed with the LNP-TB but without any cargo. The cells were then washed several times with cell culture medium to remove excess FD500 so as to avoid further uptake of the marker by pinocytosis.

*Mixing photoporation of dextran on adherent HeLa cells*

HeLa cells were seeded in a 96-well plate at 20.000 cells per well and incubated overnight in an incubator (37 °C, 5% $CO_2$). Next, the adherent HeLa cells were washed once with Opti-MEM medium and 25 $\mu$L of Opti-MEM medium containing an increasing concentration of FD500-LNP-TB was added. An additional 25 $\mu$L of Opti-MEM containing FD500 was added such that the final concentration of FD500 in the mixture was 1 mg/mL. Photoporation was then performed with a fluence of 1.6 $J/cm^2$ on the photoporation setup (pulse duration: 3 ns, 532 nm wavelength). The cells were then washed several times with cell culture medium to remove excess FD500.

**Results**

The synthesis of trypan blue-loaded lipid nanoparticles (LNP-TB NPs) was performed in order to demonstrate the ability of LNP-TB NPs to form vapour nanobubbles upon excitation by a pulsed laser. FIG. 25 shows physicochemical characterization of the synthesized LNP-TB NPs. The Z-average and zeta-potential of the LNP-TB NPs, as measured by the DLS in Hyclone water was respectively $372\pm1$ nm and $+21.1\pm1.6$ mV. The occurrence of VNBs was visualized by dark field microscopy as they intensely scatter light during their lifetime. While before laser irradiation no VNBs can be seen (FIG. 26, left image), several VNBs appeared upon illumination with a laser pulse (FIG. 26, right image).

Next, the potential of LNP-TB NPs as photoporation sensitizers for the delivery of macromolecules into cells was investigated. To deliver proof-of-concept, this was done on HeLa cells as an often used model cell line for photoporation, in which FITC-dextran of 500 kDa (FD500) was delivered as a large model macromolecule with a comparable molecular weight as eGFP-mRNA (353 kDa, 996 nucleotides).

FIG. 27 shows the delivery efficiency of 500 kDa FITC-dextran (FD500) and cell viability (2 hours after photoporation) via the mixing method in adherent HeLa cells. The percentage of FD500 positive cells was determined by flow cytometry after gating for viable cells (i.e. negative for TO-PRO-3 iodide) ('% Efficiency') and gradually increased for increasing concentrations of LNP-TB NPs. This was accompanied by a cell viability which remained close to 100% for all concentrations of LNP-TB NPs, measured two hours after treatment with the CellTiter-Glo assay.

By multiplying the percentage of viable cells with the percentage of FD500 positive cells, the delivery yield can be calculated, which is the percentage of living FD500 positive cells relative to the starting population. FIG. 24 shows the yield percentage of viable FD500 positive cells for PPy-sensitized photoporation. The highest yield value was obtained for 32x107 NPs/mL.

**Example 7: Method according to embodiments of the invention illustrating vapour nanobubble generation using protein-based nanoparticles**

**Materials and methods**

*Synthesis of Human serum albumin ICG nanoparticles (HSA ICG NPs)*

The synthesis of Human serum albumin ICG nanoparticles was performed according to a previous protocol (Z. Sheng et al., ACS Nano 2014, 8, 12310). ICG and HSA were dissolved in a 50 mM GSH (Sigma-Aldrich, St. Louis, USA) solution at a concentration of 20 mg/mL and 80 mg/mL respectively. 1 mL ICG solution was mixed with 1 mL of the HSA solution. Subsequently, 2 mL ethanol was added to precipitate the HSA-ICG NPs. The suspension was magnetically stirred (Heidolph Instruments GmbH & CO. KG, Schwabach, Germany) at room temperature for 30 minutes. Then, the suspension was transferred with a syringe into a dialysis cassette of 8 mL with a cut-off of 10,000 Da (Thermo Fisher Scientific, Waltham, USA). After the suspension was transferred, the remaining air was removed from the cassette with another syringe. The dialysis cassette was placed with a Slide-A-Lyzer (to prevent the cassette from sinking) (Thermo Fisher Scientific, Waltham, USA) in a large beaker (around 1 L) filled with deionized water and the beaker was placed on a magnetic stirrer for 24 hours at 4°C. After dialysis, the suspension was removed out of the cassette with a syringe and stored in a falcon tube protected from light at 4°C.

### Physicochemical characterization of Human serum albumin ICG nanoparticles (HSA ICG NPs)

Dynamic light scattering (DLS, Zetasizer Nano ZS, Malvern instruments Co., Ltd.) and Nanoparticle tracking analysis (NTA, NanoSight LM10, Malvern Panalytical, UK) were used to measure the hydrodynamic diameter and zeta-potential. NTA was performed in scattering mode with a 488 nm laser. NTA was also used to determine the nanoparticle concentration.

### Vapour nanobubble generation

The vapour nanobubble generation from HSA ICG NPs was visualized by dark field microscopy upon the application of a pulsed nanosecond laser (OpoletteTM HE 355 LD, OPOTEK Inc, CA, 7 ns pulse duration, 561 nm wavelength). The laser pulse energy was measured with an energy meter (J-25MBHE&LE, Energy Max-USB/RS sensors, Coherent) which was synchronized with the pulsed laser. An EMCCD camera (Prime RM16C, Photometrics, Tucson, USA) was used to record short movies which were processed with NIS elements software (Nikon, Japan).

### Results

The synthesis of Human serum albumin ICG nanoparticles (HSA ICG NPs) was performed in order to demonstrate the ability of HSA ICG NPs to form vapour nanobubbles upon excitation by a pulsed laser. FIG. 28 shows physicochemical characterization of the synthesized HSA ICG NPs. The Z-average and zeta-potential of the HSA ICG NPs, as measured by the DLS in Hyclone water was respectively 309 nm and -11.9 mV.

The occurrence of VNBs was visualized by dark field microscopy as they intensely scatter light during their lifetime. While before laser irradiation no VNBs can be seen (FIG. 29, left image), several VNBs appeared upon illumination with a laser pulse (FIG. 29, right image).

The abovementioned examples describe the use of at least one polymer-based photoresponsive organic particle, at least one protein-based photoresponsive organic particle and at least one lipid-based photoresponsive organic particle. Additionally, experiments were carried out with different polymer-based, protein-based and lipid-based photoresponsive organic particles and/or a combination thereof (data not added). The results were comparable to the results discussed above.

### Claims

1. An *in vitro* or *ex vivo* method for delivering a cargo into a cell, the method comprising:

   - contacting a cell with one or more photoresponsive organic particles and a cargo, wherein the cargo is not bound to the one or more photoresponsive organic particles, and wherein the organic particle is selected from the group consisting of a polymer particle, a protein particle, a lipid particle, and a combination thereof, thereby obtaining a mixture of the cell, the cargo, and the one or more photoresponsive organic particles; and
   - irradiating the mixture of the cell, the cargo, and the one or more photoresponsive organic particles with electromagnetic radiation, thereby causing permeabilization of a barrier of the cell and delivering the cargo into the cell; or
   - contacting a cell with one or more photoresponsive organic particles, wherein the organic particle is selected from the group consisting of a polymer particle, a protein particle, a lipid particle, and a combination thereof, thereby obtaining a mixture of the cell and the one or more photoresponsive organic particles;
   - irradiating the mixture of the cell and the one or more photoresponsive organic particles with electromagnetic radiation, thereby causing permeabilization of a barrier of the cell; and
   - contacting the mixture of the cell and the one or more photoresponsive organic particles with a cargo, thereby delivering the cargo into the cell.

2. The method according to claim 1, wherein the largest distance between two points of the one or more photoresponsive organic particles is about 250 nm to about 1250 nm; preferably about 300 nm to about 1200 nm, about 400 nm to about 1100 nm, or about 500 nm to about 1000 nm.

3. The method according to claim 1 or 2, wherein the cargo is selected from the group consisting of a nucleic acid, a protein, a chemical substance, a polysaccharide, and combinations thereof; preferably wherein the cargo is a nucleic acid; more preferably wherein the cargo is mRNA or plasmid DNA.

4. The method according to any one of claims 1 to 3, wherein the photoresponsive organic particle is a photoresponsive polymer particle; preferably wherein the photoresponsive organic particle is a photoresponsive polymer particle selected from a polydopamine (PD) particle, a poly(N-phenylglycine) (PNPG) particle, a poly-2-phenyl-benzobisthiazole (PPBBT) particle, a porphyrin particle, a phthalocyanine particle, or a polypyrrole particle.

5. The method according to any one of claims 1 to 3, wherein the photoresponsive organic particle is a polymer particle, a protein particle, or a lipid particle comprising a light absorbing molecule; preferably wherein the photoresponsive organic particle is a polymer particle, a protein particle, or a lipid particle loaded with or functionalized with a light absorbing molecule.

6. The method according to claim 5, wherein the light absorbing molecule is a molecule selected from the group consisting of a light absorbing dye, a naturally occurring light absorber, and a synthetic light absorber.

7. The method according to any one of claims 1 to 6, wherein the polymer particle comprises poly(DL-lactic-co-glycolic acid) (PLGA), poly(lactic acid) (PLA), polycaprolactone (PCL), ethyl cellulose, cellulose acetophthalate, cellulose, polyvinyl alcohol, polyethylene glycol, gelatine, collagen, silk, alginate, hyaluronic acid, dextran, starch, polycarbonate, polyacrylate, polystyrene, methoxy-PEG-polylactide, poly(alkyl cyanoacrylate) (PACA), poly(D,L-lactide-co-glycolide (PLGH), poly(allylamine hydrochloride), or a polyoxazoline.

8. The method according to any one of claims 1 to 7, wherein the photoresponsive organic particle is functionalized on the surface; preferably wherein the photoresponsive organic particle is coated with one or more compounds selected from the group consisting of albumin, polyethyleneimine (PEI), polyvinylpyrrolidone (PVP), polyethylene glycol (PEG), poly(diallyldimethylammonium chloride) (PDDAC), poly(allylamine hydrochloride) (PAH), polyamidoamine (PAA), poly(amino-co-ester) (PAE), poly[2-(N,N-dimethylamino)ethyl methacrylate] (PDMAEMA), hyaluronic acid (HA), gelatin, polyglycerol, a cyclodextrin (CD), dextran, cellulose, silica, polyoxazoline, sulfobetaine-silane (SBS), a cationic lipid, a neutral lipid, an anionic lipid, chitosan, and poly-L-Lysine.

9. The method according to any one of claims 1 to 8, wherein the photoresponsive organic particle is biodegradable.

10. The method according to any one of claims 1 to 9, wherein the photoresponsive organic particle is a polydopamine particle; preferably wherein the photoresponsive organic particle is a polydopamine particle coated with albumin.

11. The method according to any one of claims 1 to 10, wherein the cell is an immune cell; preferably wherein the immune cell is T cell, a lymphocyte, a macrophage, a dendritic cell, a monocyte, a NK cell, a NKT cell, a B cell, a neutrophil, a granulocyte, a microglial cell, or a Langerhans cell.

12. The method according to any one of claims 1 to 11, wherein the electromagnetic radiation is generated by a laser, such as a pulsed laser; preferably wherein:

- the intensity of the pulses of the laser may be at least $10^4$ W/cm$^2$, such as $10^4$ to $10^{17}$ W/cm$^2$;
- the fluence of the pulses of the laser may be at least 0.01 mJ/cm$^2$, such as 0.01 J/cm$^2$ to 100 J/cm$^2$;
- the number of pulses of the laser may be at least 1 laser pulse; such as 1 to 1000 laser pulses; and/or
- the duration of the pulses of the laser may be at least 1 fs, such as 1 fs to 100 s.

13. One or more photoresponsive organic particle as defined in any one of claims 1 to 10 and a cargo as defined in any one of claims 1 to 10, for use in a method of therapy in a subject, the method comprising:

- administering the one or more photoresponsive organic particles to the surrounding of the cell of the subject;
- irradiating at least part of the surrounding of the cell of the subject, thereby causing permeabilization of a barrier of the cell; and
- administering the cargo to the surrounding of the cell of the subject, thereby delivering the cargo into the cell;

or the method comprising:

- administering the one or more photoresponsive organic particle and the cargo to the surrounding of the cell of the subject; and
- irradiating at least part of the surrounding of the cell of the subject, thereby causing permeabilization of a barrier of the cell and delivering the cargo into the cell.

14. The one or more photoresponsive organic particles and cargo for use according to claim 13, wherein the largest distance between two points of the one or more photoresponsive organic particles is about 250 nm to about 1250 nm; preferably about 300 nm to about 1200 nm, about 400 nm to about 1100 nm, or about 500 nm to about 1000 nm.

15. The one or more photoresponsive organic particles and cargo for use according to any one of claims 13 or 14, wherein:

- the cell is an animal cell;
- the cell is a human cell;
- the cell is an immune cell; preferably wherein the immune cell is a T cell, a lymphocyte, a macrophage, a dendritic cell, a monocyte, a NK cell, a NKT cell, a B cell, a neutrophil, a granulocyte, a microglial cell, or a Langerhans cell; and/or
- wherein the electromagnetic radiation is generated by a laser, such as a pulsed laser.

**Patentansprüche**

1. In-vitro- oder Ex-vivo-Verfahren zum Abgeben einer Fracht in eine Zelle, wobei das Verfahren Folgendes umfasst:

- In-Kontakt-Bringen einer Zelle mit einem oder mehreren fotoreaktiven organischen Partikeln und einer Fracht, wobei die Fracht nicht an das eine oder die mehreren fotoreaktiven Partikel gebunden ist und wobei das organische Partikel aus der Gruppe ausgewählt wird, die aus einem Polymerpartikel, einem Proteinpartikel, einem Lipidpartikel und einer Kombination daraus besteht, wodurch eine Mischung aus der Zelle, der Fracht und dem einen oder den mehreren fotoreaktiven organischen Partikeln gewonnen wird, und
- Bestrahlen der Mischung aus der Zelle, der Fracht und dem einen oder den mehreren fotoreaktiven organischen Partikeln mit elektromagnetischer Strahlung, wodurch die Permeabilisierung einer Barriere der Zelle und die Abgabe der Fracht in die Zelle bewirkt wird, oder
- In-Kontakt-Bringen einer Zelle mit einem oder mehreren fotoreaktiven organischen Partikeln, wobei das organische Partikel aus der Gruppe ausgewählt wird, die aus einem Polymerpartikel, einem Proteinpartikel, einem Lipidpartikel und einer Kombination daraus besteht, wodurch eine Mischung aus der Zelle und dem einen oder den mehreren fotoreaktiven organischen Partikeln gewonnen wird,
- Bestrahlen der Mischung aus der Zelle und dem einen oder den mehreren fotoreaktiven organischen Partikeln mit elektromagnetischer Strahlung, wodurch die Permeabilisierung einer Barriere der Zelle bewirkt wird, und
- In-Kontakt-Bringen der Mischung aus der Zelle und dem einen oder den mehreren fotoreaktiven organischen Partikeln mit einer Fracht, wodurch die Fracht in die Zelle abgegeben wird.

2. Verfahren nach Anspruch 1, wobei der größte Abstand zwischen zwei Punkten des einen oder der mehreren fotoreaktiven organischen Partikel etwa 250 nm bis etwa 1250 nm beträgt, vorzugsweise etwa 300 nm bis etwa 1200 nm, etwa 400 nm bis etwa 1100 nm oder etwa 500 nm bis etwa 1000 nm.

3. Verfahren nach Anspruch 1 oder 2, wobei die Fracht aus der Gruppe ausgewählt wird, die aus einer Nukleinsäure, einem Protein, einer chemischen Substanz einem Polysaccharid und Kombinationen daraus besteht, wobei die Fracht vorzugsweise eine Nukleinsäure ist, wobei die Fracht bevorzugter mRNA oder Plasmid-DNA ist.

4. Verfahren nach einem der Ansprüche 1 bis 3, wobei das fotoreaktive organische Partikel ein fotoreaktives Polymerpartikel ist, wobei das fotoreaktive organische Partikel vorzugsweise ein fotoreaktives Polymerpartikel ist, das aus einem Polydopamin- (PD-) Partikel, einem Poly(N-phenylglycin-) (PNPG-) Partikel, einem Poly-2-phenyl-benzobisthiazol-(PPBBT-) Partikel, einem Porphyrinpartikel, einem Phthalocyaninpartikel oder einem Polypyrrolpartikel ausgewählt wird.

5. Verfahren nach einem der Ansprüche 1 bis 3, wobei das fotoreaktive organische Partikel ein Polymerpartikel, ein Proteinpartikel oder ein Lipidpartikel ist, das ein lichtabsorbierendes Molekül umfasst, wobei das fotoreaktive organische Partikel vorzugsweise ein Polymerpartikel, ein Proteinpartikel oder ein Lipidpartikel ist, das mit einem lichtabsorbierenden Molekül beladen oder funktionalisiert ist.

6. Verfahren nach Anspruch 5, wobei das lichtabsorbierende Molekül ein Molekül ist, das aus der Gruppe ausgewählt wird, die aus einem lichtabsorbierenden Farbstoff, einem natürlich vorkommenden Lichtabsorbens und einem synthetischen Lichtabsorbens besteht.

7. Verfahren nach einem der Ansprüche 1 bis 6, wobei das Polymerpartikel Poly(DL-milch-co-glykolsäure) (PLGA), Poly(milchsäure) (PLA), Polycaprolacton (PCL), Ethylcellulose, Celluloseacetophthalat, Cellulose, Polyvinylalkohol, Polyethylenglycol, Gelatine, Collagen, Seide, Alginat, Hyaluronsäure, Dextran, Stärke, Polycarbonat, Polyacrylat, Polystyrol, Methoxy-PEG-polylactid, Poly(alkylcyanoacrylat) (PACA), Poly(D,L-lactid-co-glycolid (PLGH), Poly(allylaminhydrochlorid) oder ein Polyoxazolin umfasst.

8. Verfahren nach einem der Ansprüche 1 bis 7, wobei das fotoreaktive organische Partikel an der Oberfläche funktionalisiert ist, wobei das fotoreaktive organische Partikel vorzugsweise mit einer oder mehreren Verbindungen beschichtet ist, die aus der Gruppe ausgewählt sind, die aus Albumin, Polyethylenimin (PEI), Polyvinylpyrrolidon (PVP), Polyethylenglycol (PEG), Poly(diallyldimethylammoniumchlorid) (PDDAC), Poly(allylaminhydrochlorid) (PAH), Polyamidoamin (PAA), Poly(amino-co-ester) (PAE), Poly[2-(N,N-dimethylamino)ethylmethacrylat] (PDMAEMA), Hyalurosäure (HA), Gelatine, Polyglycerol, einem Cyclodextrin (CD), Dextran, Cellulose, Siliziumoxid, Polyoxazolin, Sulfobetainsilan (SBS), einem kationischen Lipid, einem neutralen Lipid, einem anionischen Lipid, Chitosan, und Poly-L-Lysin besteht.

9. Verfahren nach einem der Ansprüche 1 bis 8, wobei das fotoreaktive organische Partikel biologisch abbaubar ist.

10. Verfahren nach einem der Ansprüche 1 bis 9, wobei das fotoreaktive organische Partikel ein Polydopaminpartikel ist, wobei das fotoreaktive organische Partikel vorzugsweise ein mit Albumin beschichtetes Polydopaminpartikel ist.

11. Verfahren nach einem der Ansprüche 1 bis 10, wobei die Zelle eine Immunzelle ist, wobei die Immunzelle vorzugsweise eine T-Zelle, ein Lymphozyt, ein Makrophage, eine dentritische Zelle, ein Monozyt, eine NK-Zelle, eine NKT-Zelle, eine B-Zelle, ein Neutrophil, ein Granulozyt, eine Mikrogliazelle oder eine Langerhans-Zelle ist.

12. Verfahren nach einem der Ansprüche 1 bis 11, wobei die elektromagnetische Strahlung von einem Laser erzeugt wird, wie beispielsweise einem gepulsten Laser, wobei vorzugsweise:

  - die Intensität der Impulse des Lasers mindestens $10^4$ W/cm$^2$ betragen können, wie beispielsweise $10^4$ bis $10^{17}$ W/cm$^2$,
  - die Fluenz der Impulse des Laserns mindestens 0,01 mJ/cm$^2$ betragen können, wie beispielsweise 0,01 J/cm$^2$ bis 100 J/cm$^2$,
  - die Anzahl der Impulse des Lasers mindestens 1 Laserimpuls betragen kann, wie beispielsweise 1 bis 1000 Laserimpulse, und/oder
  - die Dauer der Impulse des Lasers mindestens 1 fs betragen kann, wie beispielsweise 1 fs bis 100 fs.

13. Ein oder mehrere fotoreaktive organische Partikel, wie in einem der Ansprüche 1 bis 10 definiert, und eine Fracht, wie in einem der Ansprüche 1 bis 10 definiert, zur Verwendung in einem Verfahren zur Therapie eines Subjekts, wobei das Verfahren Folgendes umfasst:

  - Verabreichen des einen oder der mehreren fotoreaktiven organischen Partikel an die Umgebung der Zelle des Subjekts,
  - Bestrahlen mindestens eines Teils der Umgebung der Zelle des Subjekts, wodurch die Permeabilisierung einer Barriere der Zelle bewirkt wird, und
  - Verabreichen der Fracht an die Umgebung der Zelle des Subjekts, wodurch die Fracht in die Zelle abgegeben wird,

oder wobei das Verfahren Folgendes umfasst

  - Verabreichen des einen oder der mehreren fotoreaktiven organischen Partikel und der Fracht an die Umgebung der Zelle des Subjekts und
  - Bestrahlen mindestens eines Teils der Umgebung der Zelle des Subjekts, wodurch die Permeabilisierung einer Barriere der Zelle und die Abgabe der Fracht in die Zelle bewirkt wird.
- Verabreichen der Fracht an die Umgebung der Zelle des Subjekts, wodurch die Fracht in die Zelle abgegeben wird,

**14.** Ein oder mehrere fotoreaktive organische Partikel und Fracht zur Verwendung nach Anspruch 13, wobei der größte Abstand zwischen zwei Punkten des einen oder der mehreren fotoreaktiven organischen Partikel etwa 250 nm bis etwa 1250 nm beträgt, vorzugsweise etwa 300 nm bis etwa 1200 nm, etwa 400 nm bis etwa 1100 nm oder etwa 500 nm bis etwa 1000 nm.

**15.** Ein oder mehrere fotoreaktive organische Partikel und Fracht zur Verwendung nach einem der Ansprüche 13 und 14, wobei:

- die Zelle eine tierische Zelle ist,
- die Zelle eine menschliche Zelle ist,
- die Zelle eine Immunzelle ist, wobei die Immunzelle vorzugsweise eine T-Zelle, ein Lymphozyt, ein Makrophage, eine dentritische Zelle, ein Monozyt, eine NK-Zelle, eine NKT-Zelle, eine B-Zelle, ein Neutrophil, ein Granulozyt, eine Mikrogliazelle oder eine Langerhans-Zelle ist, und/oder
- wobei die elektromagnetische Strahlung durch einen Laser erzeugt wird, beispielsweise einen gepulsten Laser.

## Revendications

**1.** Procédé *in vitro* ou *ex vivo* de distribution d'une cargaison dans une cellule, le procédé comprenant :

- la mise en contact d'une cellule avec une ou plusieurs particules organiques photosensibles et une cargaison, dans lequel la cargaison n'est pas liée aux une ou plusieurs particules organiques photosensibles, et dans lequel la particule organique est choisie dans le groupe constitué d'une particule de polymère, d'une particule de protéine, d'une particule de lipide, et d'une combinaison de celles-ci, ce qui permet d'obtenir un mélange de la cellule, de la cargaison, et des une ou plusieurs particules organiques photosensibles ; et
- l'irradiation du mélange de la cellule, de la cargaison, et des une ou plusieurs particules organiques photosensibles avec un rayonnement électromagnétique, provoquant ainsi la perméabilisation d'une barrière de la cellule et la distribution de la cargaison dans la cellule ; ou
- la mise en contact d'une cellule avec une ou plusieurs particules organiques photosensibles, dans lequel la particule organique est choisie dans le groupe constitué d'une particule de polymère, d'une particule de protéine, d'une particule de lipide, et d'une combinaison de celles-ci, ce qui permet d'obtenir un mélange de la cellule et des une ou plusieurs particules organiques photosensibles ;
- l'irradiation du mélange de la cellule et des une ou plusieurs particules organiques photosensibles avec un rayonnement électromagnétique, provoquant ainsi la perméabilisation d'une barrière de la cellule ; et
- la mise en contact du mélange de la cellule et des une ou plusieurs particules organiques photosensibles avec une cargaison, ce qui permet de distribuer la cargaison dans la cellule.

**2.** Procédé selon la revendication 1, dans lequel la distance la plus grande entre deux points des une ou plusieurs particules organiques photosensibles est d'environ 250 nm à environ 1250 nm ; de préférence d'environ 300 nm à environ 1 200 nm, d'environ 400 nm à environ 1 100 nm, ou d'environ 500 nm à environ 1 000 nm.

**3.** Procédé selon la revendication 1 ou 2, dans lequel la cargaison est choisie dans le groupe constitué d'un acide nucléique, d'une protéine, d'une substance chimique, d'un polysaccharide, et de combinaisons de ceux-ci ; de préférence dans lequel la cargaison est un acide nucléique ; de manière davantage préférée dans lequel la cargaison est de l'ARNm ou de l'ADN plasmidique.

**4.** Procédé selon l'une quelconque des revendications 1 à 3, dans lequel la particule organique photosensible est une particule de polymère photosensible ; de préférence dans lequel la particule organique photosensible est une particule de polymère photosensible choisie parmi une particule de polydopamine (PD), une particule de poly(N-phénylglycine) (PNPG), une particule de poly-2-phényl-benzobisthiazole (PPBBT), une particule de porphyrine, une particule de phtalocyanine, ou une particule de polypyrrole.

**5.** Procédé selon l'une quelconque des revendications 1 à 3, dans lequel la particule organique photosensible est une particule de polymère, une particule de protéine, ou une particule de lipide comprenant une molécule absorbant la lumière ; de préférence dans lequel la particule organique photosensible est une particule de polymère, une particule de protéine, ou une particule de lipide chargée ou fonctionnalisée avec une molécule absorbant la lumière.

6. Procédé selon la revendication 5, dans lequel la molécule absorbant la lumière est une molécule choisie dans le groupe constitué d'un colorant absorbant la lumière, d'un absorbeur de lumière naturel, et d'un absorbeur de lumière synthétique.

7. Procédé selon l'une quelconque des revendications 1 à 6, dans lequel la particule de polymère comprend du poly(acide DL-lactique-co-glycolique) (PLGA), du poly(acide lactique) (PLA), de la polycaprolactone (PCL), de l'éthyl cellulose, de l'acétophtalate de cellulose, de la cellulose, de l'alcool polyvinylique, du polyéthylène glycol, de la gélatine, du collagène, de la soie, de l'alginate, de l'acide hyaluronique, du dextrane, de l'amidon, du polycarbonate, du polyacrylate, du polystyrène, du méthoxy-PEG-polylactide, du poly(cyanoacrylate d'alkyle) (PACA), du poly(D,L-lactide-co-glycolide (PLGH), du poly(chlorhydrate d'allylamine), ou une polyoxazoline.

8. Procédé selon l'une quelconque des revendications 1 à 7, dans lequel la particule organique photosensible est fonctionnalisée sur la surface ; de préférence dans lequel la particule organique photosensible est revêtue d'un ou plusieurs composés choisis dans le groupe constitué d'albumine, de polyéthylèneimine (PEI), de polyvinylpyrrolidone (PVP), de polyéthylène glycol (PEG), de poly(chlorure de diallyldiméthylammonium) (PDDAC), de poly(chlorhydrate d'allylamine) (PAH), de polyamidoamine (PAA), de poly(amino-co-ester) (PAE), de poly[méthacrylate de 2-(N,N-diméthylamino)éthyle] (PDMAEMA), d'acide hyaluronique (HA), de gélatine, de polyglycérol, d'une cyclodextrine (CD), de dextrane, de cellulose, de silice, de polyoxazoline, de sulfobétaïne-silane (SBS), d'un lipide cationique, d'un lipide neutre, d'un lipide anionique, de chitosane, et de poly-L-Lysine.

9. Procédé selon l'une quelconque des revendications 1 à 8, dans lequel la particule organique photosensible est biodégradable.

10. Procédé selon l'une quelconque des revendications 1 à 9, dans lequel la particule organique photosensible est une particule de polydopamine ; de préférence dans lequel la particule organique photosensible est une particule de polydopamine revêtue d'albumine.

11. Procédé selon l'une quelconque des revendications 1 à 10, dans lequel la cellule est une cellule immunitaire ; de préférence dans lequel la cellule immunitaire est une cellule T, un lymphocyte, un macrophage, une cellule dendritique, un monocyte, une cellule NK, une cellule NKT, une cellule B, un neutrophile, un granulocyte, une cellule microgliale, ou une cellule de Langerhans.

12. Procédé selon l'une quelconque des revendications 1 à 11, dans lequel le rayonnement électromagnétique est généré par un laser, tel qu'un laser pulsé ; de préférence dans lequel :

- l'intensité des impulsions du laser peut être d'au moins $10^4$ W/cm$^2$, telle que de $10^4$ à $10^{17}$ W/cm$^2$ ;
- la fluence des impulsions du laser peut être d'au moins 0,01 mJ/cm$^2$, telle que de 0,01 J/cm$^2$ à 100 J/cm$^2$ ;
- le nombre d'impulsions du laser peut être d'au moins 1 impulsion laser ; tel que 1 à 1 000 impulsions laser ; et/ou
- la durée des impulsions du laser peut être d'au moins 1 fs, telle que 1 fs à 100 s.

13. Une ou plusieurs particules organiques photosensibles selon l'une quelconque des revendications 1 à 10 et une cargaison selon l'une quelconque des revendications 1 à 10, pour une utilisation dans un procédé de thérapie chez un sujet, le procédé comprenant :

- l'administration des une ou plusieurs particules organiques photosensibles à l'environnement de la cellule du sujet ;
- l'irradiation d'au moins une partie de l'environnement de la cellule du sujet, provoquant ainsi la perméabilisation d'une barrière de la cellule ; et
- l'administration de la cargaison à l'environnement de la cellule du sujet, ce qui permet de distribuer la cargaison dans la cellule ;

ou le procédé comprenant :

- l'administration des une ou plusieurs particules organiques photosensibles et de la cargaison à l'environnement de la cellule du sujet ; et

- l'irradiation d'au moins une partie de l'environnement de la cellule du sujet, provoquant ainsi la perméabilisation d'une barrière de la cellule et la distribution de la cargaison dans la cellule.

**14.** Une ou plusieurs particules organiques photosensibles et cargaison pour une utilisation selon la revendication 13, dans lesquelles la distance la plus grande entre deux points des une ou plusieurs particules organiques photosensibles est d'environ 250 nm à environ 1 250 nm ; de préférence d'environ 300 nm à environ 1 200 nm, d'environ 400 nm à environ 1 100 nm, ou d'environ 500 nm à environ 1 000 nm.

**15.** Une ou plusieurs particules organiques photosensibles et cargaison pour une utilisation selon l'une quelconque des revendications 13 ou 14, dans lesquelles :

- la cellule est une cellule animale ;
- la cellule est une cellule humaine ;
- la cellule est une cellule immunitaire ; de préférence dans lesquelles la cellule immunitaire est une cellule T, un lymphocyte, un macrophage, une cellule dendritique, un monocyte, une cellule NK, une cellule NKT, une cellule B, un neutrophile, un granulocyte, une cellule microgliale, ou une cellule de Langerhans ; et/ou
- dans lesquelles le rayonnement électromagnétique est généré par un laser, tel qu'un laser pulsé.

FIG. 1

FIG. 2A

FIG. 2B

FIG. 2C

## FIG. 2D

## FIG. 2E

## FIG. 2F

FIG. 2G

FIG. 2H

FIG. 2I

FIG. 3A

FIG. 3B

FIG. 3C

## FIG. 3D

## FIG. 3E

## FIG. 3F

FIG. 4A

■ Positive cells
• 24h viability
▨ rMFI

FIG. 4B

■ Transfected
▨ 24h viability
▨ Transfection yield

FIG. 4C

## FIG. 5A

■ Positive cells
● 24h viability
▨ rMFI

## FIG. 5B

## FIG. 5C

■ Transfected
● 24h viability
▨ rMFI

## FIG. 5D

Photoporation control

Photoporation mRNA

## FIG. 5E

■ Transfected
▨ 24h viability
▨ rMFI

## FIG. 5F

***

## FIG. 6A

## FIG. 6B

| Dh (nm) | PDI |
|---------|-------|
| 134.3 | 0.104 |
| 282.0 | 0.027 |
| 449.1 | 0.101 |
| 753.1 | 0.060 |
| 1079 | 0.177 |

## FIG. 7A

PD-BSA NPs 100 nm ($\times 10^9$ NPs/mL)

FIG. 7B

PD-BSA NPs 100 nm (x10$^9$ NPs/mL)

FIG. 8A

PD-BSA NPs 100 nm (x10$^9$ NPs/mL)

FIG. 8B

PD-BSA NPs 100 nm (x10$^9$ NPs/mL)

## FIG. 9A

PD-BSA NPs 300 nm (x10$^8$ NPs/mL)

## FIG. 9B

PD-BSA NPs 300 nm (x10$^8$ NPs/mL)

## FIG. 10A

PD-BSA NPs 300 nm (x10$^8$ NPs/mL)

FIG. 10B

PD-BSA NPs 300 nm (x10⁸ NPs/mL)

FIG. 11A

PD-BSA NPs 500 nm (x10⁸ NPs/mL)

FIG. 11B

PD-BSA NPs 500 nm (x10⁸ NPs/mL)

FIG. 12A

PD-BSA NPs 500 nm (x10$^8$ NPs/mL)

FIG. 12B

PD-BSA NPs 500 nm (x10$^8$ NPs/mL)

FIG. 13A

PD-BSA NPs 750 nm (x10$^8$ NPs/mL)

FIG. 13B

PD-BSA NPs 750 nm (x10$^8$ NPs/mL)

FIG. 14A

PD-BSA NPs 750 nm (x10$^8$ NPs/mL)

FIG. 14B

PD-BSA NPs 750 nm (x10$^8$ NPs/mL)

FIG. 15A

FIG. 15B

FIG. 16A

FIG. 16B

PD-BSA NPs 1000 nm (x$10^8$ NPs/mL)

FIG. 17A

PD-PEI NPs 500 nm (x$10^8$ NPs/mL)

FIG. 17B

PD-PEI NPs 500 nm (x$10^8$ NPs/mL)

FIG. 18A

PD-PEI NPs 500 nm ($\times 10^8$ NPs/mL)

FIG. 18B

PD-PEI NPs 500 nm ($\times 10^8$ NPs/mL)

FIG. 19A

PD NPs 500 nm ($\times 10^8$ NPs/mL)

FIG. 19B

FIG. 20A

FIG. 20B

FIG. 21

PPy-PVA NPs

FIG. 22

FIG. 23

**FD500**

FIG. 24

## Yield% - FD500 delivery - PPy NPs

FIG. 25

# LNP-TB NPs

Z-average = 372 ± 1 nm
Zeta-potential = + 21.1 ± 1.6 mV

FIG. 26

Laser off                    Laser on

FIG. 27

**LNP-TB photo - FD500**

FIG. 28

Size Distribution by Intensity

Zeta Potential Distribution

| | Hydrodynamic diameter (nm) | Zeta-potential (mV) |
|---|---|---|
| HSA-ICG NPs | 309 | - 11.9 |

## FIG. 29

Laser off

Laser on

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4816567 A
- US 5270163 A

**Non-patent literature cited in the description**

- **L. RAES et al.** Intracellular delivery of mRNA in adherent and suspension cells by vapor nanobubble photoporation. *NANO-MICRO LETTERS*, 2020, vol. 12 (1)
- **J. LIU et al.** Surface functionalization with polyethylene glycol and polyethyleneimine improves the performance of graphene-based materials for safe and efficient intracellular delivery by laser-induced photoporation. *INTERNATIONAL JOURNAL OF MOLECULAR SCIENCES*, 2020, vol. 21 (4)
- **J. LIU et al.** Repeated photoporation with graphene quantum dots enables homogeneous labeling of live cells with extrinsic markers for fluorescence microscopy. *LIGHT-SCIENCE & APPLICATIONS*, 2018, vol. 7
- **SAKLAYEN et al.** *ACS Nano*, 2017, vol. 11 (4), 3671-3680
- **KURRECK**. *Eur J Biochem*, 2003, vol. 270, 1628-1644
- **HORWELL**. *Trends Biotechnol*, 1995, vol. 13, 132-134
- **KOHLER et al.** *Nature*, 1975, vol. 256, 495
- **CLACKSON et al.** *Nature*, vol. 352, 624-628
- **MARKS et al.** *J Mol Biol*, 1991, vol. 222, 581-597
- **HARLOW** ; **LANE**. Antibodies: A Laboratory Manual. Cold Spring Harbour Laboratory, 1988
- **HARLOW** ; **LANE**. Using Antibodies: A Laboratory Manual. Cold Spring Harbour Laboratory, 1999
- Monoclonal Antibodies: A Manual of Techniques. CRC Press, 1987
- Monoclonal Antibodies: A Practical Approach. Oxford University Press, 2000
- Antibody Engineering: Methods and Protocols. Methods in Molecular Biology. Humana Press, 2004, vol. 248

- **ELLINGTON** ; **SZOSTAK**. *Nature*, 1990, vol. 346, 818-822
- **TUERK** ; **GOLD**. *Science*, 1990, vol. 249, 505-510
- The Aptamer Handbook: Functional Oligonucleotides and Their Applications. Wiley-VCH, 2006
- **SAXENA et al.** *Int J Pharm*, 2004, vol. 278 (2), 293-301
- **SHENG et al.** *ACS Nano*, 2014, vol. 8 (12), 12310-22
- **PATEL et al.** *J. Control. Release*, 2019, vol. 303, 91-100
- **LAJUNEN et al.** *J. Control. Release*, 2018, vol. 284, 213-223
- *CHEMICAL ABSTRACTS*, 72-57-1
- *CHEMICAL ABSTRACTS*, 2869-83-2
- *CHEMICAL ABSTRACTS*, 548-62-9
- *CHEMICAL ABSTRACTS*, 115-39-9
- *CHEMICAL ABSTRACTS*, 3536-49-0
- *CHEMICAL ABSTRACTS*, 6104-59-2
- *CHEMICAL ABSTRACTS*, 5141-20-8
- *CHEMICAL ABSTRACTS*, 2353-45-9
- *CHEMICAL ABSTRACTS*, 3599-32-4
- *CHEMICAL ABSTRACTS*, 61-73-4
- *CHEMICAL ABSTRACTS*, 92-31-9
- *CHEMICAL ABSTRACTS*, 518-47-8
- *CHEMICAL ABSTRACTS*, 4159-77-7
- *CHEMICAL ABSTRACTS*, 989-38-8
- **JU et al.** *Biomacromolecules*, 2011, vol. 12, 625
- **S. E. ZAYAN et al.** *AIP Conference Proceedings*, 2019, vol. 2190, 020025
- **D.MIRANDA et al.** *Biomater. Sci.*, 2019, vol. 7, 3158
- **Z. SHENG et al.** *ACS Nano*, 2014, vol. 8, 12310